# EUROPEAN PATENT APPLICATION

(11) **EP 4 417 604 A1**
(43) Date of publication of application: **21.08.2024**
(21) Application number: 22881328.3
(22) Date of filing: 12.10.2022
(51) Int. Cl.: C07D 405/14, C07D 407/14, C07D 413/14, C07D 417/14, C07D 471/04, C07D 409/14, H10K 99/00

(54) **NOVEL COMPOUND FOR CAPPING LAYER, AND ORGANIC LIGHT-EMITTING DEVICE COMPRISING SAME**

(30) Priority: 13.10.2021 KR 20210135616
(71) Applicant: Dongjin Semichem Co., Ltd., Incheon 22824 (KR)
(72) Inventor: HAM, Ho Wan, Hwaseong-si Gyeonggi-do 18635 (KR); AN, Hyun Cheol, Hwaseong-si Gyeonggi-do 18635 (KR); MIN, Byung Cheol, Hwaseong-si Gyeonggi-do 18635 (KR); KIM, Dong Jun, Hwaseong-si Gyeonggi-do 18635 (KR); HAN, Jeong Woo, Hwaseong-si Gyeonggi-do 18635 (KR); LEE, Hyung Jin, Hwaseong-si Gyeonggi-do 18635 (KR); ANN, Ja Eun, Hwaseong-si Gyeonggi-do 18635 (KR); KWON, Dong Yuel, Hwaseong-si Gyeonggi-do 18635 (KR)
(74) Representative: Grünecker Patent- und Rechtsanwälte PartG mbB
(86) International application number: PCT/KR2022/015349
(87) International publication number: WO 2023/063701

(57) **Abstract**

A novel compound for a capping layer, and an organic light emitting device containing the same are disclosed.

## Description

### BACKGROUND

### 1. Technical Field

The present disclosure relates to a novel compound for a capping layer and an organic light emitting device including the same.

### 2. Description of the Related Art

Materials used for organic layers in organic light emitting devices can be classified into light emitting materials, hole injection materials, hole transport materials, electron transport materials, electron injection materials, and the like, according to the functions thereof.

In addition, the light emitting materials can be classified into a fluorescent material derived from a singlet excited state of an electron and a phosphorescent material derived from a triplet excited state of an electron according to the light emitting mechanisms and also classified into blue, green, and red light emitting materials according to the emission colors.

A typical organic light emitting device may have a structure in which an anode is disposed on a substrate, and a hole transport layer, a light emitting layer, an electron transport layer, and a cathode are sequentially stacked on the anode. Here, the hole transport layer, the light emitting layer, and the electron transport layer are organic thin films made of organic compounds.

The principle for driving such an organic light emitting device having the structure will be described below.

When a voltage is applied between the anode and the cathode, holes injected from the anode move to the light emitting layer through the hole transport layer, and electrons injected from the cathode move to the light emitting layer through the electron transport layer. The holes and electrons recombine in the light emitting layer to generate excitons. The holes and electrons recombine in the light emitting layer to generate excitons.

Light is generated when the excitons change from an excited state to a ground state. Regarding the efficiency of the organic light emitting device, internal luminous efficiency and external luminous efficiency are considered. The internal luminous efficiency is related to how efficiently excitons are generated and photoconverted in the organic layers interposed between the anode and cathode, such as the hole transport layer, the light emitting layer, and the electron transport layer. The internal luminous efficiency is theoretically known to be 25% for the fluorescence and 100% for the phosphorescence.

On the other hand, the external luminous efficiency refers to how efficiently extract the light generated in the organic layers so that the light can exit the organic light emitting device, and it is known that the level of the external luminous efficiency is about 20% of level of the internal luminous efficiency. To increase the light extraction, various organic compounds with a refractive index of 1.7 or more have been used for a capping layer to prevent light from being lost by total reflection. In addition, to improve the performance of organic light emitting devices, efforts have been made to develop organic compounds with high refractive index and thin film stability that increase external light emission efficiency.

### SUMMARY

The present disclosure is to provide a compound having a structure in which four or more ring-fused groups are bonded in two directions around an amine core and a heteroaryl group is bonded in one direction, wherein the compound has a wide band gap that makes it difficult to absorb visible light, a high refractive index, and an increased absorption property in the ultraviolet region. In addition, the purpose of the present invention is to provide an organic light emitting device having high color purity, high efficiency, and long lifespan.

In addition, the purpose of the present invention is to provide a compound having a structure in which an amine core and four or more ring-fused group are linearly bonded to have excellent intermolecular thin film arrangement, a higher refractive index and improved stability against external air and moisture. In addition, the purpose of the present invention is to provide a compound having high Tg and high Td to prevent intermolecular recrystallization and maintain a stable thin film from heat generated when driving an organic light-emitting device. In addition, the purpose of the present invention is to provide an organic light emitting device having improved external quantum efficiency and improved lifespan.

The above-mentioned objectives and other objectives will be understood from the description provided below.

To address the above challenges, in one aspect, the present disclosure provides a compound for a capping layer, wherein the compound is represented by the following Formula 1:

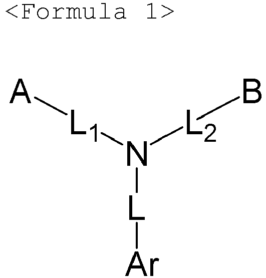

In Formula 1,
A and B are each independently Formula 1-1 or Formula 1-2,
L, L1 and L2 are each independently a direct bond, a substituted or unsubstituted C5~C50 arylene group, or a substituted or unsubstituted C2~C50 heteroarylene group, and
Ar is a substituted or unsubstituted C2~C50 heteroaryl group, or a C5~C50 aryl group substituted with a cyano group:

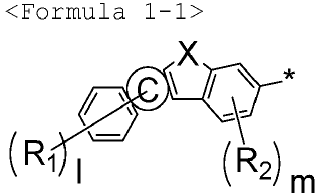

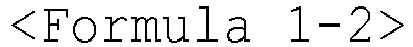

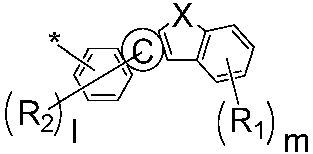

In Formula 1-1 and Formula 1-2,
X is each independently O, S, CRR' or NR,
C-ring is each independently a substituted or unsubstituted C5~C30 aryl group, or a substituted or unsubstituted C2~C30 heteroarylene group,
R, R', R1 and R2 are each independently hydrogen, deuterium, halogen, nitro group, nitrile group, hydroxy group, thiol group, a substituted or unsubstituted amino group, a substituted or unsubstituted C1~C30 alkyl group, a substituted or unsubstituted C2~C30 alkenyl group, a substituted or unsubstituted C1~C30 alkoxy group, a substituted or unsubstituted C1~C30 sulfide group, a substituted or unsubstituted C0~C30 silyl group, a substituted or unsubstituted C5~C50 aryl group, or a substituted or unsubstituted C2~C50 heteroaryl group, and a plurality of R1 adjacent to each other, a plurality of R2 adjacent to each other, or a plurality of R and R' adjacent to each other, may be joined together to form or not to form a ring.

In addition, in one embodiment, there is provided an organic light emitting device including the compound described above.

The compound for a capping layer according to one embodiment of the present disclosure has a structure in which four or more ring-fused groups are bonded in two directions around an amine core and a heteroaryl group is bonded in one direction, wherein the compound has a wide band gap that makes it difficult to absorb visible light, a high refractive index, and an increased absorption property in the ultraviolet region, so when used in the capping layer of an organic light-emitting device, an organic light-emitting device with high color purity, high efficiency, and long lifespan can be provided.

In addition, the compound for a capping layer according to one embodiment of the present disclosure has a structure in which an amine core and four or more ring-fused group are linearly bonded to have excellent intermolecular thin film arrangement, a higher refractive index and improved stability against external air and moisture. In addition, the compound has high Tg and high Td to prevent intermolecular recrystallization and maintains a stable thin film from heat generated when driving an organic light-emitting device, so organic light emitting device having improved external quantum efficiency and improved lifespan can be provided.

The above effects and other effects will be described in detail below.

### BRIEF DESCRIPTION OF THE DRAWINGS

FIG. 1 is a schematic cross-sectional view illustrating the construction of an organic light emitting device according to one embodiment of the present disclosure, and
FIG. 2 shows the absorption intensity measured in the wavelength range of 340 nm to 500 nm.

### ** Explanation of drawing symbols **

100: substrate
200: Hole injection layer
300: Hole transport layer
400: light emitting layer
500: electron transport layer
600: Electron injection layer
1000: first electrode(anode)
2000: Second electrode(cathode)
3000: capping layer

### DETAILED DESCRIPTION

Prior to a description of the present disclosure, it should be noted that the terms used in the present specification are used only to describe specific examples and are not intended to limit the scope of the present disclosure which will be defined only by the appended claims.

Unless otherwise defined herein, all terms including technical and scientific terms used herein have the same meaning as commonly understood by those who are ordinarily skilled in the art to which the present disclosure pertains.

Unless otherwise stated herein, it will be further understood that the terms "comprise", "comprises", and "comprising", when used in this specification, specify the presence of stated features, regions, integers, steps, operations, elements and/or components but do not preclude the presence or addition of one or more other features, regions, integers, steps, operations, elements, components and/or groups thereof.

Throughout the specification and claims of the disclosure, the term "aryl" refers to a functional group having a C5-50 aromatic hydrocarbon ring, and examples thereof include phenyl, benzyl, naphthyl, biphenyl, terphenyl, fluorene, phenanthrenyl, triphenylenyl, perylenyl, chrysenyl, fluoranthenyl, benzofluorenyl, benzotriphenylenyl, benzochrysenyl, anthracenyl, stilbenyl, or pyrenyl. The term "heteroaryl" refers to a C2-50 aromatic ring structure containing at least one heteroatom, and it includes a heterocyclic ring formed from pyrrolyl, pyrazinyl, pyridinyl, indolyl, isoindolyl, furyl, benzofuranyl, isobenzofuranyl, dibenzofuranyl, benzothiophenyl, dibenzothiophenyl, quinolyl group, isoquinolyl, quinoxalyl, carbazolyl, phenanthridinyl, acridinyl, phenanthrolinyl, thienyl, a pyridine ring, a pyrazine ring, a pyrimidine ring, a pyridazine ring, a triazine ring, an indole ring, a quinoline ring, an acridine ring, a pyrrolidine ring, a dioxane ring, a piperidine ring, a morpholine ring, a piperazine ring, a carbazole ring, a furan ring, a thiophene ring, an oxazole ring, an oxadiazole ring, a benzofuran ring, a thiazole ring, a thiadiazole ring, a benzothiophene ring, a triazole ring, an imidazole ring, a benzoimidazole ring, a pyran ring, or a dibenzofuran ring.

In chemical formulas: Arx (where x is an integer) means a substituted or unsubstituted C6~C50 aryl group, or a substituted or unsubstituted C2~C50 heteroaryl group, unless otherwise defined; Lx (where x is an integer) means a directly bonded and a substituted or unsubstituted C6~C50 arylene group or a substituted or unsubstituted C2~C50 heteroarylene group, unless otherwise defined; and Rx (where x is an integer), means a hydrogen, deuterium, halogen, a nitro group, a nitrile group, a substituted or unsubstituted C1~C30 alkyl group, a substituted or unsubstituted C2~C30 alkenyl group, a substituted or unsubstituted C1~C30 alkoxy group, a substituted or unsubstituted C1~C30 sulfide group, a substituted or unsubstituted C6~C50 aryl group, or a substituted or unsubstituted C2~C50 heteroaryl group, unless otherwise defined.

Throughout the present specification and claims, the term "substituted or unsubstituted" means that a portion is substituted or unsubstituted by at least one selected from the group consisting of deuterium, halogen, amino group, cyano group, nitrile group, nitro group, nitroso group, sulfamoyl group, isothiocyanate group, thiocyanate group, carboxyl group, C1~C30 alkyl group, C1~C30 alkylsulfinyl group, C1~C30 alkylsulfonyl group, C1~C30 alkylsulfanyl group, C1~C12 fluoroalkyl group, C2~C30 alkenyl group, C1~C30 alkoxy group, C1~C12 N-alkylamino group, C2~C20 N,N-dialkylamino group, substituted or unsubstituted C1~C30 sulfide group, C1~C6 N-alkylsulfamoyl group, C2~C12 N,N-dialkylsulfamoyl group, C0~C30 silyl group, C3~C20 cycloalkyl group, C3~C20 heterocycloalkyl group, C6~C50 aryl group, C3~C50 heteroaryl group, etc, but is not particularly limited thereto. In addition, the same symbols throughout the present specification may have the same meaning unless otherwise specified.

All or some embodiments described herein may be selectively combined and configured so that the embodiments may be modified in various ways unless the context clearly indicates otherwise.

Hereinafter, embodiments of the present disclosure and the effects thereof will be described in detail below.

Embodiments of the present invention will be described in detail below.

The compound for the capping layer according to the present invention may be represented by the following formula 1:

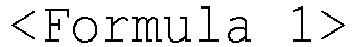

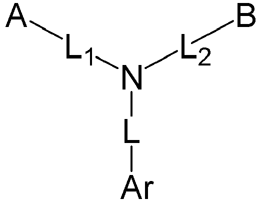

In Formula 1,
A and B are each independently Formula 1-1 or Formula 1-2,
L, L1 and L2 are each independently a direct bond, a substituted or unsubstituted C5~C50 arylene group, or a substituted or unsubstituted C2~C50 heteroarylene group, and
Ar is a substituted or unsubstituted C2~C50 heteroaryl group, or a C5~C50 aryl group substituted with a cyano group.

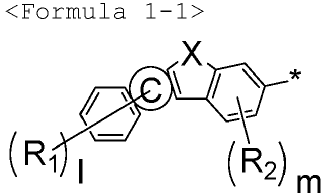

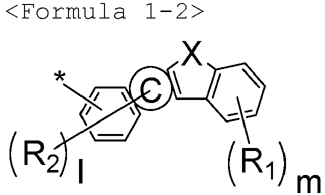

In Formula 1-1 and Formula 1-2,
X is each independently O, S, CRR' or NR,
C-ring is each independently a substituted or unsubstituted C5~C30 aryl group, or a substituted or unsubstituted C2~C30 heteroarylene group,
R, R', R1 and R2 are each independently hydrogen, deuterium, halogen, nitro group, nitrile group, hydroxy group, thiol group, a substituted or unsubstituted amino group, a substituted or unsubstituted C1~C30 alkyl group, a substituted or unsubstituted C2~C30 alkenyl group, a substituted or unsubstituted C1~C30 alkoxy group, a substituted or unsubstituted C1~C30 sulfide group, a substituted or unsubstituted C0~C30 silyl group, a substituted or unsubstituted C5~C50 aryl group, or a substituted or unsubstituted C2~C50 heteroaryl group, and a plurality of R1 adjacent to each other, a plurality of R2 adjacent to each other, or a plurality of R and R' adjacent to each other, may be joined together to form or not to form a ring.

The capping layer compound represented by Formula 1 has a structure in which four or more ring-fused groups are bonded in two directions around an amine core and a heteroaryl group is bonded in one direction, and has a wide band gap that makes it difficult to absorb visible light, a high refractive index, and an increased absorption property in the ultraviolet region, so when used in the capping layer of an organic light-emitting device, an organic light-emitting device with high color purity, high efficiency, and long lifespan can be provided.

In addition, the compound for a capping layer has a structure in which an amine core and four or more ring-fused group are linearly bonded to have excellent intermolecular thin film arrangement, a higher refractive index and improved stability against external air and moisture. In addition, the compound has high Tg and high Td to prevent intermolecular recrystallization and maintains a stable thin film from heat generated when driving an organic light-emitting device, so organic light emitting device having improved external quantum efficiency and improved lifespan can be provided.

Specifically, Formula 1 may be represented by Formula 2 or Formula 3 shown below:

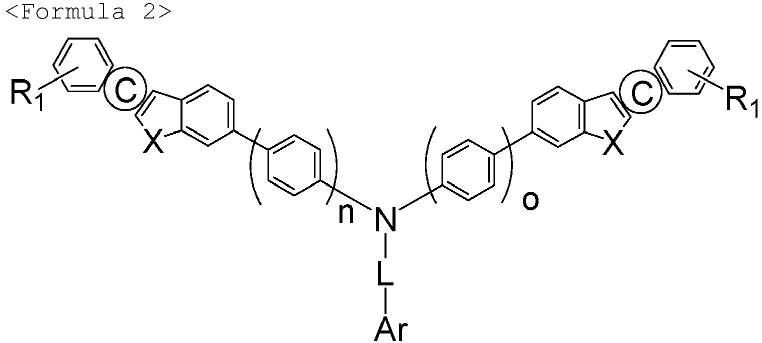

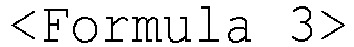

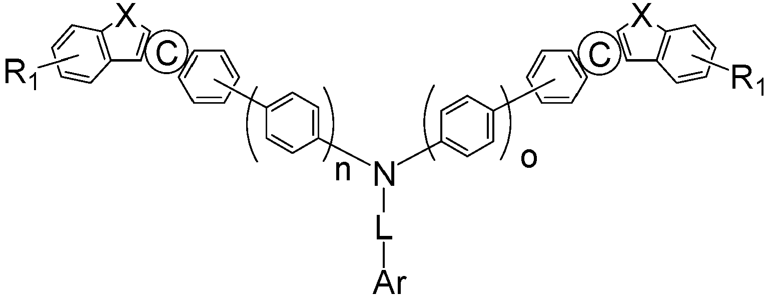

In Formulas 2 and 3,
X, C-ring, L, Ar and R1 are the same as defined in Formula 1, Formula 1-1 and Formula 1-2, and
n and o are each independently an integer in a range of from 0 to 3, and are more specifically 0 or 1.

The compound for the capping layer represented by Formula 2 or Formula 3 has a structure in which the amine core and the four ring-fused group are linearly bonded, so it has excellent thin film arrangement, a high refractive index, and greatly improved absorption intensity in the ultraviolet region. In addition, a higher refractive index can be achieved by bonding the four ring-fused group to the amine core via para-phenylene.

In addition, Formula 1 may be represented by Formula 4 or Formula 5 shown below:

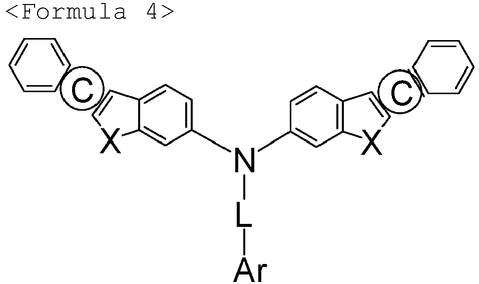

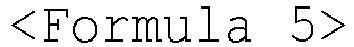

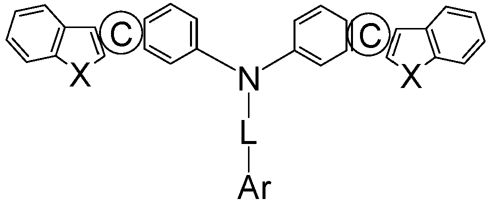

In Formulas 4 and 5,
X, C-ring, L and Ar are the same as defined in Formula 1, Formula 1-1 and Formula 1-2 above.

The compound for the capping layer represented by Formula 4 or Formula 5 has a structure in which the amine core and the four ring-fused group are directly bonded, so the thin film arrangement is excellent, a high refractive index can be maintained, and at the same time, the absorption wavelength intensity in the ultraviolet region can be greatly improved.

More specifically, Formula 1 may be represented by Formula 6 or Formula 7 shown below:

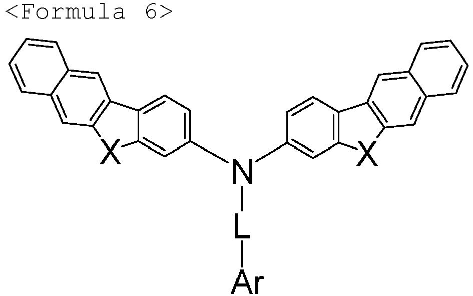

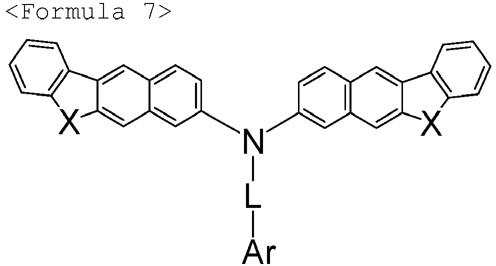

In Formulas 6 and 7,
X, L and Ar are the same as defined in Formula 1, Formula 1-1 and Formula 1-2 above.

The capping layer compound represented by Formula 6 or Formula 7 has minimized bulk characteristics and has a structure in which the four ring-fused group and the amine core are directly bonded in a linear manner, thereby providing maximized intramolecular polarizability, higher refractive index, and greatly improved thermal stability.

Meanwhile, in any one or more of Formulas 1 to 7, X may each independently be O or S. In this case, it is possible to maintain a high refractive index by minimizing bulk characteristics, and at the same time, it is effective in lowering a deposition temperature by reducing the molecular weight.

In addition, in any one or more of Formulas 1 to 7, Ar may include a heteroaryl group composed of one ring or a heteroaryl group fused with two rings. In this case, by having a substituent with minimized bulkiness, excellent intermolecular thin film arrangement, a high refractive index and excellent thermal stability can be obtained.

Specifically, in any one or more of Formulas 1 to 7, Ar may be selected from the group consisting of a quinoline group, a benzofuran group, a benzothiophene group, an indole group, a benzoxazole group, a benzothiazole group, a benzoimidazole group, an indolizine group, an imidazopyridine group, a benzotriazole group, a furan group, a thiophene group, an oxadiazole group, and a thiadiazole group. These are substituents with high polarizability, so higher refractive index can be obtained.

More specifically, Ar may be selected from the group consisting of a quinoline group, a benzofuran group, a benzothiophene group, and an imidazopyridine group. In this case, a high refractive index can be maintained and absorption in the visible light region can be minimized.

In addition, in any one or more of Formulas 1 to 7, L may be a phenylene group or a biphenylene group. In this case, the polarization rate of the compound is increased, the thin film arrangement is excellent, and a high refractive index can be obtained.

As an example, in any one or more of the formulas 1 to 7, *-L-Ar may each independently be selected from the structures represented by Structural Formulas A-1 to A-17 shown below:

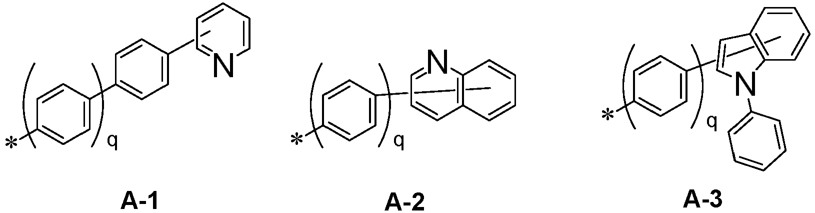

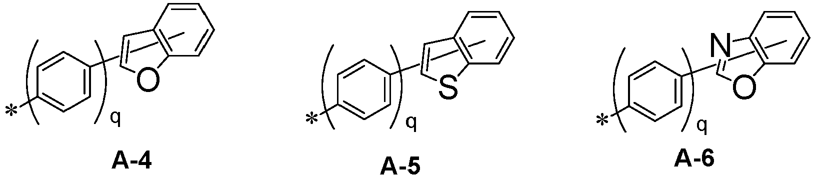

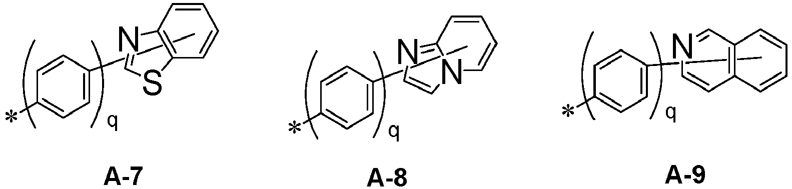

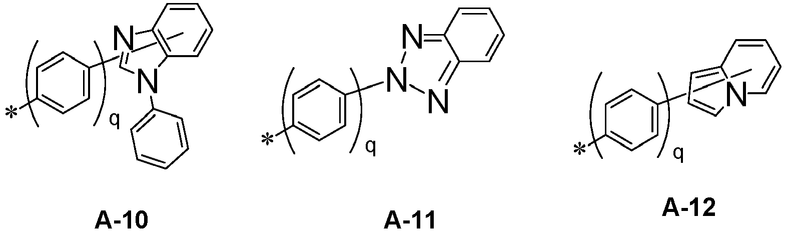

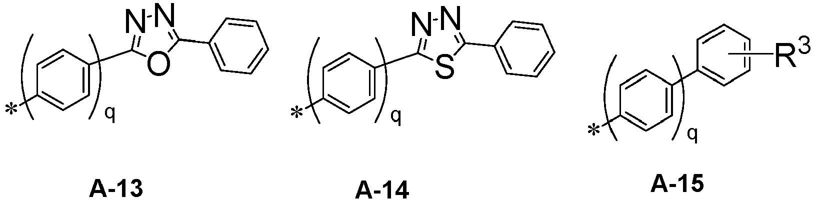

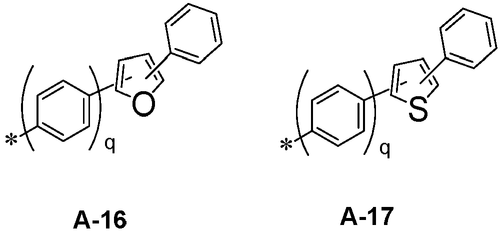

In Structural Formulas A-1 to A-17,
q is each independently an integer in a range of from 0 to 2, R3 is a nitrile group, and "*" is a bonding position bonded to N of Formula 1 above.

In the case of having this structure, absorption in the visible light region is minimized and a high refractive index can be maintained even with a lower molecular weight.

In addition, when the refractive index is measured in the thickness range of 20 nm to 100 nm, more specifically in the thickness range of 50 nm to 100 nm, the capping layer compound represented by any one or more of the formulas 1 to 7 may have a refractive index of 2.40 or more, more specifically, 2.45 or more at a wavelength of 450 nm, and may have an ultraviolet absorption intensity of 0.8 or more, more specifically 1.0 or more at a wavelength of 380 nm.

The compounds shown below are specific examples of compounds according to the present invention. The compounds shown below are presented only to help in understanding the present disclosure, and thus the scope of the present disclosure is not limited thereby.

| | | |
|---|---|---|
| 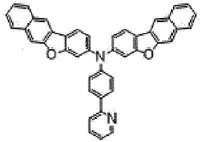 | 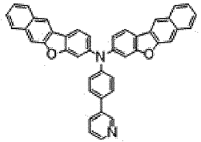 | 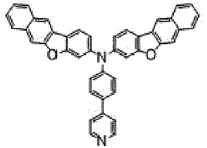 |
| **1** | **2** | **3** |
| 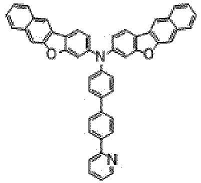 | 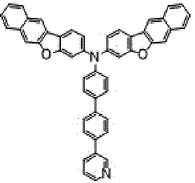 | 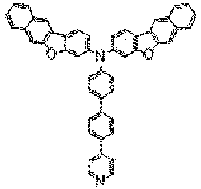 |
| **4** | **5** | **6** |
| 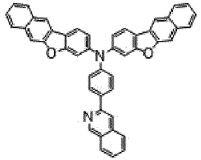 | 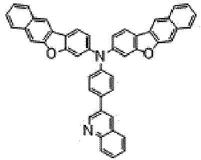 | 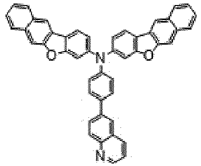 |
| **7** | **8** | **9** |
| 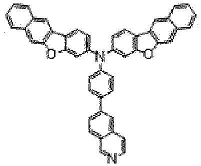 | 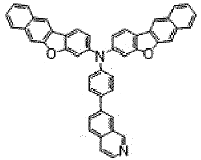 | 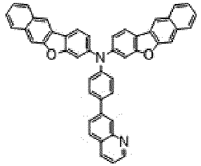 |
| **10** | **11** | **12** |
| 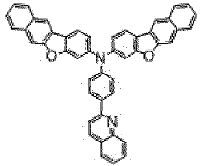 | 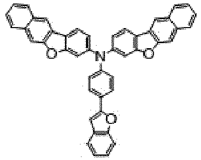 | 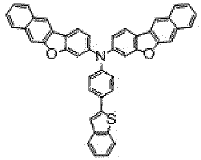 |
| **13** | **14** | **15** |
| 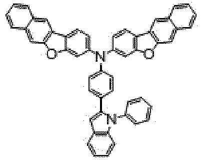 | 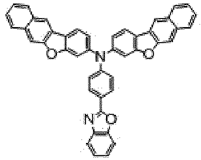 | 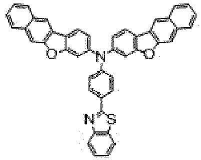 |
| **16** | **17** | **18** |
| 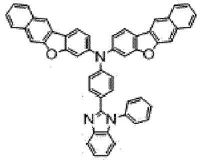 | 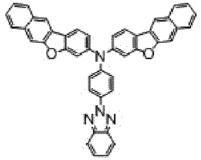 | 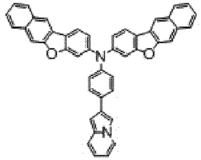 |
| **19** | **20** | **21** |
| 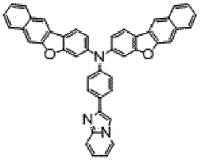 | 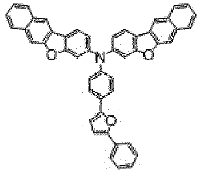 | 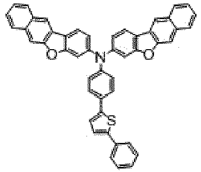 |
| **22** | **23** | **24** |
| 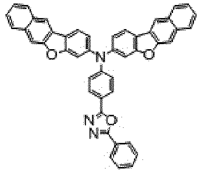 | 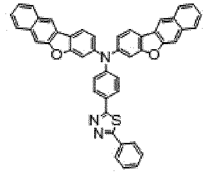 | 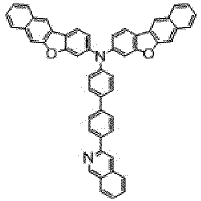 |
| **25** | **26** | **27** |
| 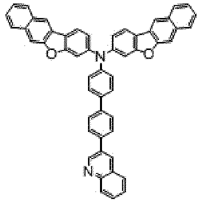 | 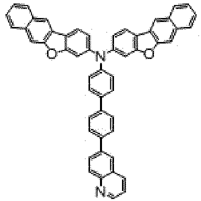 | 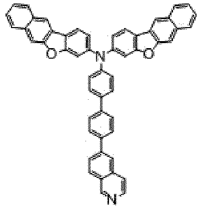 |
| **28** | **29** | **30** |
| 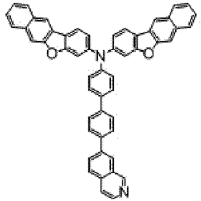 | 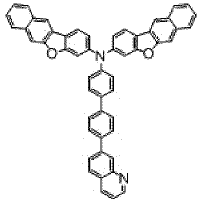 | 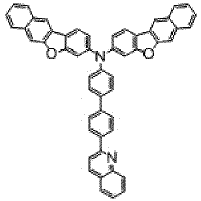 |
| **31** | **32** | **33** |
| 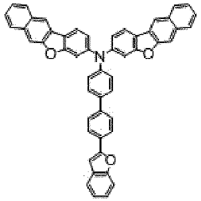 | 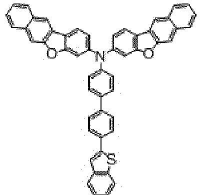 | 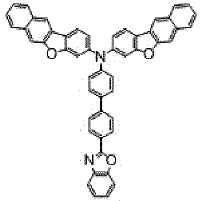 |
| **34** | **35** | **36** |
| 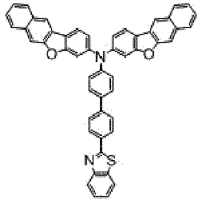 | 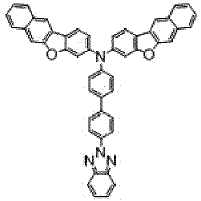 | 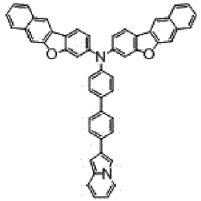 |
| **37** | **38** | **39** |
| 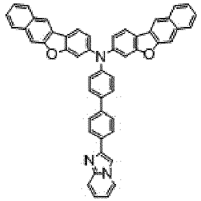 | 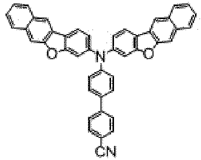 | 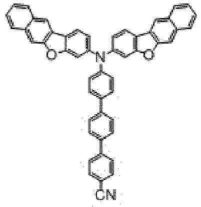 |
| **40** | **41** | **42** |
| 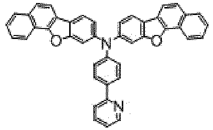 | 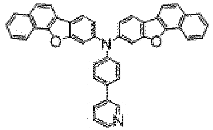 | 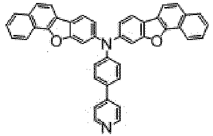 |
| **43** | **44** | **45** |
| 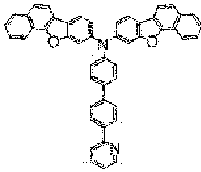 | 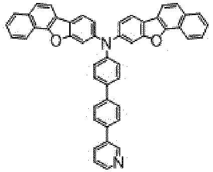 | 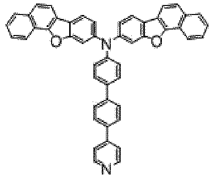 |
| **46** | **47** | **48** |
| 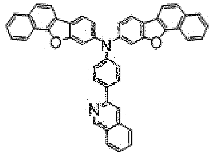 | 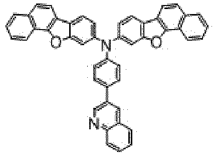 | 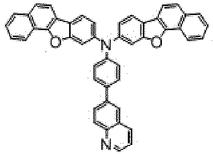 |
| **49** | **50** | **51** |
| 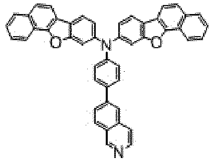 | 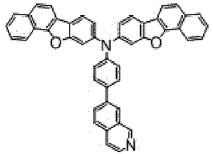 | 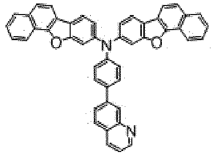 |
| **52** | **53** | **54** |
| 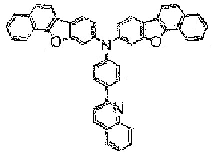 | 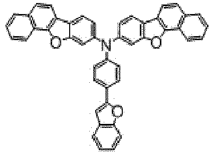 | 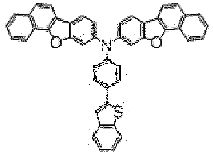 |
| **55** | **56** | **57** |
| 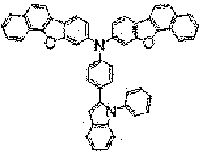 | 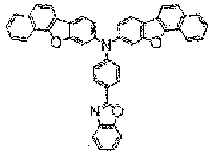 | 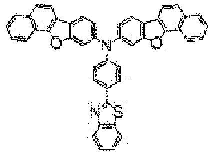 |
| **58** | **59** | **60** |
| 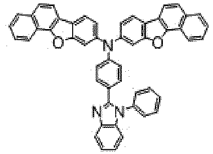 | 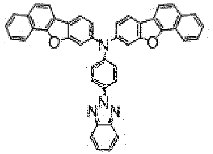 | 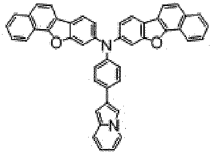 |
| **61** | **62** | **63** |
| 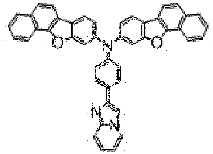 | 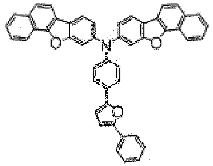 | 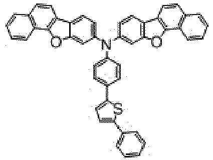 |
| **64** | **65** | **66** |
| 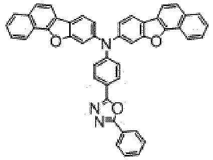 | 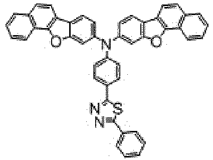 | 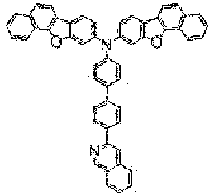 |
| **67** | **68** | **69** |
| 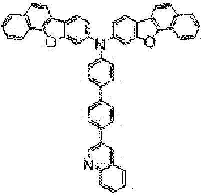 | 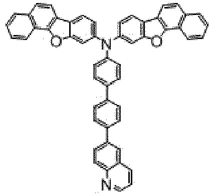 | 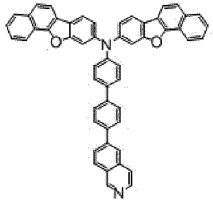 |
| **70** | **71** | **72** |
| 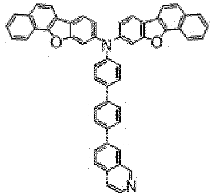 | 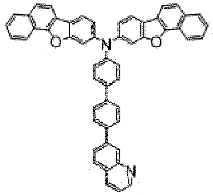 | 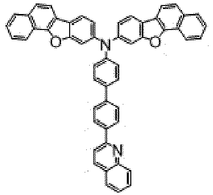 |
| **73** | **74** | **75** |
| 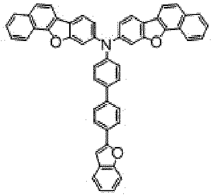 | 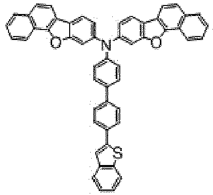 | 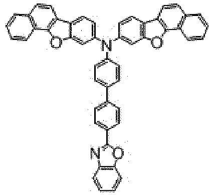 |
| **76** | **77** | **78** |
| 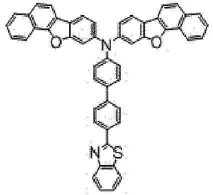 | 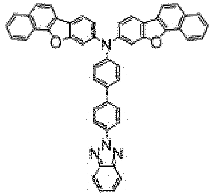 | 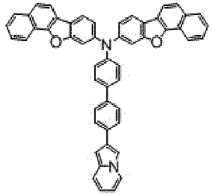 |
| **79** | **80** | **81** |
| 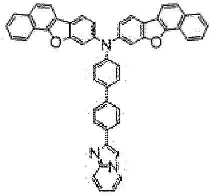 | 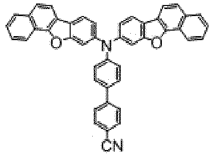 | 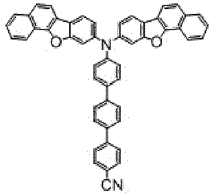 |
| **82** | **83** | **84** |
| 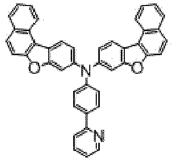 | 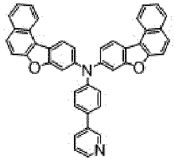 | 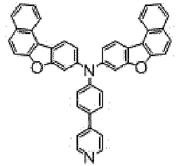 |
| **85** | **86** | **87** |
| 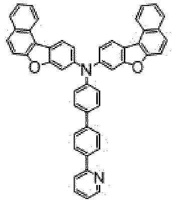 | 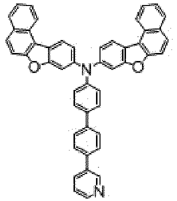 | 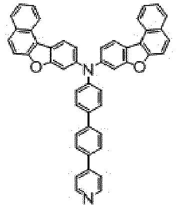 |
| **88** | **89** | **90** |
| 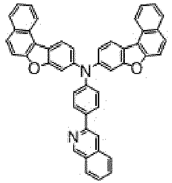 | 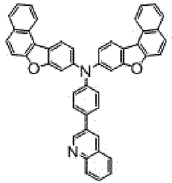 | 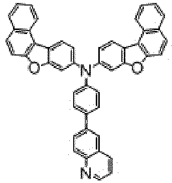 |
| **91** | **92** | **93** |
| 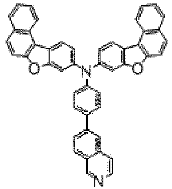 | 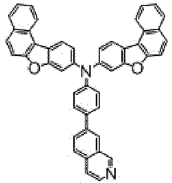 | 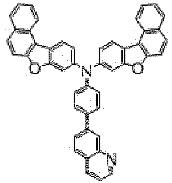 |
| **94** | **95** | **96** |
| 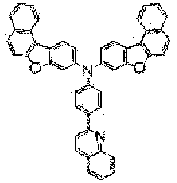 | 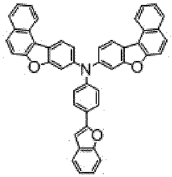 | 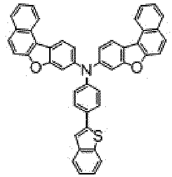 |
| **97** | **98** | **99** |
| 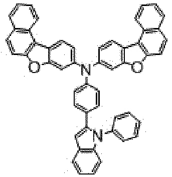 | 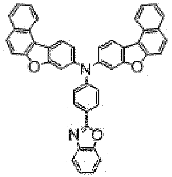 | 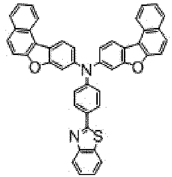 |
| **100** | **101** | **102** |
| 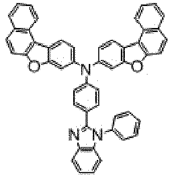 | 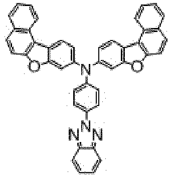 | 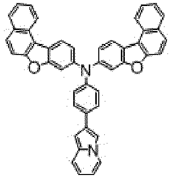 |
| **103** | **104** | **105** |
| 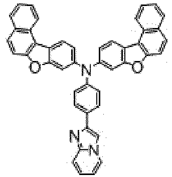 | 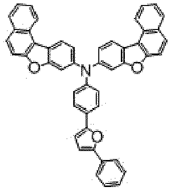 | 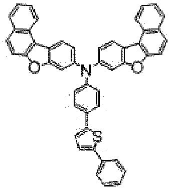 |
| **106** | **107** | **108** |
| 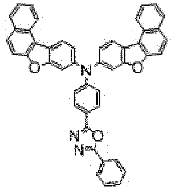 | 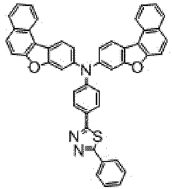 | 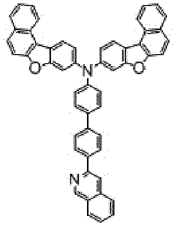 |
| **109** | **110** | **111** |
| 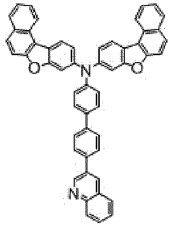 | 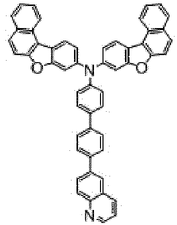 | 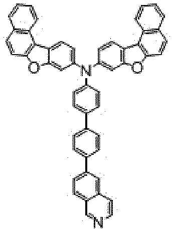 |
| **112** | **113** | **114** |
| 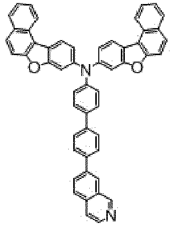 | 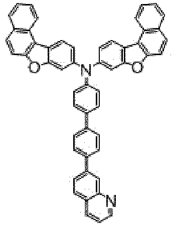 | 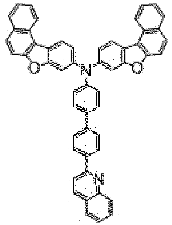 |
| **115** | **116** | **117** |
| 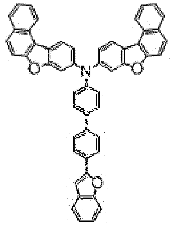 | 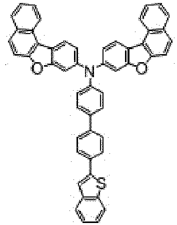 | 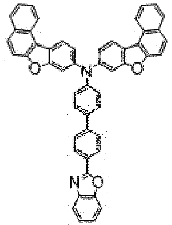 |
| **118** | **119** | **120** |
| 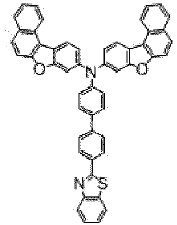 | 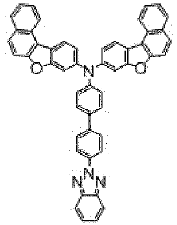 | 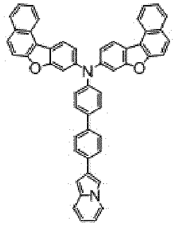 |
| **121** | **122** | **123** |
| 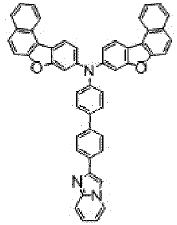 | 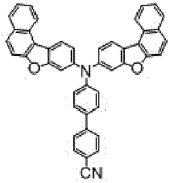 | 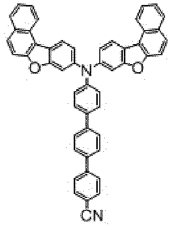 |
| **124** | **125** | **126** |
| 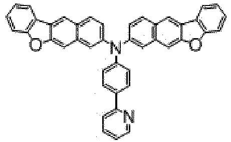 | 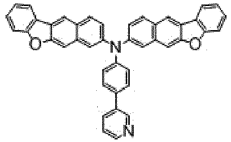 | 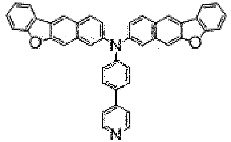 |
| **127** | **128** | **129** |
| 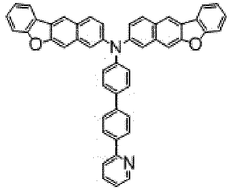 | 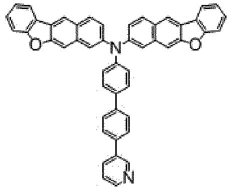 | 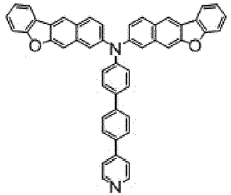 |
| **130** | **131** | **132** |
| 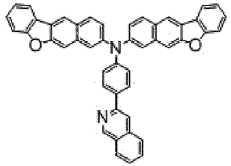 | 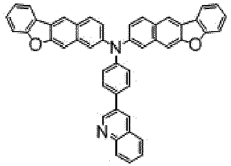 | 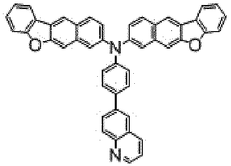 |
| **133** | **134** | **135** |
| 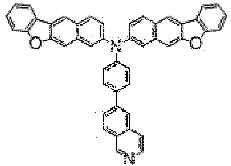 | 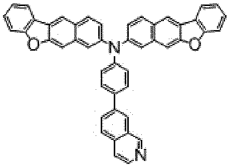 | 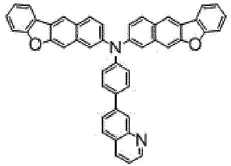 |
| **136** | **137** | **138** |
| 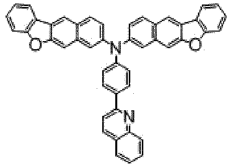 | 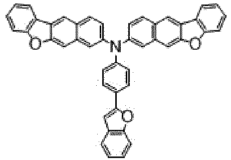 | 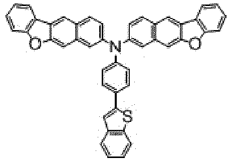 |
| **139** | **140** | **141** |
| 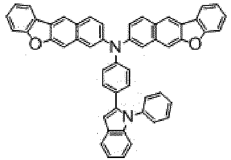 | 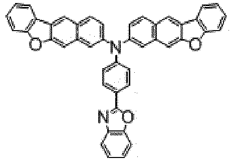 | 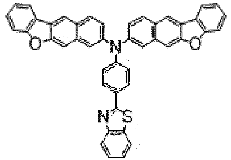 |
| **142** | **143** | **144** |
| 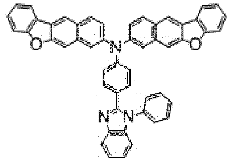 | 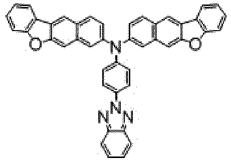 | 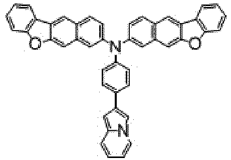 |
| **145** | **146** | **147** |
| 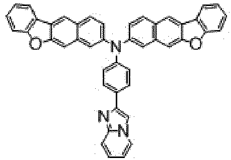 | 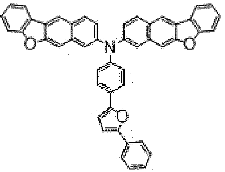 | 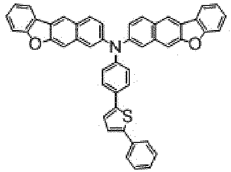 |
| **148** | **149** | **150** |
| 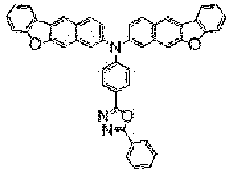 | 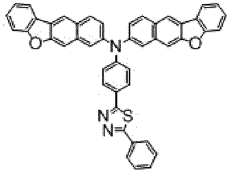 | 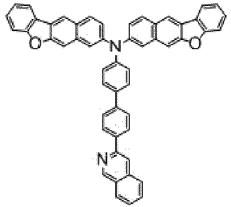 |
| **151** | **152** | **153** |
| 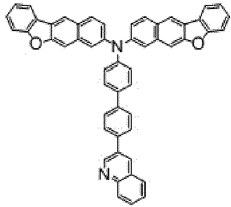 | 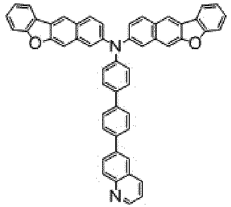 | 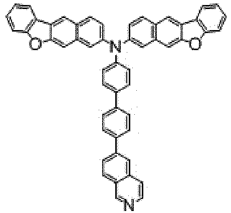 |
| **154** | **155** | **156** |
| 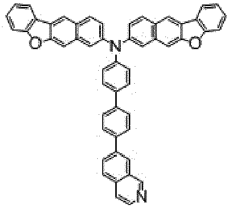 | 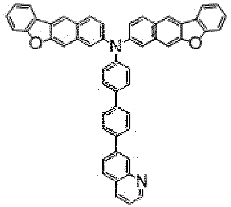 | 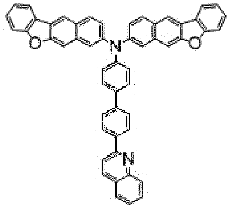 |
| **157** | **158** | **159** |
| 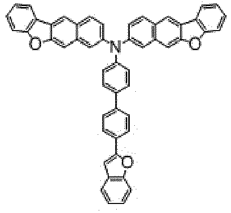 | 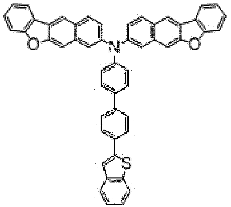 | 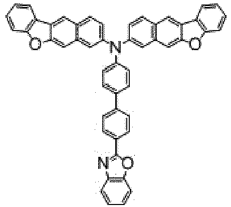 |
| **160** | **161** | **162** |
| 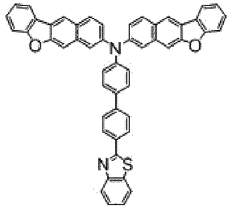 | 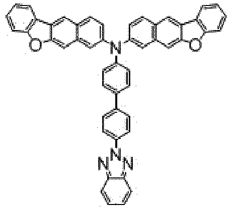 | 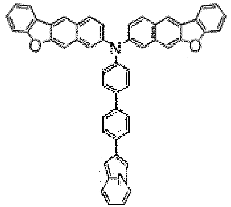 |
| **163** | **164** | **165** |
| 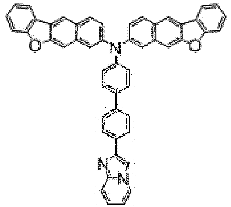 | 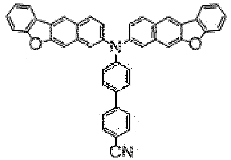 | 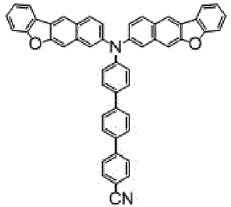 |
| **166** | **167** | **168** |
| 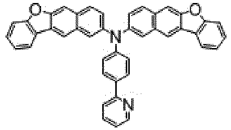 | 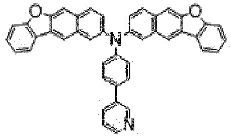 | 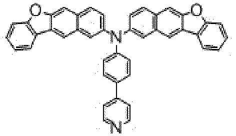 |
| **169** | **170** | **171** |
| 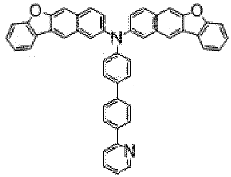 | 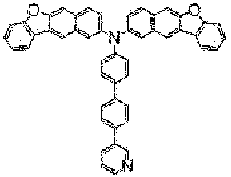 | 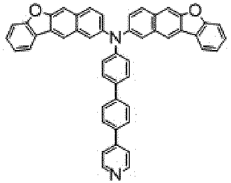 |
| **172** | **173** | **174** |
| 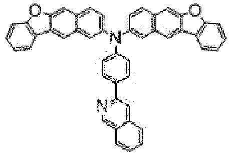 | 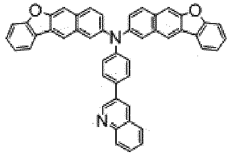 | 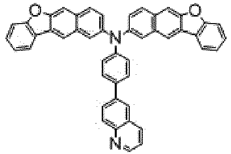 |
| **175** | **176** | **177** |
| 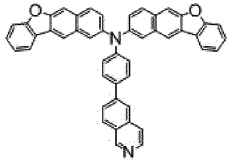 | 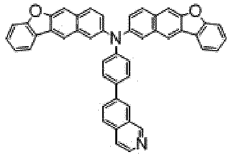 | 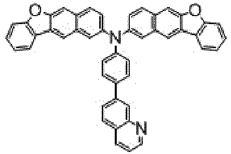 |
| **178** | **179** | **180** |
| 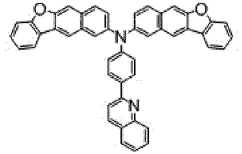 | 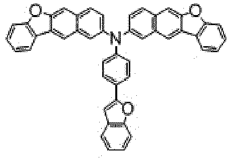 | 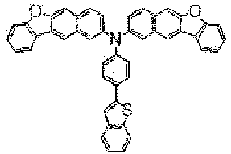 |
| **181** | **182** | **183** |
| 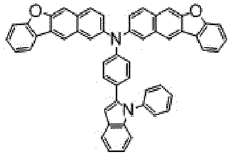 | 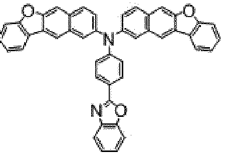 | 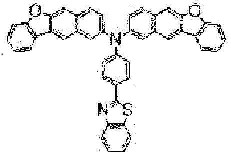 |
| **184** | **185** | **186** |
| 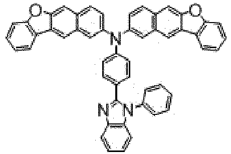 | 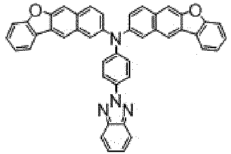 | 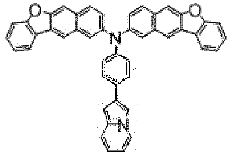 |
| **187** | **188** | **189** |
| 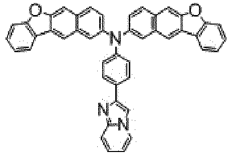 | 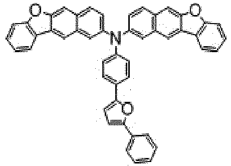 | 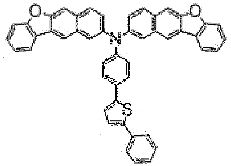 |
| **190** | **191** | **192** |
| 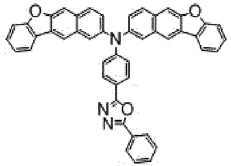 | 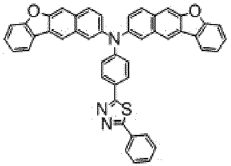 | 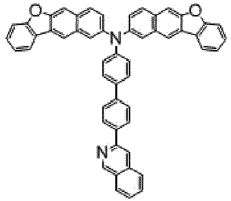 |
| **193** | **194** | **195** |
| 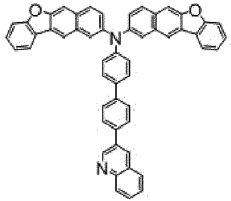 | 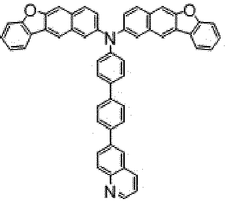 | 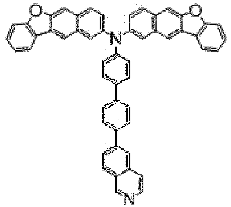 |
| **196** | **197** | **198** |
| 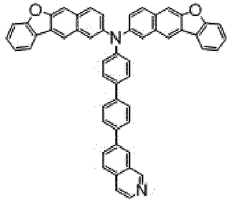 | 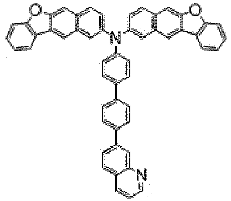 | 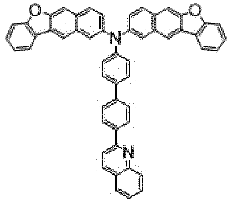 |
| **199** | **200** | **201** |
| 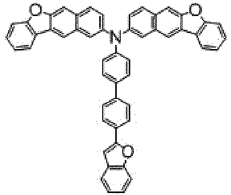 | 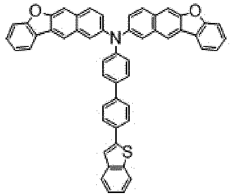 | 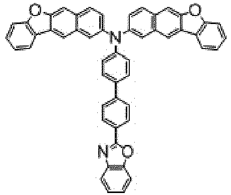 |
| **202** | **203** | **204** |
| 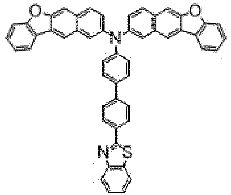 | 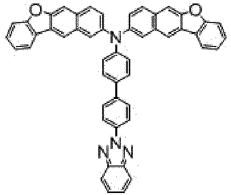 | 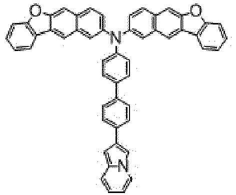 |
| **205** | **206** | **207** |
| 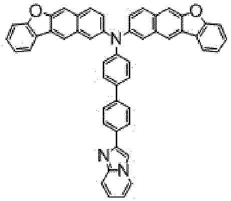 | 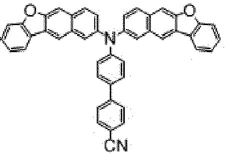 | 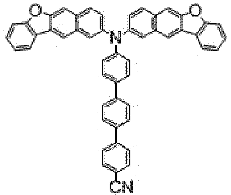 |
| **208** | **209** | **210** |
| 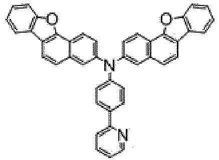 | 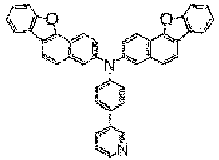 | 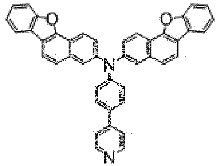 |
| **211** | **212** | **213** |
| 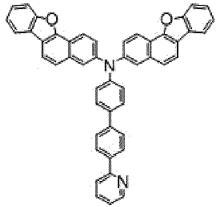 | 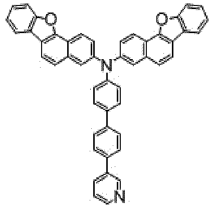 | 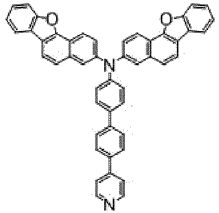 |
| **214** | **215** | **216** |
| 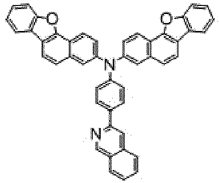 | 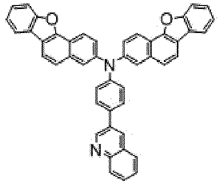 | 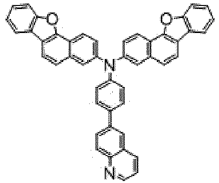 |
| **217** | **218** | **219** |
| 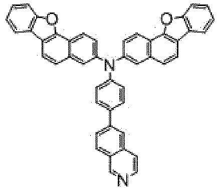 | 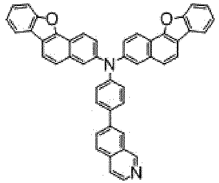 | 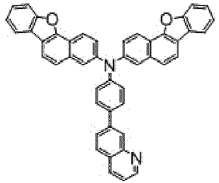 |
| **220** | **221** | **222** |
| 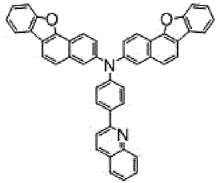 | 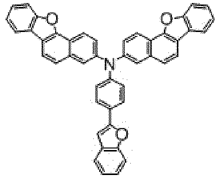 | 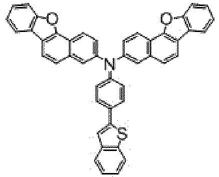 |
| **223** | **224** | **225** |
| 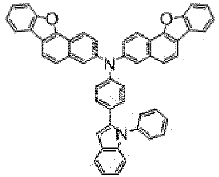 | 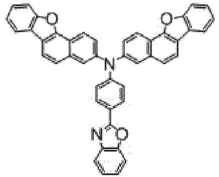 | 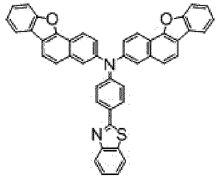 |
| **226** | **227** | **228** |
| 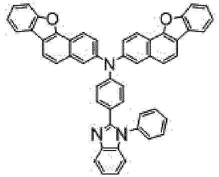 | 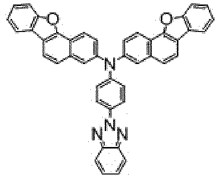 | 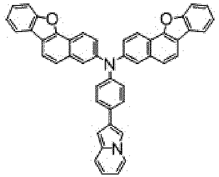 |
| **229** | **230** | **231** |
| 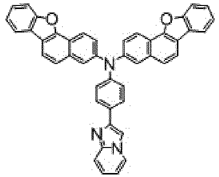 | 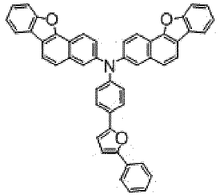 | 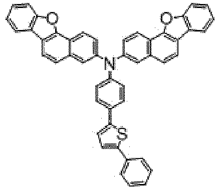 |
| **232** | **233** | **234** |
| 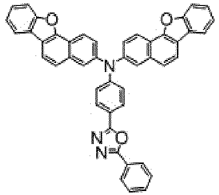 | 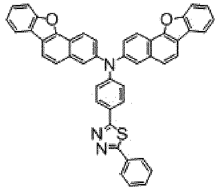 | 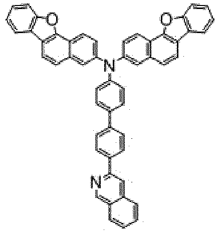 |
| **235** | **236** | **237** |
| 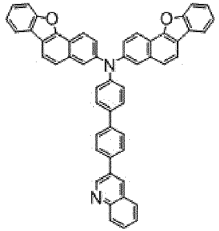 | 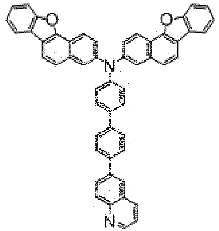 | 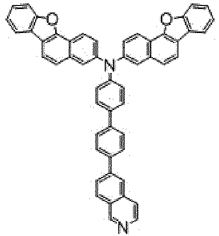 |
| **238** | **239** | **240** |
| 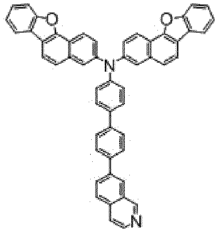 | 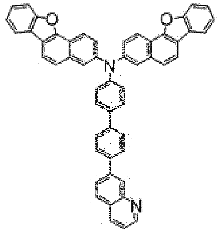 | 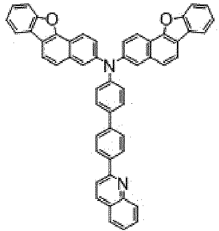 |
| **241** | **242** | **243** |
| 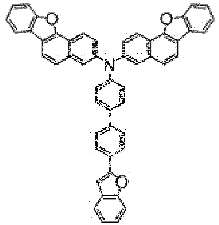 | 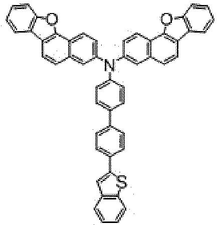 | 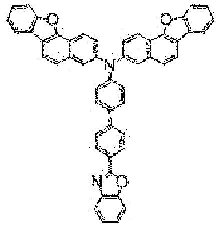 |
| **244** | **245** | **246** |
| 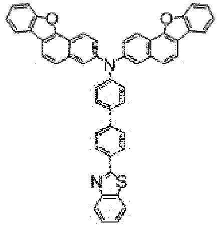 | 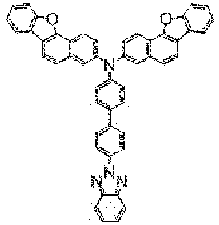 | 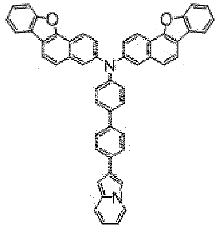 |
| **247** | **248** | **249** |
| 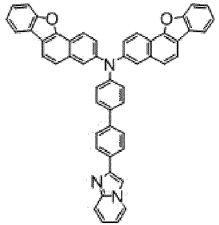 | 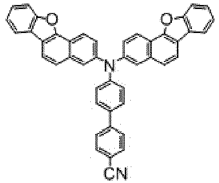 | 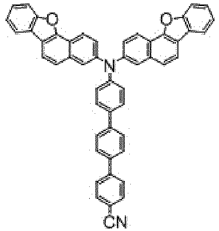 |
| **250** | **251** | **252** |
| 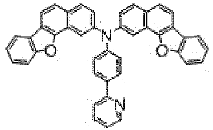 | 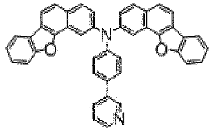 | 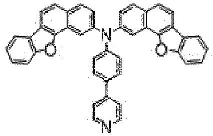 |
| **253** | **254** | **255** |
| 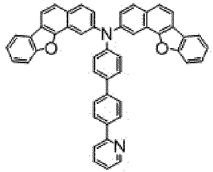 | 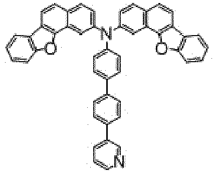 | 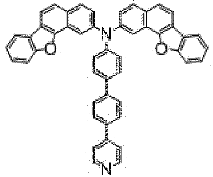 |
| **256** | **257** | **258** |
| 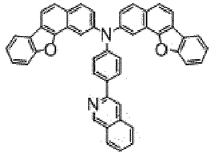 | 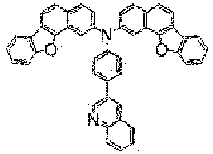 | 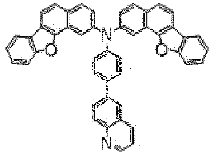 |
| **259** | **260** | **261** |
| 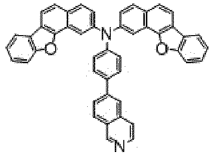 | 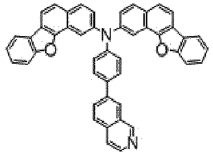 | 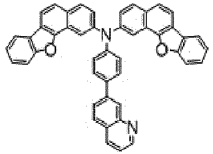 |
| **262** | **263** | **264** |
| 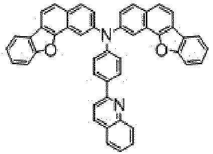 | 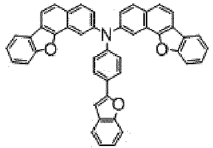 | 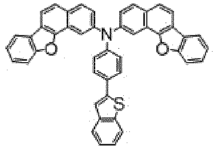 |
| **265** | **266** | **267** |
| 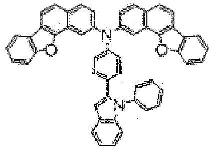 | 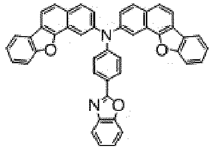 | 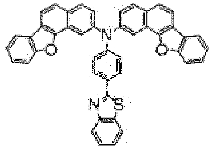 |
| **268** | **269** | **270** |
| 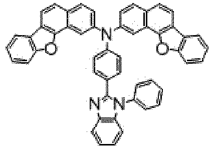 | 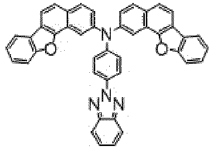 | 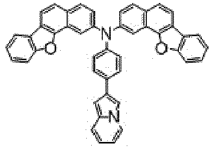 |
| **271** | **272** | **273** |
| 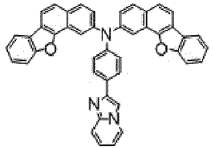 | 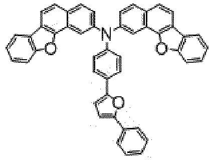 | 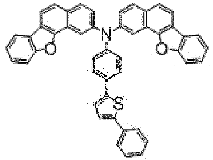 |
| **274** | **275** | **276** |
| 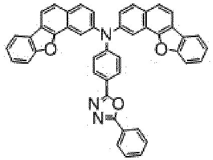 | 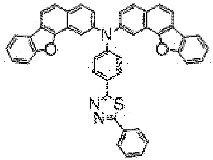 | 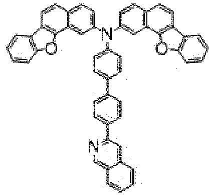 |
| **277** | **278** | **279** |
| 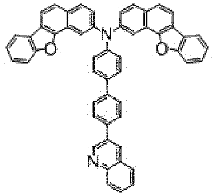 | 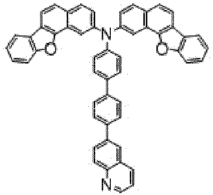 | 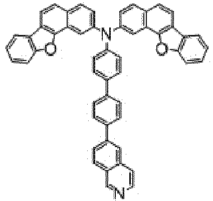 |
| **280** | **281** | **282** |
| 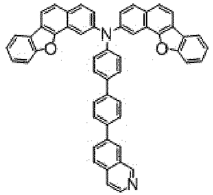 | 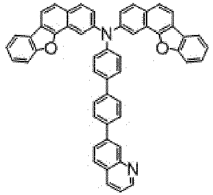 | 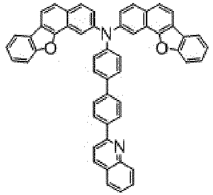 |
| **283** | **294** | **285** |
| 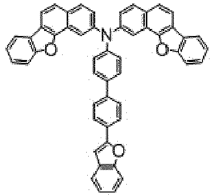 | 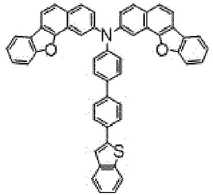 | 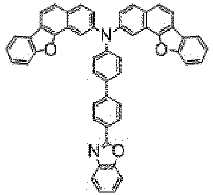 |
| **286** | **287** | **288** |
| 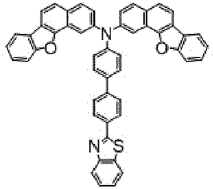 | 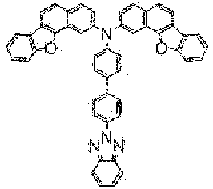 | 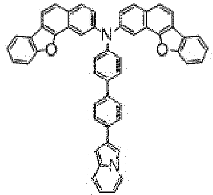 |
| **289** | **290** | **291** |
| 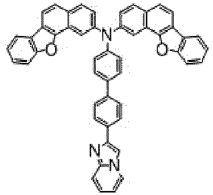 | 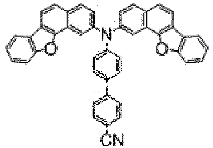 | 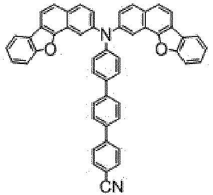 |
| **292** | **293** | **294** |
| 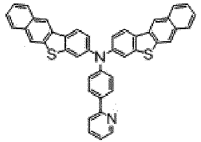 | 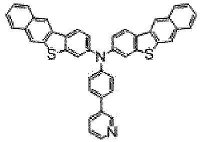 | 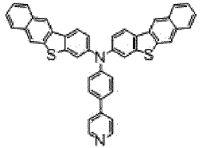 |
| **295** | **296** | **297** |
| 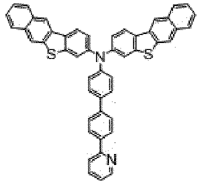 | 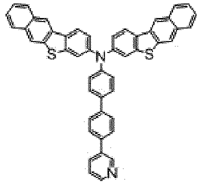 | 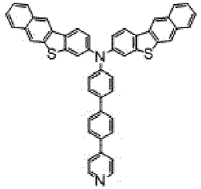 |
| **298** | **299** | **300** |
| 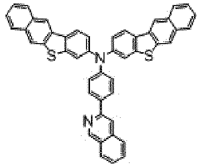 | 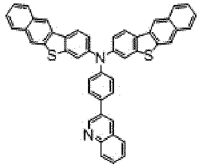 | 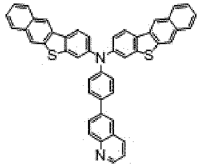 |
| **301** | **302** | **303** |
| 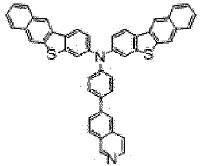 | 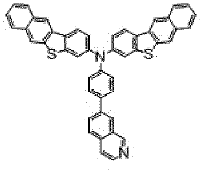 | 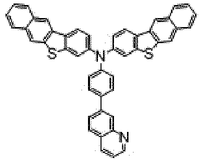 |
| **304** | **305** | **306** |
| 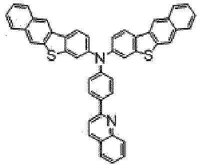 | 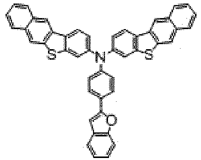 | 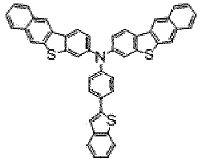 |
| **307** | **308** | **309** |
| 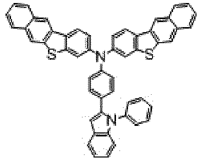 | 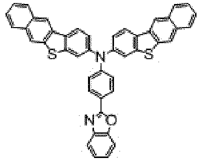 | 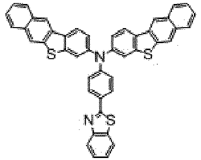 |
| **310** | **311** | **312** |
| 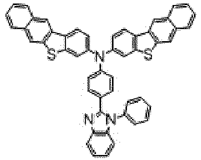 | 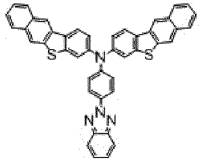 | 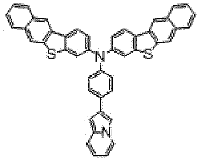 |
| **313** | **314** | **315** |
| 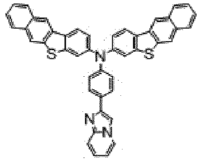 | 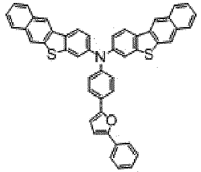 | 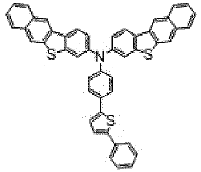 |
| **316** | **317** | **318** |
| 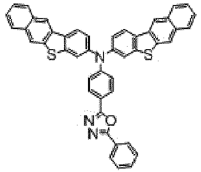 | 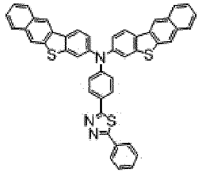 | 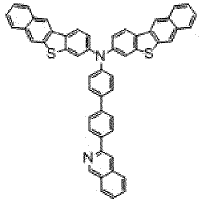 |
| **319** | **320** | **321** |
| 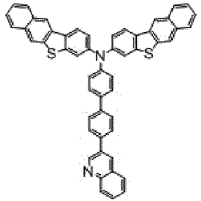 | 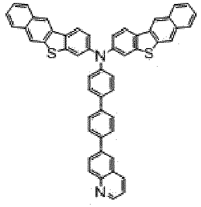 | 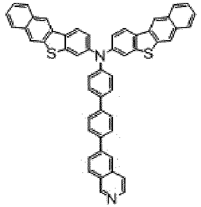 |
| **322** | **323** | **324** |
| 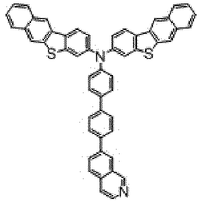 | 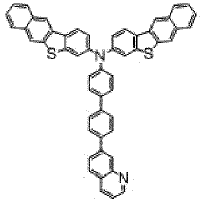 | 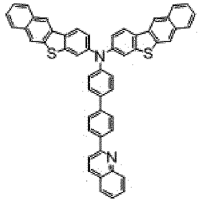 |
| **325** | **326** | **327** |
| 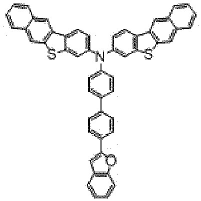 | 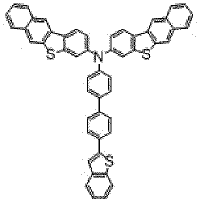 | 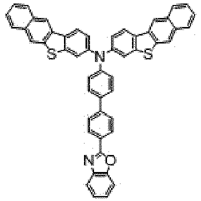 |
| **328** | **329** | **330** |
| 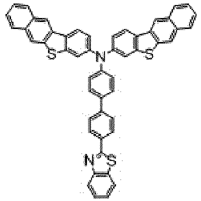 | 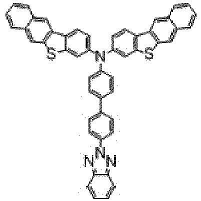 | 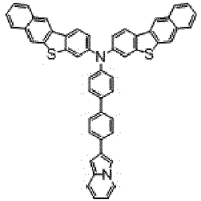 |
| **331** | **332** | **333** |
| 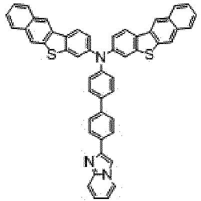 | 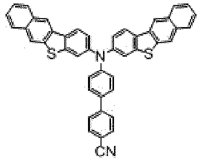 | 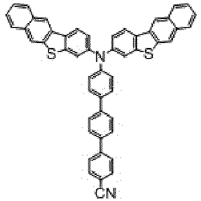 |
| **334** | **335** | **336** |
| 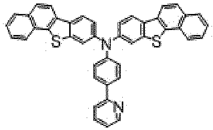 | 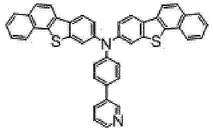 | 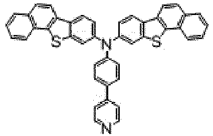 |
| **337** | **338** | **339** |
| 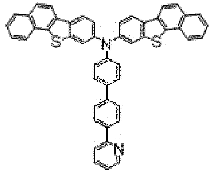 | 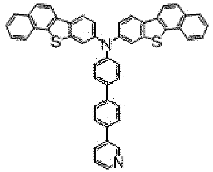 | 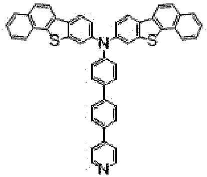 |
| **340** | **341** | **342** |
| 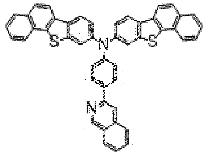 | 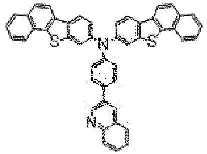 | 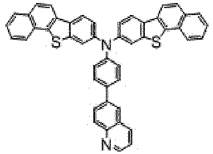 |
| **343** | **344** | **345** |
| 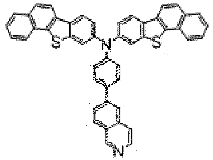 | 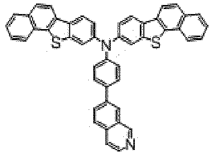 | 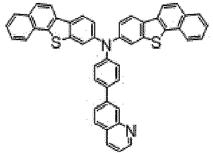 |
| **346** | **347** | **348** |
| 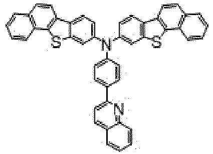 | 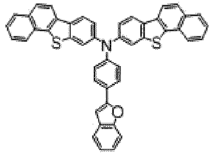 | 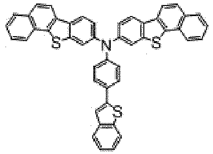 |
| **349** | **350** | **351** |
| 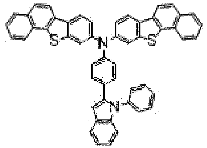 | 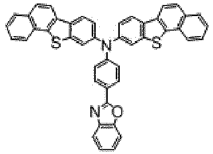 | 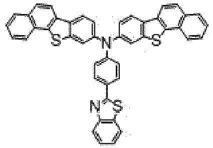 |
| **352** | **353** | **354** |
| 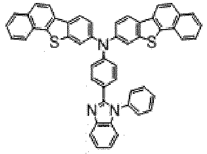 | 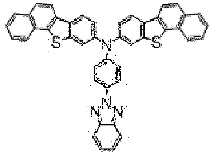 | 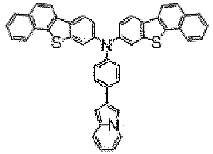 |
| **355** | **356** | **357** |
| 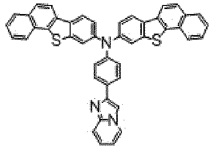 | 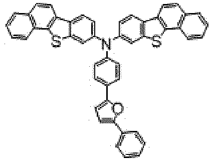 | 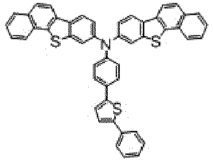 |
| **358** | **359** | **360** |
| 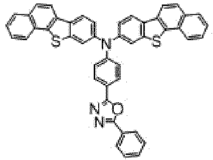 | 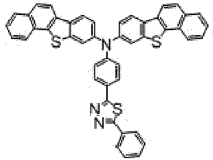 | 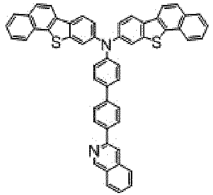 |
| **361** | **362** | **363** |
| 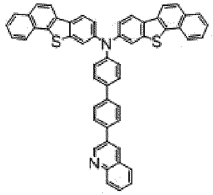 | 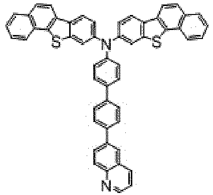 | 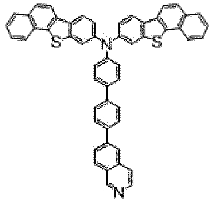 |
| **364** | **365** | **366** |
| 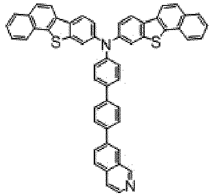 | 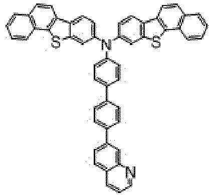 | 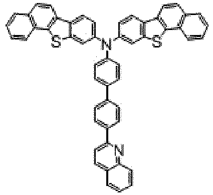 |
| **367** | **368** | **369** |
| 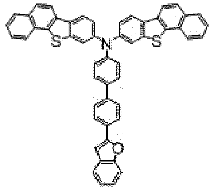 | 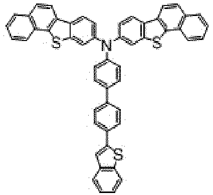 | 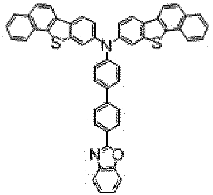 |
| **370** | **371** | **372** |
| 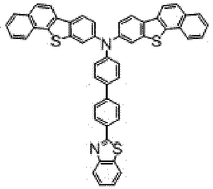 | 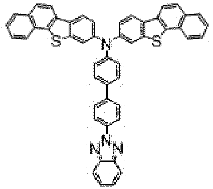 | 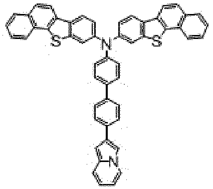 |
| **373** | **374** | **375** |
| 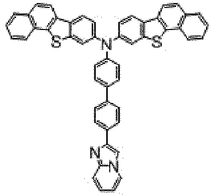 | 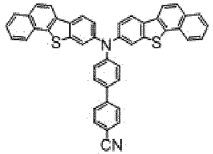 | 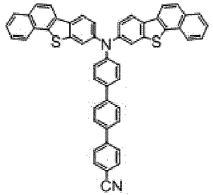 |
| **376** | **377** | **378** |
| 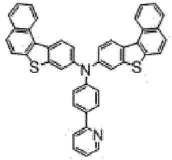 | 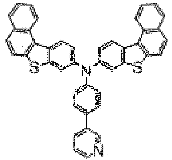 | 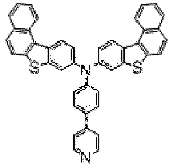 |
| **379** | **380** | **381** |
| 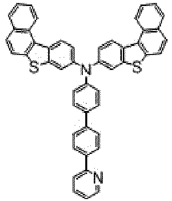 | 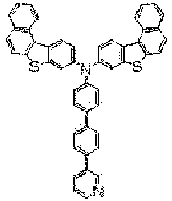 | 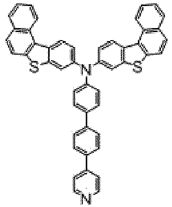 |
| **382** | **383** | **384** |
| 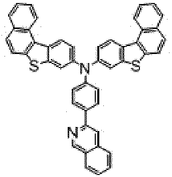 | 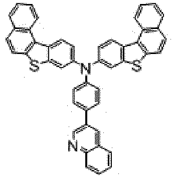 | 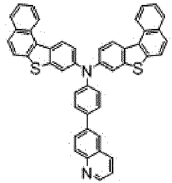 |
| **385** | **386** | **387** |
| 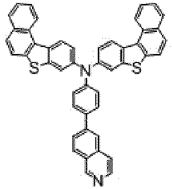 | 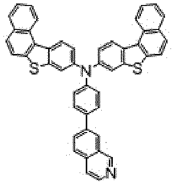 | 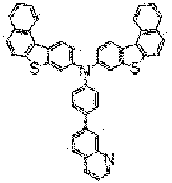 |
| **388** | **389** | **390** |
| 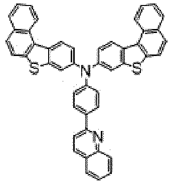 | 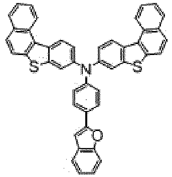 | 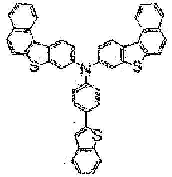 |
| **391** | **392** | **393** |
| 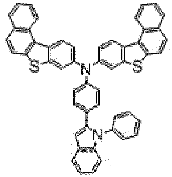 | 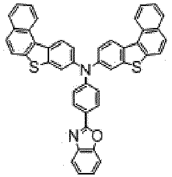 | 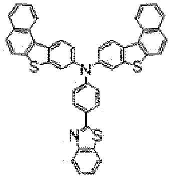 |
| **394** | **395** | **336** |
| 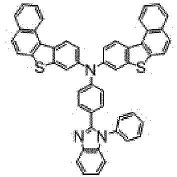 | 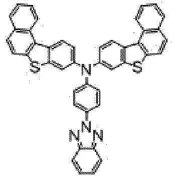 | 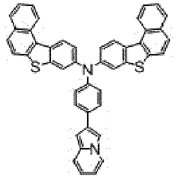 |
| **397** | **398** | **399** |
| 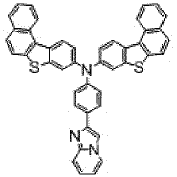 | 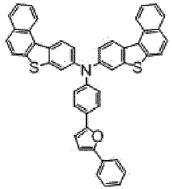 | 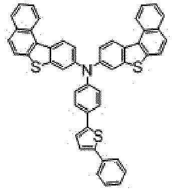 |
| **400** | **401** | **402** |
| 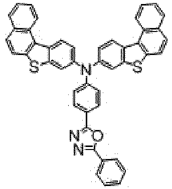 | 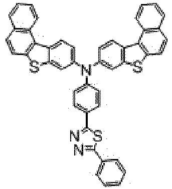 | 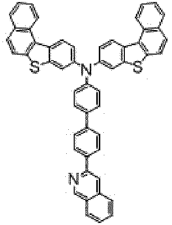 |
| **403** | **404** | **405** |
| 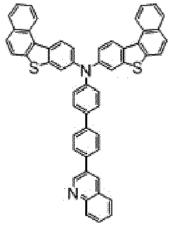 | 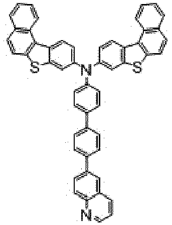 | 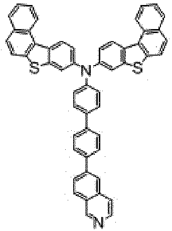 |
| **406** | **407** | **408** |
| 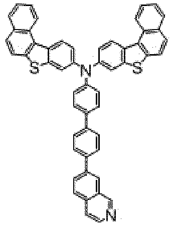 | 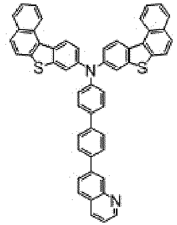 | 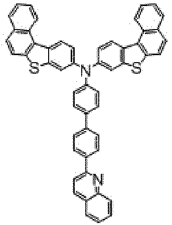 |
| **409** | **410** | **411** |
| 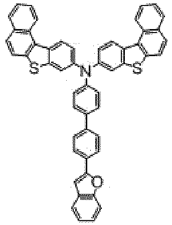 | 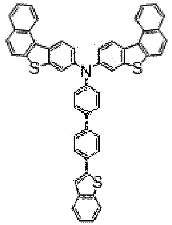 | 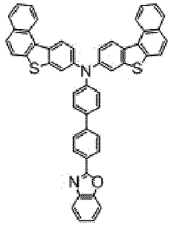 |
| **412** | **413** | **414** |
| 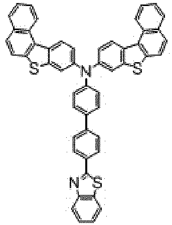 | 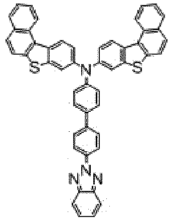 | 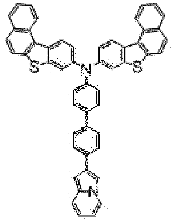 |
| **415** | **416** | **417** |
| 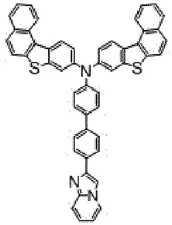 | 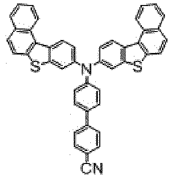 | 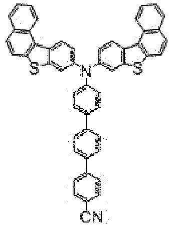 |
| **418** | **419** | **420** |
| 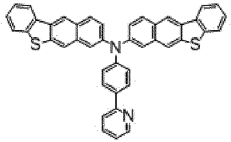 | 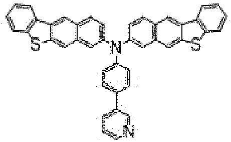 | 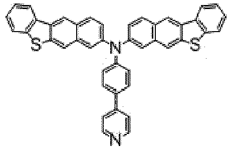 |
| **421** | **422** | **423** |
| 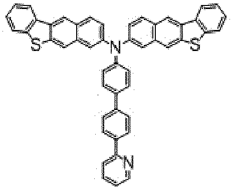 | 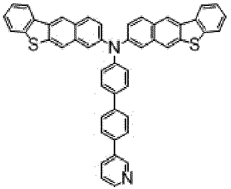 | 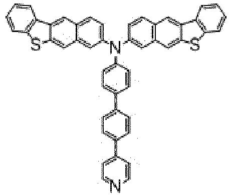 |
| **424** | **425** | **426** |
| 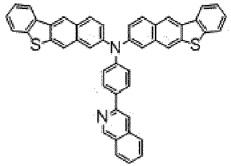 | 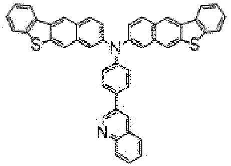 | 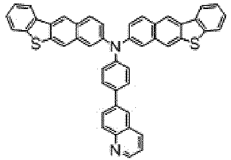 |
| **427** | **428** | **429** |
| 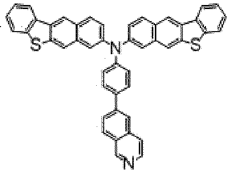 | 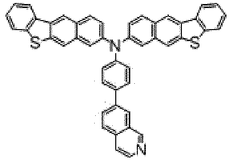 | 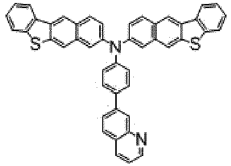 |
| **430** | **431** | **432** |
| 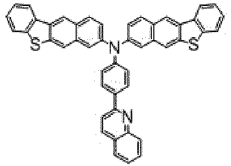 | 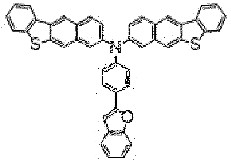 | 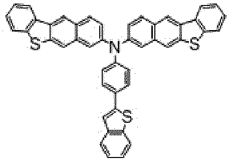 |
| **433** | **434** | **435** |
| 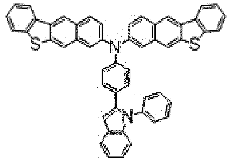 | 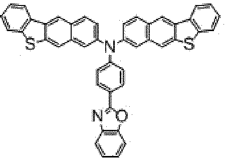 | 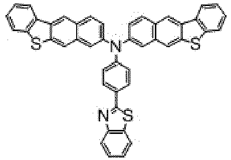 |
| **436** | **437** | **438** |
| 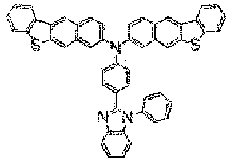 | 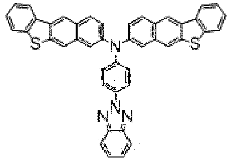 | 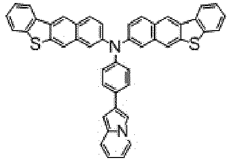 |
| **439** | **440** | **441** |
| 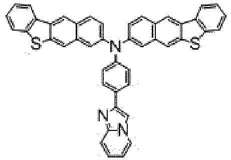 | 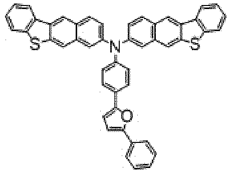 | 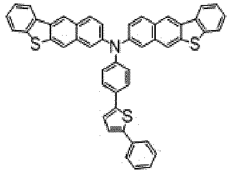 |
| **442** | **443** | **444** |
| 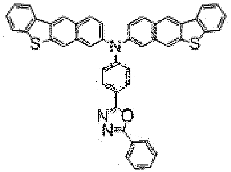 | 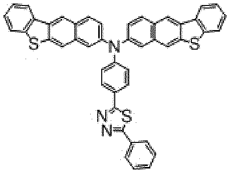 | 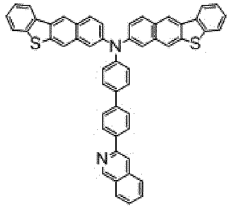 |
| **445** | **446** | **447** |
| 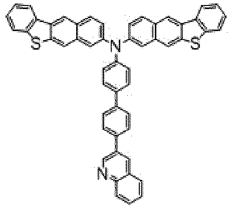 | 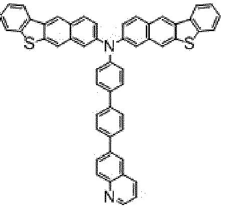 | 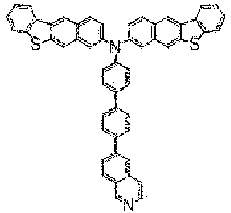 |
| **448** | **449** | **450** |
| 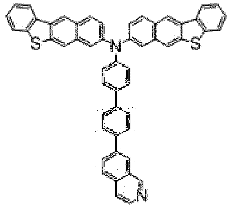 | 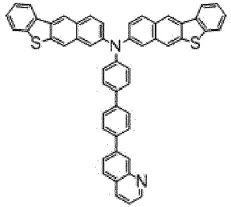 | 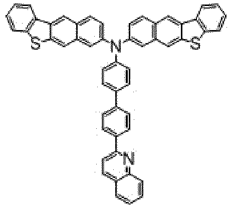 |
| **451** | **452** | **453** |
| 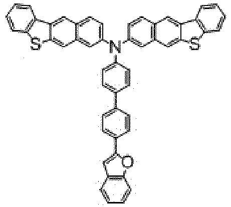 | 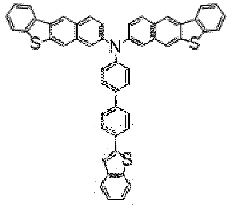 | 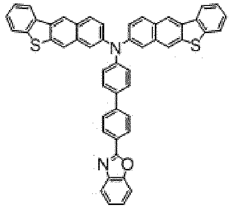 |
| **454** | **455** | **456** |
| 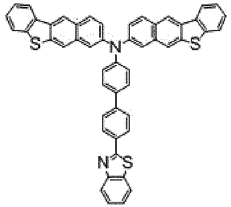 | 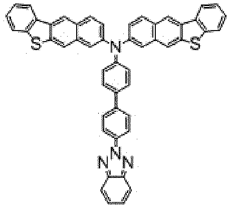 | 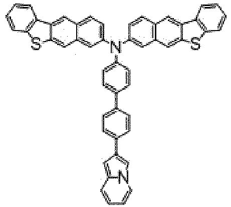 |
| **457** | **458** | **459** |
| 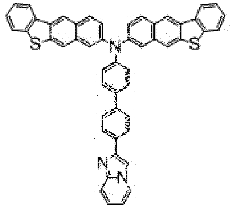 | 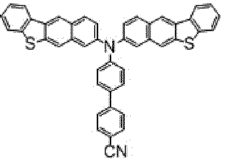 | 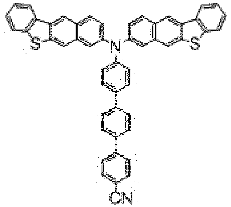 |
| **460** | **461** | **462** |
| 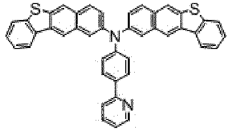 | 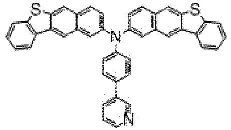 | 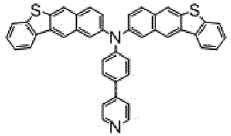 |
| **463** | **464** | **465** |
| 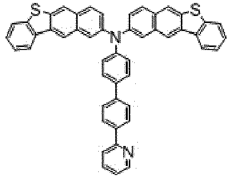 | 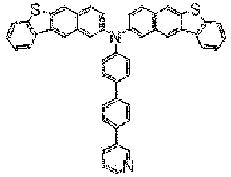 | 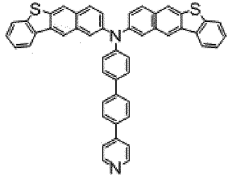 |
| **466** | **467** | **468** |
| 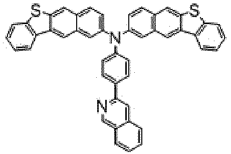 | 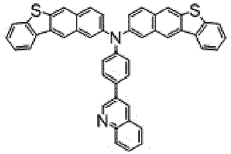 | 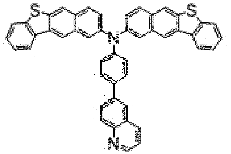 |
| **469** | **470** | **471** |
| 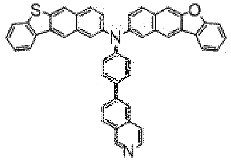 | 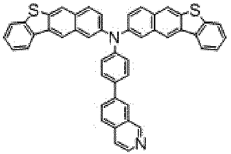 | 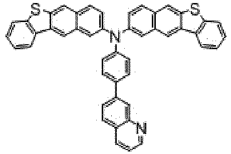 |
| **472** | **473** | **474** |
| 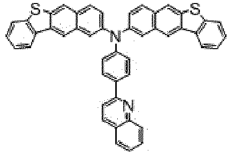 | 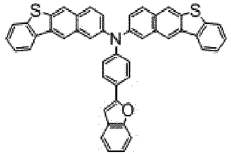 | 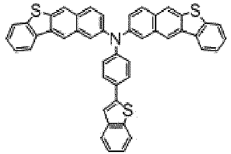 |
| **475** | **476** | **477** |
| 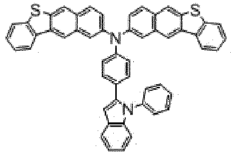 | 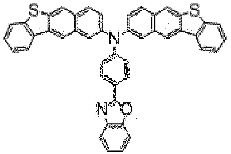 | 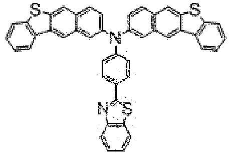 |
| **478** | **479** | **480** |
| 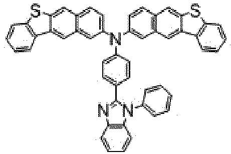 | 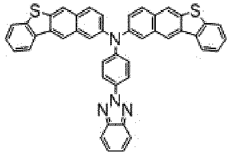 | 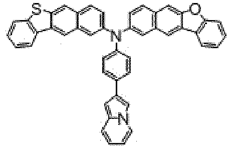 |
| **481** | **482** | **483** |
| 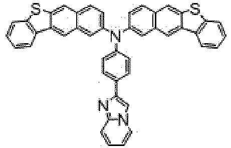 | 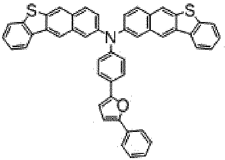 | 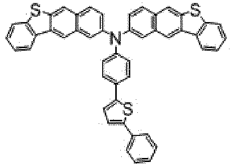 |
| **484** | **485** | **486** |
| 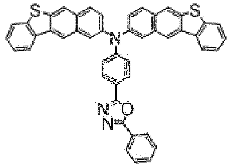 | 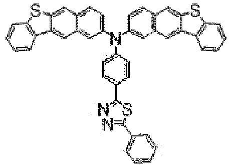 | 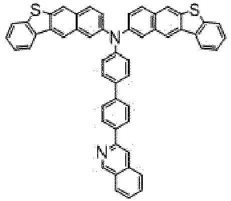 |
| **487** | **488** | **489** |
| 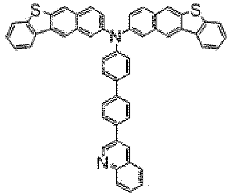 | 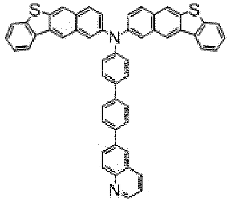 | 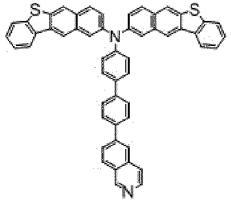 |
| **490** | **491** | **492** |
| 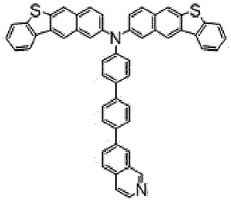 | 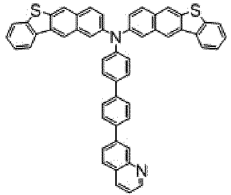 | 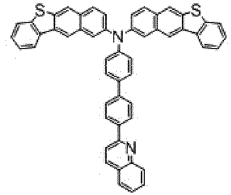 |
| **493** | **494** | **495** |
| 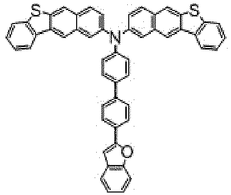 | 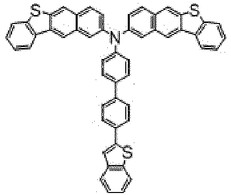 | 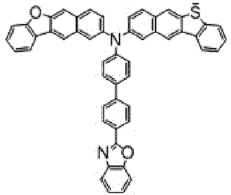 |
| **496** | **497** | **498** |
| 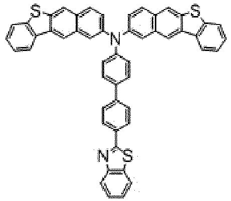 | 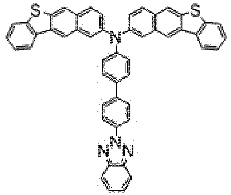 | 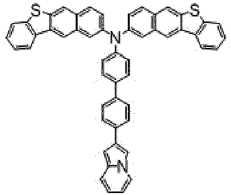 |
| **499** | **500** | **501** |
| 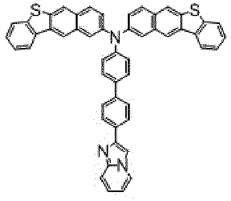 | 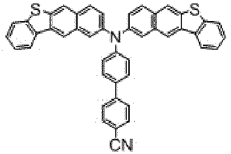 | 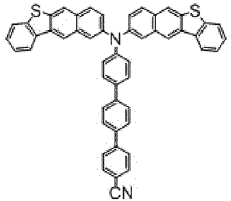 |
| **502** | **503** | **504** |
| 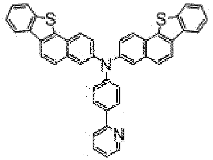 | 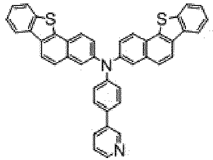 | 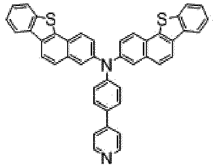 |
| **505** | **506** | **507** |
| 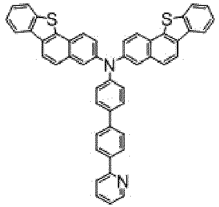 | 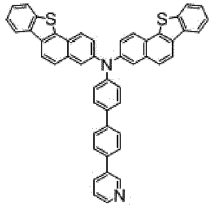 | 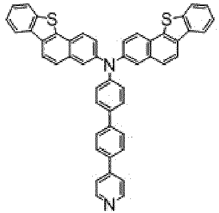 |
| **508** | **509** | **510** |
| 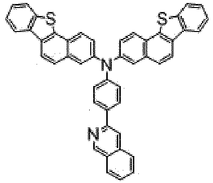 | 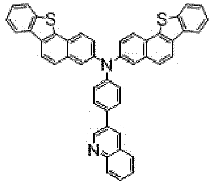 | 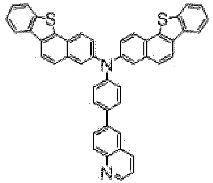 |
| **511** | **512** | **513** |
| 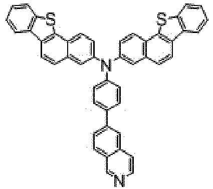 | 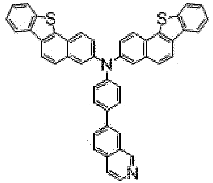 | 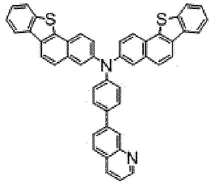 |
| **514** | **515** | **516** |
| 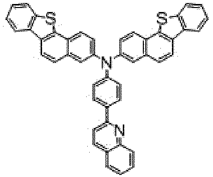 | 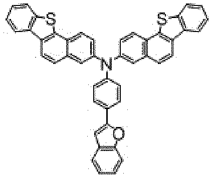 | 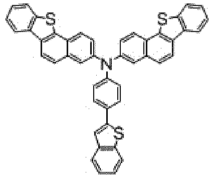 |
| **517** | **518** | **519** |
| 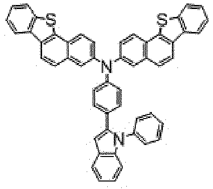 | 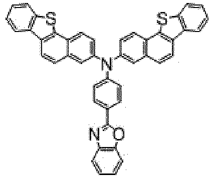 | 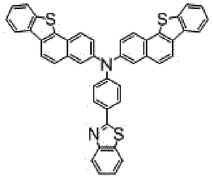 |
| **520** | **521** | **522** |
| 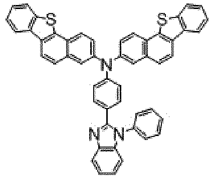 | 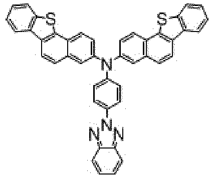 | 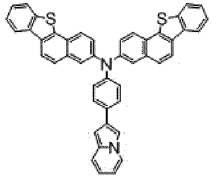 |
| **523** | **524** | **525** |
| 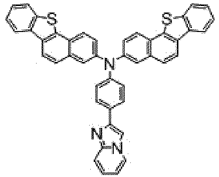 | 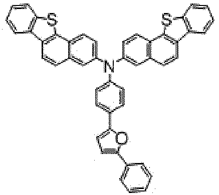 | 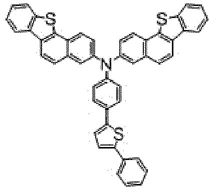 |
| **526** | **527** | **528** |
| 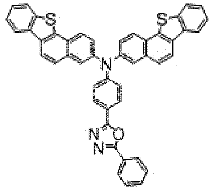 | 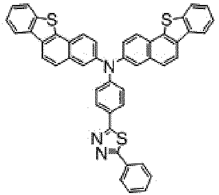 | 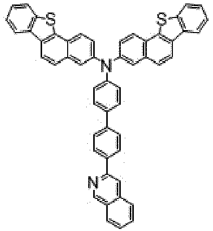 |
| **529** | **530** | **531** |
| 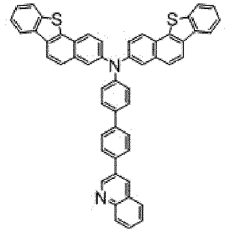 | 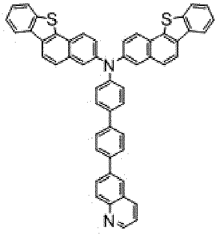 | 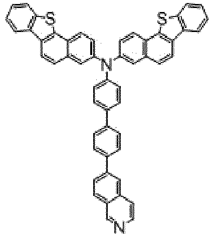 |
| **532** | **533** | **534** |
| 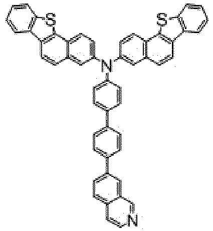 | 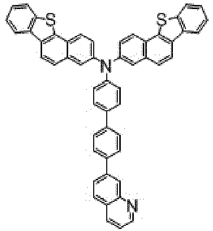 | 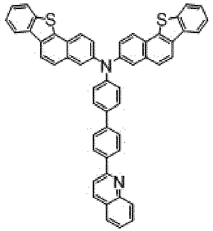 |
| **535** | **536** | **537** |
| 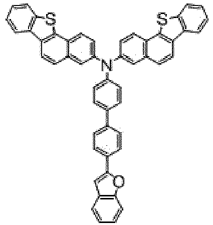 | 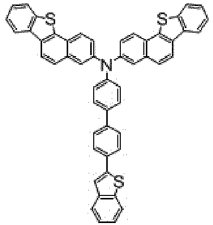 | 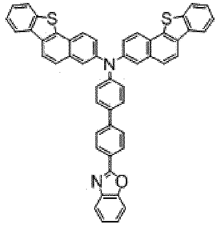 |
| **538** | **539** | **540** |
| 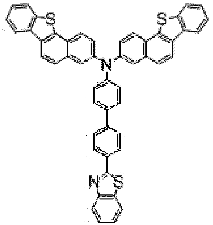 | 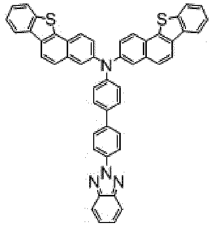 | 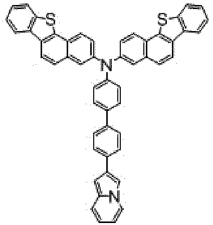 |
| **541** | **542** | **543** |
| 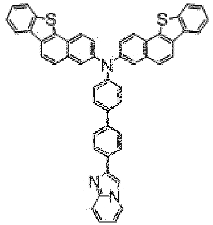 | 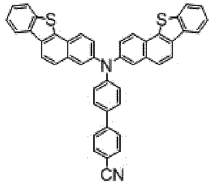 | 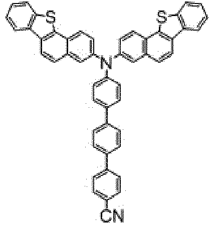 |
| **544** | **545** | **546** |
| 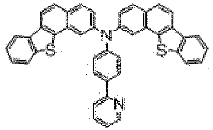 | 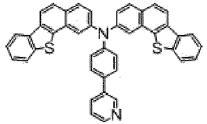 | 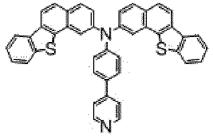 |
| **547** | **548** | **549** |
| 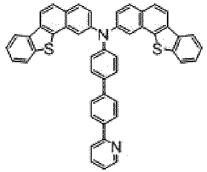 | 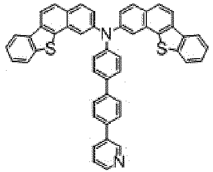 | 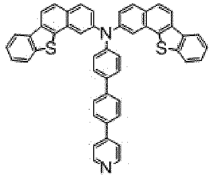 |
| **550** | **551** | **552** |
| 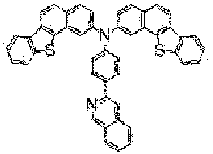 | 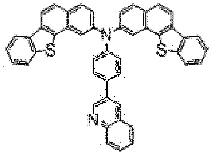 | 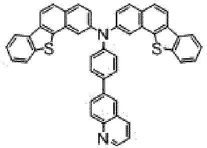 |
| **553** | **554** | **555** |
| 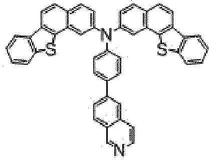 | 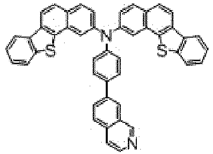 | 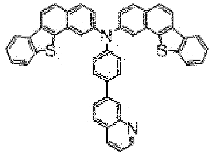 |
| **556** | **557** | **558** |
| 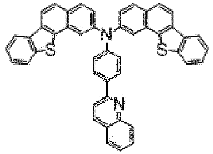 | 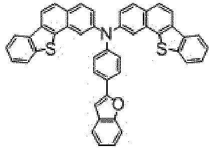 | 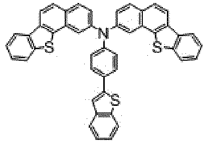 |
| **559** | **560** | **561** |
| 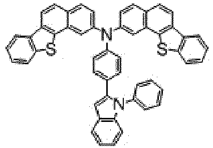 | 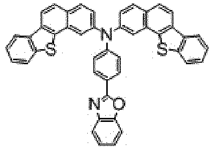 | 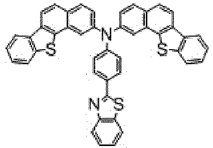 |
| **562** | **563** | **564** |
| 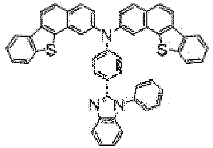 | 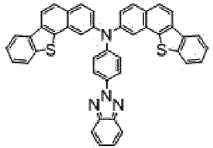 | 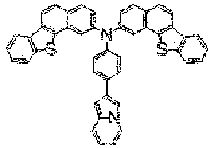 |
| **565** | **566** | **567** |
| 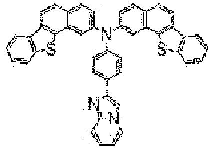 | 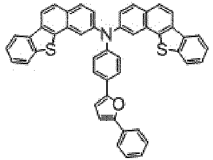 | 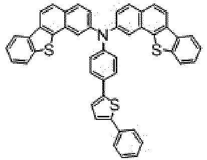 |
| **568** | **569** | **570** |
| 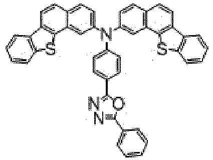 | 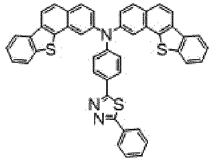 | 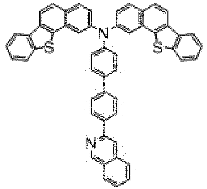 |
| **571** | **572** | **573** |
| 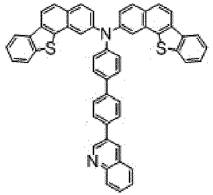 | 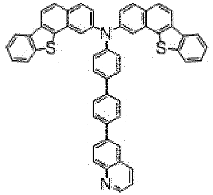 | 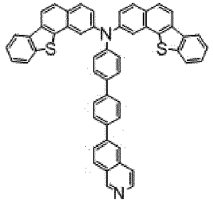 |
| **574** | **575** | **576** |
| 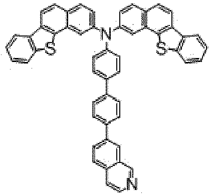 | 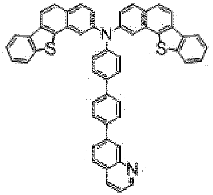 | 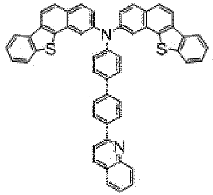 |
| **577** | **578** | **579** |
| 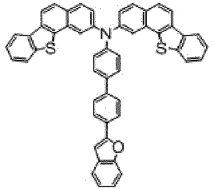 | 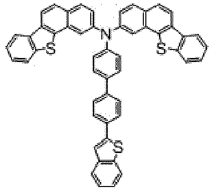 | 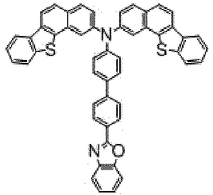 |
| **580** | **581** | **582** |
| 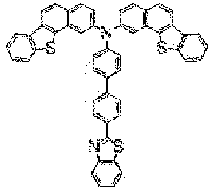 | 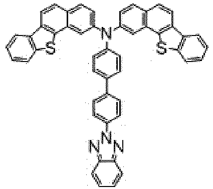 | 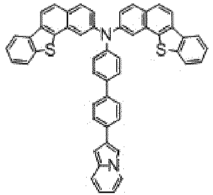 |
| **583** | **584** | **585** |
| 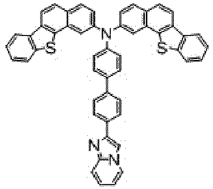 | 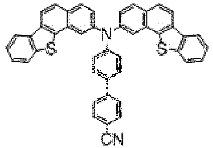 | 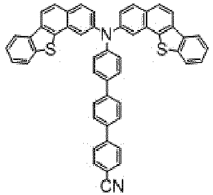 |
| **586** | **587** | **588** |

One example of the capping layer compound of the present invention can be synthesized through an amination reaction, and the schematic reaction formula is as follows.

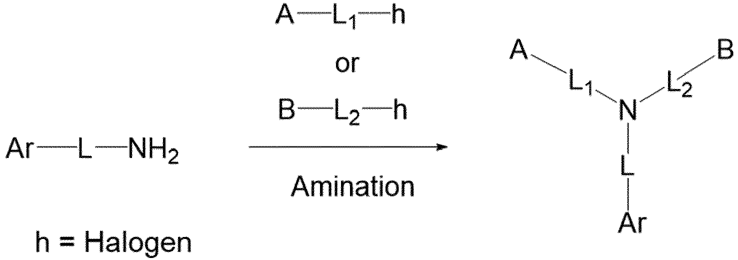

In another embodiment, the present invention provides an organic light emitting device including the capping layer compound according to the present invention described above in a capping layer.

Hereinafter, an organic light emitting device according to one embodiment of the present disclosure will be described in detail.

According to one embodiment of the present disclosure, the organic light emitting device includes a first electrode, a second electrode, one or more organic layers interposed between the first electrode and the second electrode, and a capping layer, in which the capping layer is disposed outside one or more of the first electrode or the second electrode.

Specifically, of both surfaces of each of the first and second electrodes, a surface adjacent to the organic layer interposed between the first electrode and the second electrode is referred to as an inner surface, and a surface not adjacent to the organic material layer is referred to as an outer surface. That is, when the capping layer is disposed on the outer surface of the first electrode, the first electrode is interposed between the capping layer and the organic layer. When the capping layer is disposed on the outer surface of the second electrode, the second electrode is interposed between the capping layer and the organic layer.

According to one embodiment of the present disclosure, in the organic light emitting device, one or more organic layers may be interposed between the first electrode and the second electrode or may be disposed on the outer surface of at least one of the first or second electrodes. That is, the capping layer may be formed on the outer surface of each of the first and second electrodes or may be formed on the outer surface of either one of the first and second electrodes. The capping layer may have a thickness of 100 to 2000 Å.

The capping layer may contain the capping layer compound according to the present disclosure. The capping layer may contain only one compound or two or more compounds, selected from the capping layer compounds of the present disclosure. The capping layer may contain one or more of the capping layer compounds and other known compounds.

Furthermore, the capping layer may have a refractive index of 2.40 or more, more specifically 2.45 or more at a wavelength of 450 nm. In addition, the capping layer may have an ultraviolet absorption intensity of 0.8 or more at 380 nm, more specifically 1.0 or more.

Further, the organic layers may include a hole transport layer, a light emitting layer, and an electron transport layer that constitute a light emitting unit but may not be limited thereto.

More specifically, the organic light emitting device according to one embodiment of the present disclosure includes one or more organic layers between the first electrode (anode) and the second electrode (cathode), in which the organic layers are layers selected from a hole injection layer (HIL), a hole transport layer (HTL), and a light emitting layer (EML), an electron transport layer (ETL), or an electron injection layer (EIL) .

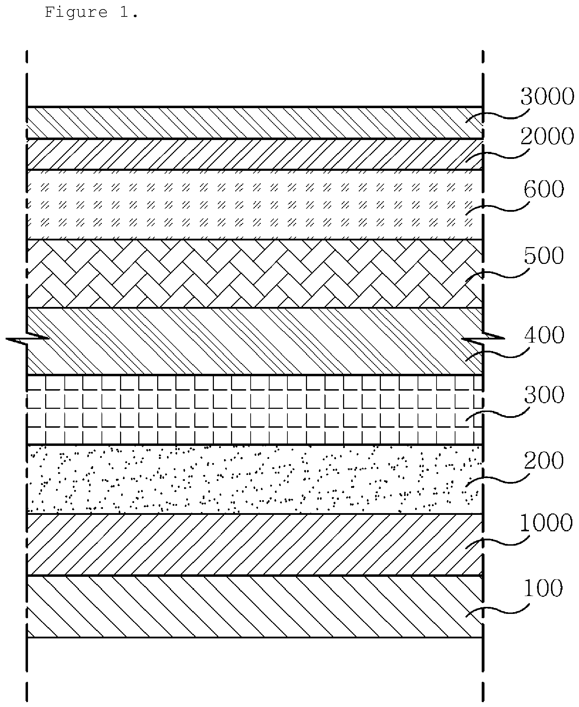

FIG. 1 is a cross-sectional view schematically illustrating the construction of an organic light emitting device according to one embodiment of the present disclosure. The organic light emitting device according to one embodiment of the present disclosure may be manufactured to have a structure illustrated in FIG. 1.

Referring to FIG. 1, the organic light emitting device includes a substrate 100, a first electrode 1000, a hole injection layer 200, a hole transport layer 300, a light emitting layer 400, an electron transport layer 500, an electron injection layer 600, a second electrode 2000, and a capping layer 3000 that are stacked in this order.

Here, as the substrate 100, a substrate that is commonly used for organic light emitting devices may be used. Specifically, a transparent glass substrate or a flexible plastic substrate excellent in mechanical strength, thermal stability, transparency, surface flatness, easy handling, and waterproofness may be used.

In the organic light emitting device, the first electrode 1000 is used as a hole injection electrode for injecting holes.

The first electrode 1000 is made of a material having a low work function to enable hole injection. Specifically, the first electrode 1000 is made of a transparent material such as indium tin oxide (ITO), indium zinc oxide (IZO), or graphene.

The hole injection layer 200 may be formed by depositing a hole injection material on the first electrode 1000 by a method such as a vacuum deposition method, a spin coating method, a casting method, Langmuir-Blodgett (LB), or the like. In the case of using a vacuum deposition method to form the hole injection layer 200, the deposition conditions vary depending on the compound used as the material of the hole injection layer 200, the structure and thermal characteristics of the desired hole injection layer 200, and the like. The conditions are appropriately set to fall within a temperature range of 50°C to 500°C, a vacuum degree range of 10⁻⁸ to 10⁻³ torr, a deposition rate range of 0.01 to 100 Å/sec, and a layer thickness range of 10 Å to 5 um. A charge generating layer may be optionally deposited on the surface of the hole injection layer 200 if necessary. A conventional material may be used as the material of the charge generation layer. For example, HATCN may be used.

The hole transport layer 300 may be formed by depositing a hole transport material on the hole injection layer 200 by a method such as a vacuum deposition method, a spin coating method, a casting method, or LB. In the case of forming the hole transport layer 300 by the vacuum deposition method, the deposition conditions vary depending on the compound used. However, the conditions may be selected from the same ranges described in connection with the hole injection layer 200. The hole transport layer 300 may be formed using a known compound. The hole transport layer 300 may be a single layer structure or a multilayer structure. Although not illustrated in FIG. 1, a light emitting auxiliary layer may be additionally formed on the hole transport layer 300.

The light emitting layer 400 may be formed by depositing a light emitting material on the hole transport layer 300 or the light emitting auxiliary layer by a method such as a vacuum deposition method, a spin coating method, a casting method, or LB. In the case of using a vacuum deposition method to form the light emitting layer 400, the deposition conditions vary depending on the compound used. However, the conditions may be selected from the same ranges as in the deposition conditions of the hole injection layer 200. As the light emitting material, a known compound may be used as a host or a dopant.

Here, in the case where the phosphorescent dopant and the light emitting material are used together, a hole-blocking material (HBL) may be deposited on the light emitting layer 400 by a vacuum deposition method or a spin coating method to prevent triplet excitons or holes from diffusing into the electron transport layer 500. The material that can be used as the hole-blocking material is not particularly limited, and an arbitrary existing material may be selected and used. Examples of the hole blocking material include oxadiazole derivatives, triazole derivatives, phenanthroline derivatives, and hole blocking materials described in Japanese Patent Application Publication No. H11-329734 (A1). By way of example, Balq(bis(8-hydroxy-2-methylquinolinolato)-aluminum biphenoxide), and phenanthrolines-based compounds (for example, Bathocuproine (BCP) available from UDC) may be used. The light emitting layer 400 used in an embodiment of the present disclosure may include one or more blue light emitting layers.

The electron transport layer 500 is formed on the light emitting layer 400 by a vacuum deposition method, a spin coating method, a casting method, or the like. The deposition conditions for the electron transport layer 500 vary depending on the compound used. However, the conditions may be selected from almost the same ranges as in the deposition conditions of the hole injection layer 200.

The electron injection layer 600 is formed by depositing an electron injection material on the electron transport 500 by a vacuum deposition method, a spin coating method, a casting method, or the like.

The second electrode 2000 is used as an electron injection electrode and may be formed on the electron injection layer 600 by a method such as a vacuum deposition method or a sputtering method. Various metals may be used to form the second electrode 2000. Specific examples include, but are not limited to, aluminum(Al), gold(Au), silver(Ag), magnesium(Mg), or the like.

The organic light emitting device according to the present disclosure may be an organic light emitting device including the first electrode 1000, the hole injection layer 200, the hole transport layer 300, the light emitting layer 400, the electron transport layer 500, the electron injection layer 600, the second electrode 2000, and the capping layer 300. The organic light emitting device may further include one or more intermediate layers if necessary.

Further, the thickness of each organic layer formed according to the present disclosure can be adjusted as desired. Specifically, the thickness may fall within a range of from 10 to 1,000 nm and more specifically a range from 20 to 150 nm.

The capping layer 3000 may be formed on the outer surface of the first electrode 1000, of both surfaces of the first electrode 1000. That is, the capping layer 3000 may be formed on the surface that is not adjacent to the hole injection layer 200. In addition, of both surfaces (inner and outer surfaces) of the second electrode 2000, the capping layer 3000 may also be formed on the outer surface of the second electrode 2000. The outer surface of the second electrode 2000 is a surface that is not adjacent to the electron injection layer 600. The capping layer 3000 may be formed by a deposition process, and the capping layer 3000 may have a thickness in a range from 100 Å to 2,000 Å and more specifically a range from 300 Å to 1,000 Å. The thickness is adjusted to prevent a decrease in the transmittance of the capping layer 3000.

In addition, although not illustrated in FIG. 1, according to one embodiment of the present disclosure, organic layers having various functions may be additionally formed between the capping layer 3000 and the first electrode 1000 or between the capping layer 3000 and the second electrode 2000. Alternatively, organic layers having various functions may be additionally formed on the top surface (outer surface) of the capping layer 3000. However, the present disclosure is not limited to the structure described above.

Hereinafter, the present disclosure will be described in greater detail with reference to compound synthesis examples and examples of organic light emitting devices (OLEDs).

The OLED examples and compound synthesis examples described below are presented only for illustrative purposes and the scope of the present disclosure is not limited thereby.

### Synthesis Example 1: Synthesis of Compound 8

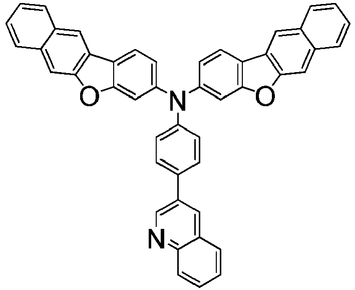

In a round bottom flask, 5.0 g of 3-bromobenzo[b]naphtho[2,3-d]furan, 1.9 g of 4-(quinolin-3-yl)aniline, 2.0 g of t-BuONa, 0.5 g of Pd2(dba)3, and 0.1 ml of (t-Bu)3P were dissolved in 200 ml of toluene and stirred under reflux. The reaction was confirmed by TLC and terminated after adding water. The organic layer was extracted with MC, filtered under reduced pressure, and then column purified and recrystallized to obtain 7.8 g of compound 8 (yield 71%).
m/z: 652.2151 (100.0%), 653.2184 (50.8%), 654.2218 (12.6%), 655.2251 (2.1%)

### Synthesis Example 2: Synthesis of Compound 14

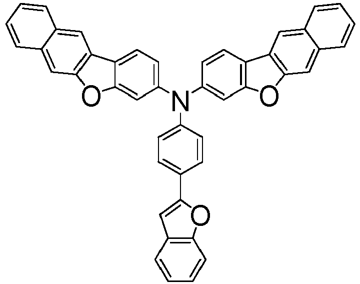

Compound 14 was synthesized using 4-(benzofuran-2-yl)aniline instead of 4-(quinolin-3-yl)aniline in the same manner as in Synthesis Example 1 (yield 73%).
m/z: 641.1991 (100.0%), 642.2024 (49.8%), 643.2058 (12.1%), 644.2092 (1.9%)

### Synthesis Example 3: Synthesis of Compound 15

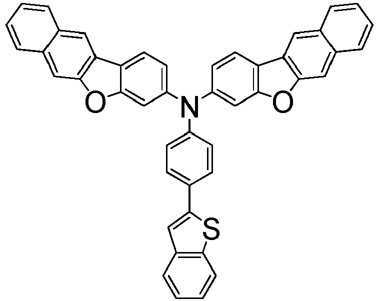

Compound 15 was synthesized using 4-(benzo[b]thiophen-2-yl)aniline instead of 4-(quinolin-3-yl)aniline in the same manner as in Synthesis Example 1 (yield 75%).
m/z: 657.1762 (100.0%), 658.1796 (49.8%), 659.1830 (12.1%), 659.1720 (4.5%), 660.1754 (2.2%), 660.1863 (1.9%)

### Synthesis Example 4: Synthesis of Compound 17

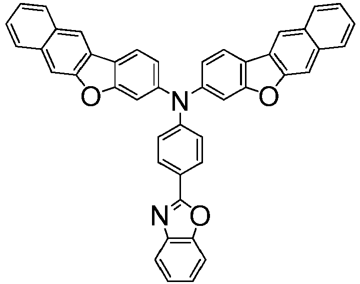

Compound 17 was synthesized using 4-(benzo[d]oxazol-2-yl)aniline instead of 4-(quinolin-3-yl)aniline in the same manner as in Synthesis Example 1 (yield 67%).
m/z: 642.1943 (100.0%), 643.1977 (48.7%), 644.2011 (11.6%), 645.2044 (1.8%)

### Synthesis Example 5: Synthesis of Compound 22

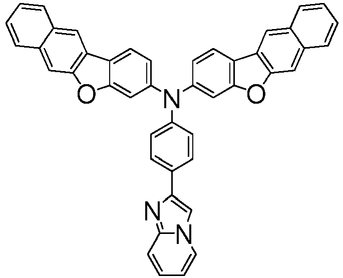

Compound 22 was synthesized using 4-(imidazo[1,2-a]pyridin-2-yl)aniline instead of 4-(quinolin-3-yl)aniline in the same manner as in Synthesis Example 1 (yield 70%).
m/z: 641.2103 (100.0%), 642.2137 (48.7%), 643.2170 (11.6%), 644.2204 (1.8%), 642.2074 (1.1%)

### Manufacturing organic light emitting device

FIG. 1 illustrates the construction of a typical organic light emitting device. In the present disclosure, an organic light emitting device having the structure shown in FIG. 1 was manufactured as an example, but an electron injection layer (not shown) was additionally introduced between the electron transport layer 500 and the cathode 2000. Specifically, in the manufactured organic light emitting device, an anode (hole injection electrode 1000), a hole injection layer 200, a hole transport layer 300, a light emitting layer 400, an electron transport layer 500, an electron injection layer 600, a cathode (electron injection electrode 2000), and a capping layer 3000 are stacked in this order from the bottom.

When manufacturing the organic light emitting device, as a substrate 10, a transparent glass substrate or a flexible plastic substrate is used.

In the organic light emitting device, the hole injection electrode 1000 is used as the anode. For the hole injection electrode, a material having a low work function is used to enable hole injection. Specifically, the hole injection electrode is made of a transparent material such as indium tin oxide (ITO), indium zinc oxide (IZO), or graphene.

The materials used for the hole injection layer 200, hole transport layer 300, light emitting layer 400, electron transport layer 500, electron injection layer 600, and high refractive index capping layer are summarized in Table 1 below.

In addition, the cathode 2000 for electron injection was formed on the electron injection layer 600. Various metals can be used to form the cathode. Specific examples of the metal include aluminum, gold, silver, etc.

**[Table 1]**

| | | |
|---|---|---|
| 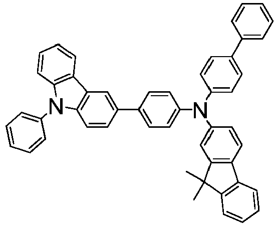 | 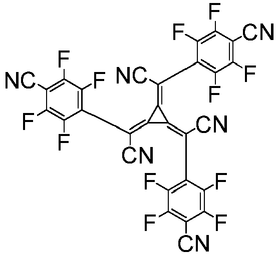 | 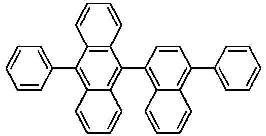 |
| **HT01** | **NDP9** | **BH01** |
| 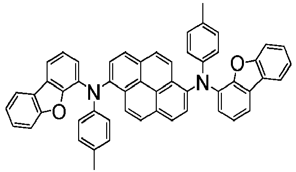 | 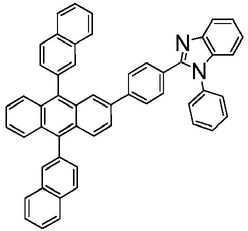 | 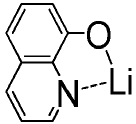 |
| **BD01** | **ET01** | **Liq** |

### Example 1

An indium tin oxide (ITO) substrate with a reflective layer containing silver (Ag) was cleaned with distilled water by ultrasonic cleaning. After the completion of the distilled water washing, the substrate was cleaned with a solvent such as isopropyl alcohol, acetone, or methanol by ultrasonic cleaning and then dried. As a hole-injection layer, HT01 doped with 3% by weight of NDP9 was formed to have a thickness of 100 Å on top of the ITO substrate. Next, HT01 with a thickness of 1000 Å was deposited thereon as a hole transport layer. BH01 (host layer) doped with 3% by weight of BD01 (dopant) was formed to have a thickness of 250 Å as a light emitting layer. Next, an electron transport layer with a thickness of 300 Å was deposited using a mixture of ET01 and Liq (in a weight ratio of 1:1), and then LiF was deposited to form an electron injection layer with a thickness of 10 Å. MgAg was then deposited to a thickness of 15 nm to form a cathode. Next, a capping layer with a thickness of 600 Å was formed on the cathode by depositing the compound prepared in Synthesis Example 1. The resulting structure was encapsulated in a glove box to produce an organic light emitting device.

### Examples 2 to 5

Organic light emitting devices were manufactured in the same manner as in Example 1 except that the compounds prepared in Synthesis Examples 2 to 5 were used, respectively, to form the capping layer.

### Comparative Examples 1 to 4

Organic light emitting devices were manufactured in the same manner as in Example 1 except that Comparative Compounds 1 to 4 listed in Table 2 were respectively used to form the capping layer.

**[Table 2]**

| | | |
|---|---|---|
| 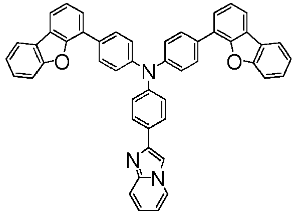 | 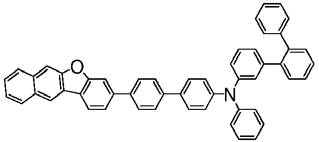 | 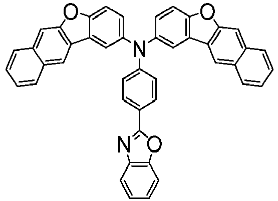 |
| **Comparative Compound 1** | **Comparative Compound 2** | **Comparative Compound 3** |
| 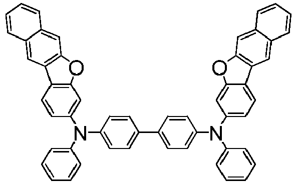 | | |
| **Comparative Compound 4** | | |

### <Experimental Example 1> Performance evaluation of organic light emitting devices

Electrons and holes were injected by applying a voltage using a source measure unit (Kiethley 2400 manufactured by Keithley Instruments, Inc.) and the luminance was measured using a spectroradiometer (CS-2000 manufactured by Konica Minolta Inc.) when light is emitted. To evaluate the performance of each of the organic light emitting devices of Examples 1 to 5, and Comparative Examples 1 to 4, the current density and luminance with respect to the applied voltage were measured under atmospheric pressure, and the results are shown in Table 3.

**[Table 3]**

| | Op. V | mA/cm2 | Cd/A | CIEx | CIEy | LT97 |
|---|---|---|---|---|---|---|
| Example 1 | 3.45 | 10 | 7.85 | 0.140 | 0.043 | 162 |
| Example 2 | 3.45 | 10 | 8.33 | 0.140 | 0.043 | 168 |
| Example 3 | 3.45 | 10 | 8.50 | 0.139 | 0.042 | 175 |
| Example 4 | 3.44 | 10 | 7.54 | 0.139 | 0.042 | 155 |
| Example 5 | 3.45 | 10 | 8.25 | 0.139 | 0.042 | 170 |

| | | | | | | |
|---|---|---|---|---|---|---|
| Comparative Example 1 | 3.47 | 10 | 6.44 | 0.134 | 0.050 | 100 |
| Comparative Example 2 | 3.46 | 10 | 6.12 | 0.135 | 0.052 | 105 |
| Comparative Example 3 | 3.46 | 10 | 5.95 | 0.137 | 0.054 | 90 |
| Comparative Example 4 | 3.46 | 10 | 6.84 | 0.137 | 0.048 | 133 |

Compared to Comparative Example 1 and Comparative Example 2, the capping layer compound used in Examples 1 to 5 of the present invention contains two 4-ring fused hetero groups and has a heteroaryl group at the same time to have a high refractive index and increased absorption intensity of ultraviolet region, so high efficiency, high color purity, and long lifespan effects can be realized.

In addition, compared to Comparative Example 3, the capping layer compound used in Examples 1 to 5 of the present invention has a linear connection structure of an amine core and a ring-fused hetero group, so it had excellent thin film arrangement and high polarizability. Therefore, a higher refractive index was achieved.

In addition, compared to Comparative Example 4, the present invention has a single arylamine structure, so it minimizes bulky characteristics, had a high refractive index even with a small molecular weight, and has excellent thermal stability, so an organic light-emitting device with high efficiency, high color purity, and long lifespan was realized.

### <Experimental Example 2> Evaluation of refractive index

With the use of each of Compound 8, Compound 15, Compound 17, Compound 22 and Comparative Compounds 1 to 4, deposition films with a thickness of 60 nm were formed on respective silicon substrates, and the refractive index at a wavelength of 450 nm was measured using an ellipsometer device (M-2000X available from J.A.Woollam Co., Inc.). The measurement results are summarized in Table 4.

**[Table 4]**

| @450nm | Compara tive Compou nd 1 | Compara tive Compou nd 2 | Compara tive Compou nd 3 | Compara tive Compou nd 4 | Compou nd 8 | Compou nd 15 | Compou nd 17 | Compou nd 22 |
|---|---|---|---|---|---|---|---|---|
| refractive index, n | 2.22 | 2.15 | 2.09 | 2.30 | 2.47 | 2.50 | 2.45 | 2.48 |

As shown in Table 4 above, it can be seen that Compound 8, Compound 15, Compound 17, and Compound 22 exhibited a high refractive index of 2.40 or more, more specifically, 2.45 or more.

### <Experimental Example 3> Evaluation of absorption intensity in the ultraviolet region

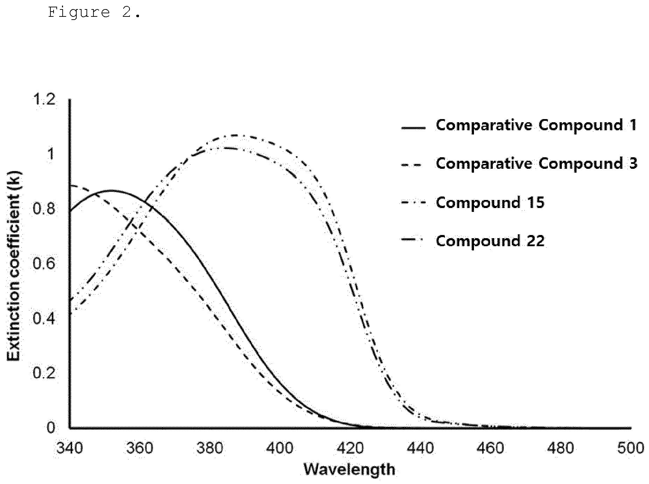

With the use of each of Compound 15, Compound, Comparative Compound 1 and Comparative Compound 3, deposition films with a thickness of 30 nm were formed on respective silicon substrates, and the absorption intensity within the range of 340 nm to 500 nm was measured using an ellipsometer device (M-2000X available from J.A.Woollam Co., Inc.). the results were shown in Figure 2.

Based on the wavelength of 380 nm in the ultraviolet absorption region, the absorption intensity of Compound 15 and Compound 22 is 0.8 or more, more specifically 1.0 or more, confirming an increase in absorption intensity. As such, the compound according to the present invention has the effect of increasing the absorption wavelength in the ultraviolet region, so when applied to a capping layer, an organic light-emitting device with high color purity, high efficiency, and long lifespan can be implemented.

## Claims

1. A compound for a capping layer, in which the compound being represented by Formula 1 below: 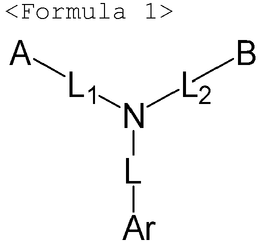 In Formula 1,
A and B are each independently Formula 1-1 or Formula 1-2,
L, L1 and L2 are each independently a direct bond, a substituted or unsubstituted C5-C50 arylene group, or a substituted or unsubstituted C2-C50 heteroarylene group, and
Ar is a substituted or unsubstituted C2-C50 heteroaryl group, or a C5-C50 aryl group substituted with a cyano group, 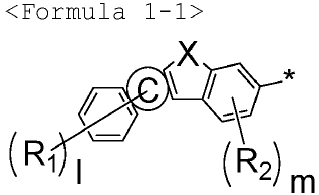 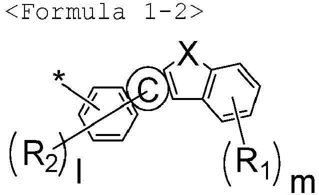 In Formula 1-1 and Formula 1-2,
X is each independently O, S, CRR' or NR,
C-ring is each independently a substituted or unsubstituted C5-C30 aryl group, or a substituted or unsubstituted C2-C30 heteroarylene group, and
R, R', R1 and R2 are each independently hydrogen, deuterium, halogen, nitro group, nitrile group, hydroxy group, thiol group, a substituted or unsubstituted amino group, a substituted or unsubstituted C1~C30 alkyl group, a substituted or unsubstituted C2-C30 alkenyl group, a substituted or unsubstituted C1-C30 alkoxy group, a substituted or unsubstituted C1-C30 sulfide group, a substituted or unsubstituted C0∼C30 silyl group, a substituted or unsubstituted C5-C50 aryl group, or a substituted or unsubstituted C2-C50 heteroaryl group, and a plurality of R1 adjacent to each other, a plurality of R2 adjacent to each other, or a plurality of R and R' adjacent to each other, may be joined together to form or not to form a ring.

2. The compound for a capping layer of claim 1, wherein Formula 1 is represented by Formula 2 or Formula 3 below: 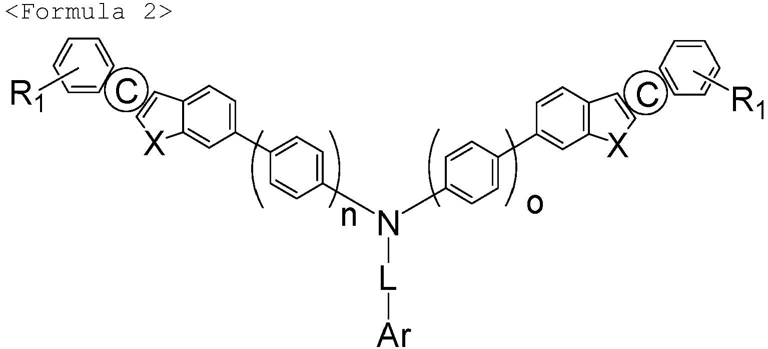 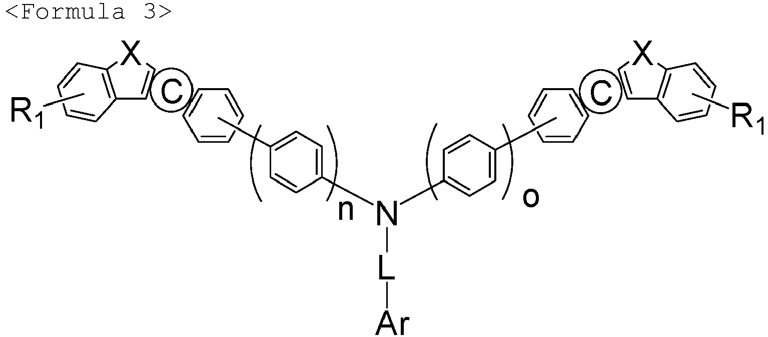 In Formulas 2 and 3,
X, C-ring, L, Ar and R1 are the same as defined in Formula 1, Formula 1-1 and Formula 1-2, and
n and o are each independently an integer in a range from 0 to 3.

3. The compound for a capping layer of claim 1, wherein Formula 1 is represented by Formula 4 or Formula 5 below: 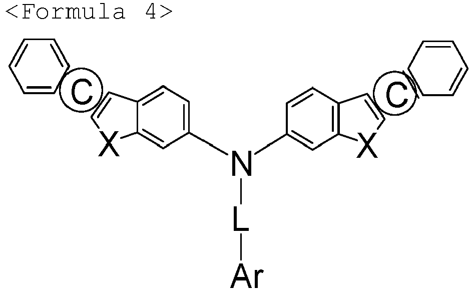 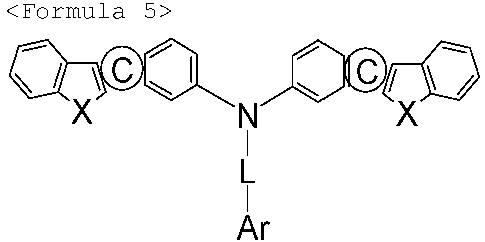
wherein, in Formulas 4 and 5,
X, C-ring, L and Ar are the same as defined in Formula 1, Formula 1-1 and Formula 1-2 above.

4. The compound for a capping layer of claim 1, wherein Formula 1 is represented by Formula 6 or Formula 7 below: 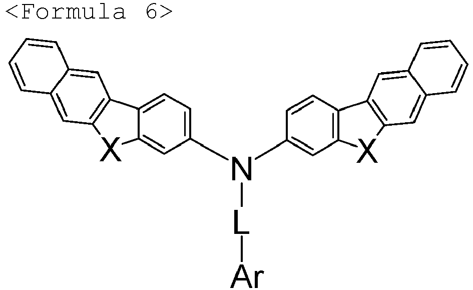 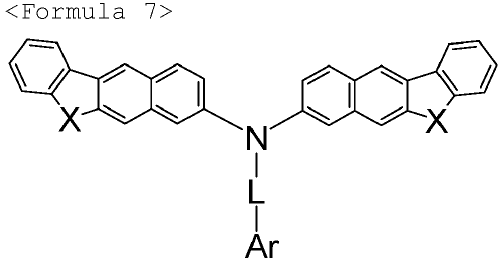
wherein, in Formulas 6 and 7,
X, L and Ar are the same as defined in Formula 1, Formula 1-1 and Formula 1-2 above.

5. The compound for a capping layer of claim 1, wherein X is independently O or S.

6. The compound for a capping layer of claim 1, wherein Ar includes a heteroaryl group composed of one ring or a heteroaryl group fused with two rings.

7. The compound for a capping layer of claim 1, wherein Ar is selected from the group consisting of a quinoline group, a benzofuran group, a benzothiophene group, an indole group, a benzoxazole group, a benzothiazole group, a benzoimidazole group, an indolizine group, an imidazopyridine group, a benzotriazole group, a furan group, a thiophene group, an oxadiazole group, and a thiadiazole group.

8. The compound for a capping layer of claim 1, wherein Ar is selected from the group consisting of a quinoline group, a benzofuran group, a benzothiophene group, and an imidazopyridine group.

9. The compound for a capping layer of claim 1, wherein L is a phenylene group or a biphenylene group.

10. The compound for a capping layer of claim 1, wherein *-L-Ar is selected from the structures represented by Structural Formulas A-1 to A-17 shown below: 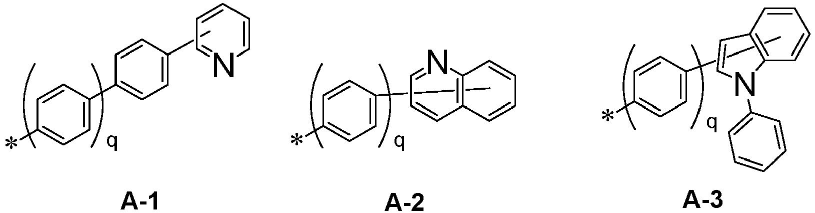 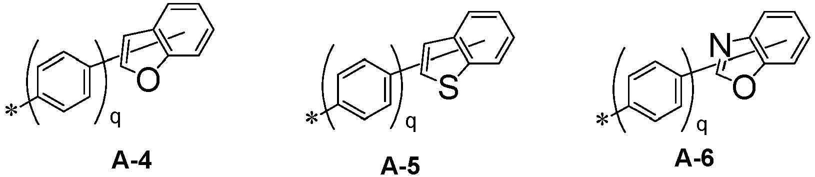 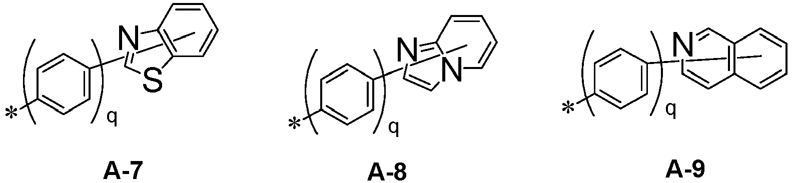 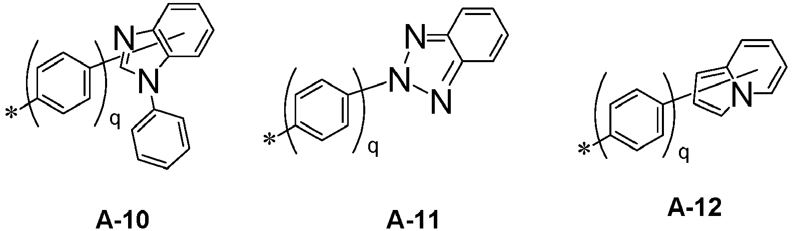 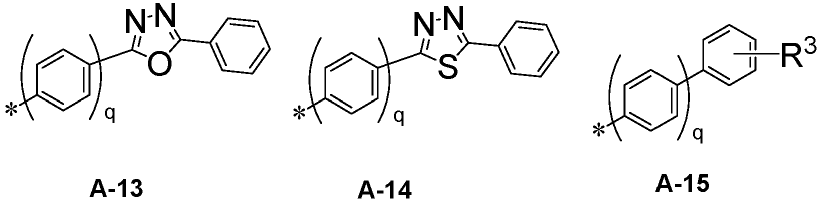 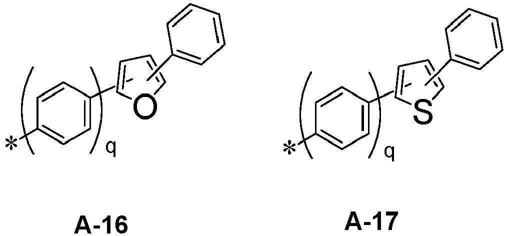 In Structural Formulas A-1 to A-17,
q is each independently an integer in a range from 0 to 2, R3 is a nitrile group, and "*" is a bonding position bonded to N of Formula 1 above.

11. The compound for a capping layer of claim 1, wherein the capping layer compound of Formula 1 is any one of the following compounds:
| | | |
|---|---|---|
| 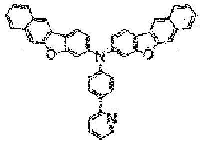 | 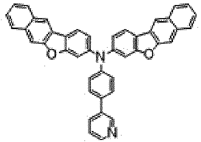 | 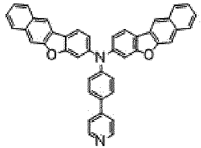 |
| **1** | **2** | **3** |
| 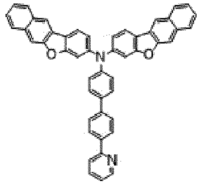 | 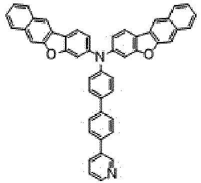 | 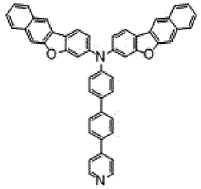 |
| **4** | **5** | **6** |
| 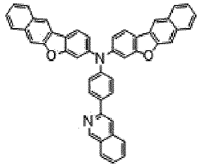 | 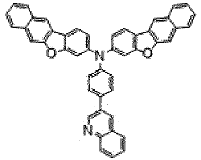 | 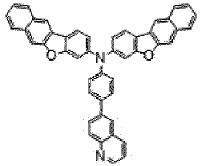 |
| **7** | **8** | **9** |
| 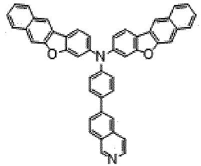 | 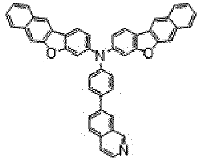 | 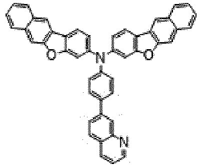 |
| **10** | **11** | **12** |
| 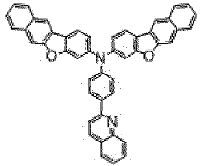 | 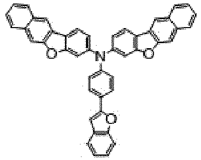 | 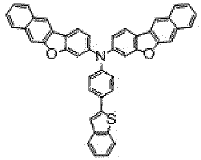 |
| **13** | **14** | **15** |
| 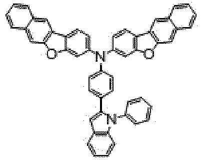 | 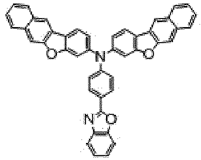 | 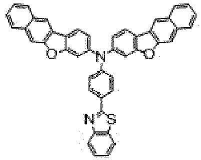 |
| **16** | **17** | **18** |
| 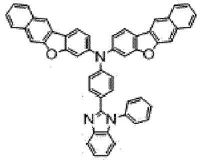 | 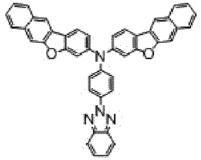 | 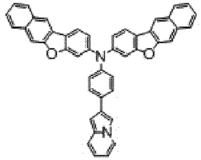 |
| **19** | **20** | **21** |
| 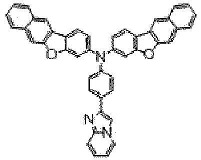 | 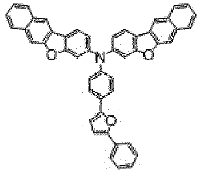 | 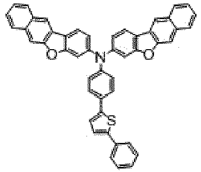 |
| **22** | **23** | **24** |
| 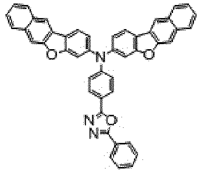 | 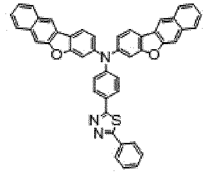 | 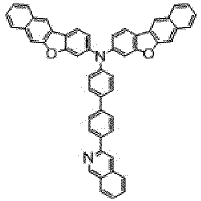 |
| **25** | **26** | **27** |
| 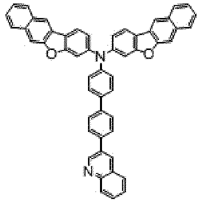 | 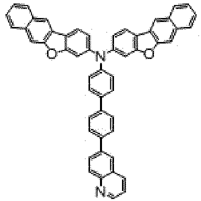 | 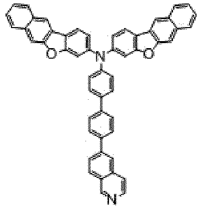 |
| **28** | **29** | **30** |
| 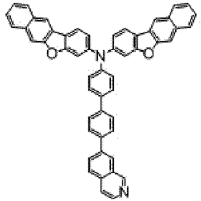 | 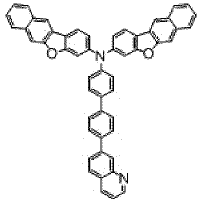 | 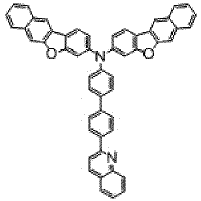 |
| **31** | **32** | **33** |
| 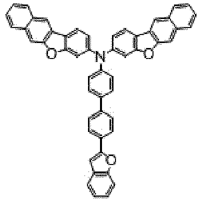 | 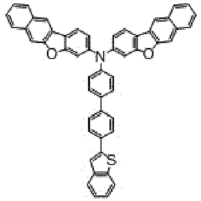 | 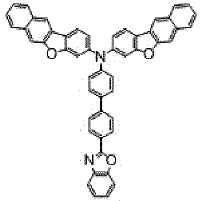 |
| **34** | **35** | **36** |
| 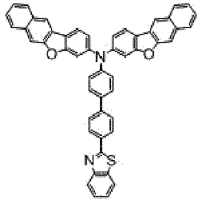 | 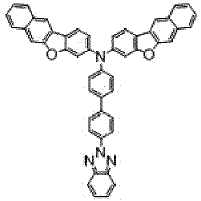 | 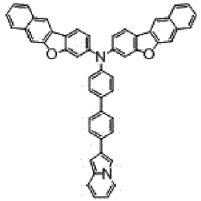 |
| **37** | **38** | **39** |
| 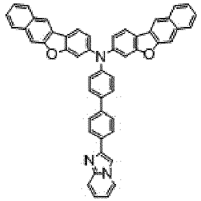 | 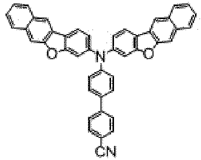 | 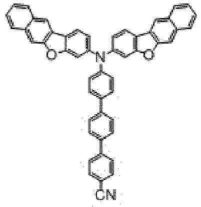 |
| **40** | **41** | **42** |
| 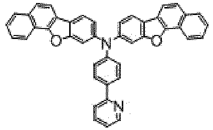 | 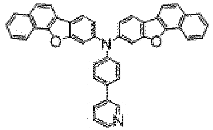 | 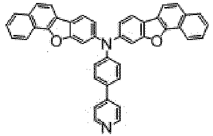 |
| **43** | **44** | **45** |
| 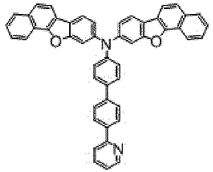 | 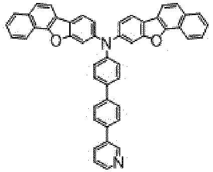 | 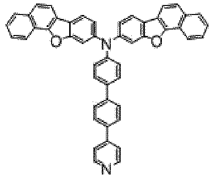 |
| **46** | **47** | **48** |
| 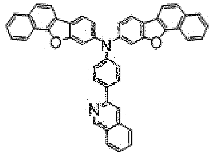 | 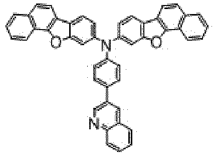 | 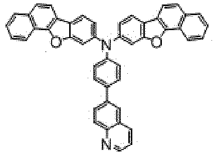 |
| **49** | **50** | **51** |
| 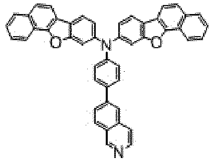 | 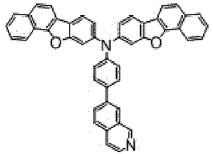 | 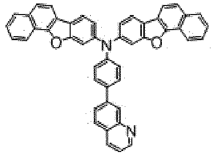 |
| **52** | **53** | **54** |
| 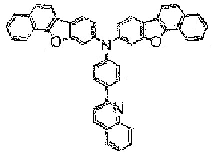 | 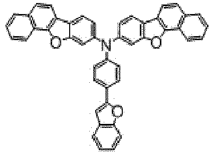 | 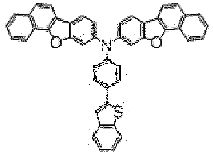 |
| **55** | **56** | **57** |
| 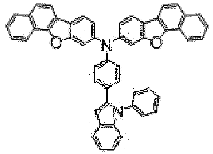 | 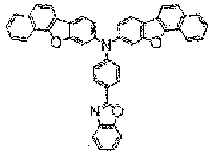 | 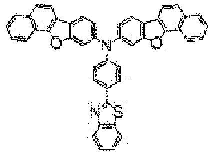 |
| **58** | **59** | **60** |
| 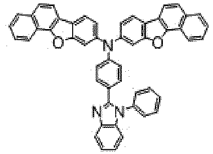 | 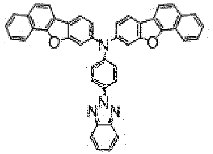 | 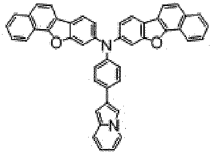 |
| **61** | **62** | **63** |
| 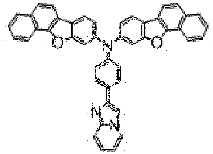 | 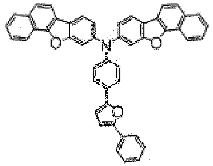 | 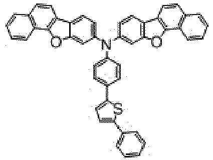 |
| **64** | **65** | **66** |
| 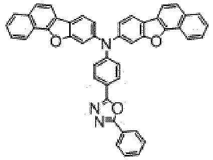 | 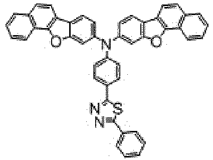 | 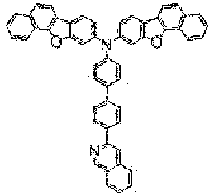 |
| **67** | **68** | **69** |
| 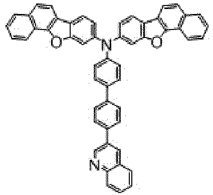 | 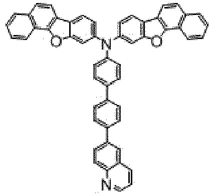 | 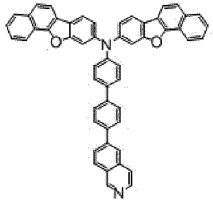 |
| **70** | **71** | **72** |
| 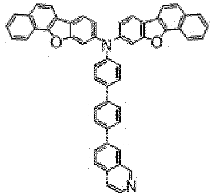 | 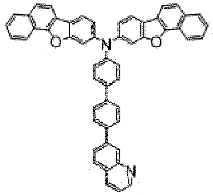 | 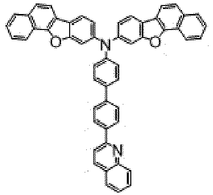 |
| **73** | **74** | **75** |
| 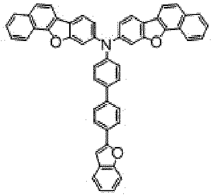 | 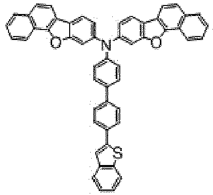 | 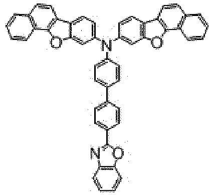 |
| **76** | **77** | **78** |
| 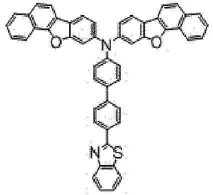 | 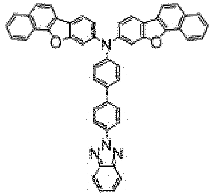 | 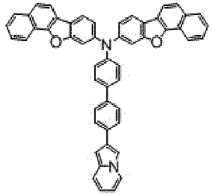 |
| **79** | **80** | **81** |
| 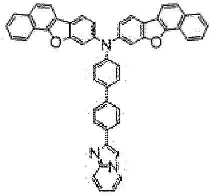 | 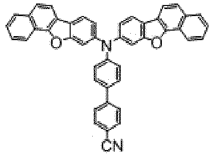 | 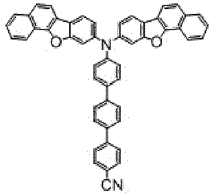 |
| **82** | **83** | **84** |
| 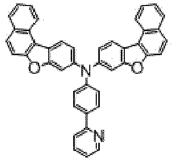 | 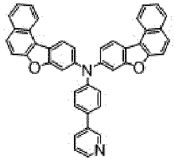 | 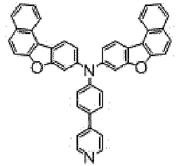 |
| **85** | **86** | **87** |
| 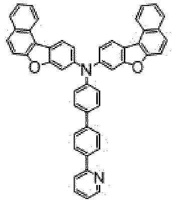 | 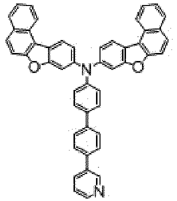 | 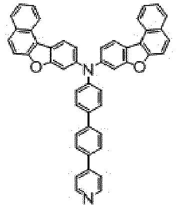 |
| **88** | **89** | **90** |
| 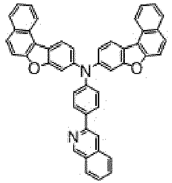 | 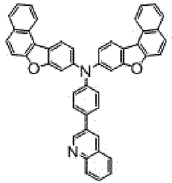 | 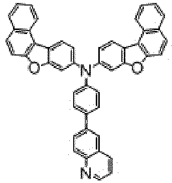 |
| **91** | **92** | **93** |
| 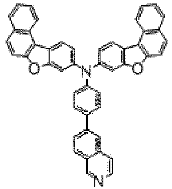 | 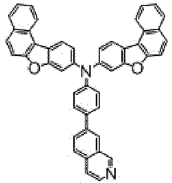 | 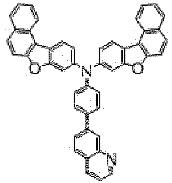 |
| **94** | **95** | **96** |
| 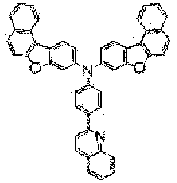 | 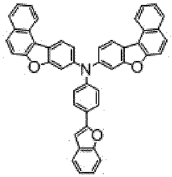 | 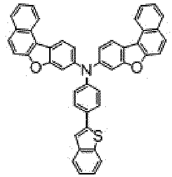 |
| **97** | **98** | **99** |
| 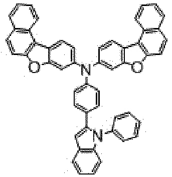 | 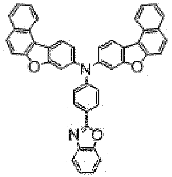 | 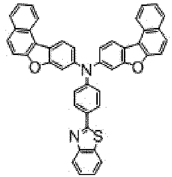 |
| **100** | **101** | **102** |
| 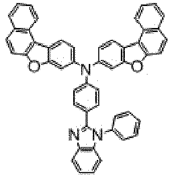 | 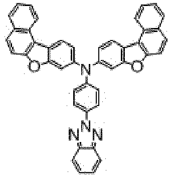 | 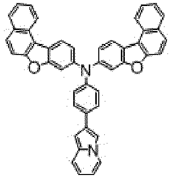 |
| **103** | **104** | **105** |
| 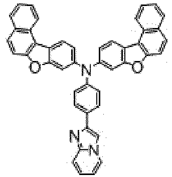 | 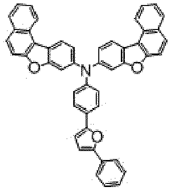 | 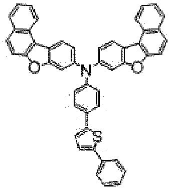 |
| **106** | **107** | **108** |
| 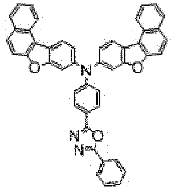 | 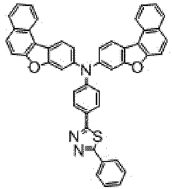 | 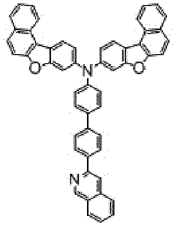 |
| **109** | **110** | **111** |
| 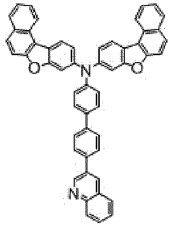 | 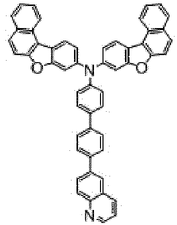 | 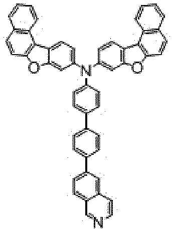 |
| **112** | **113** | **114** |
| 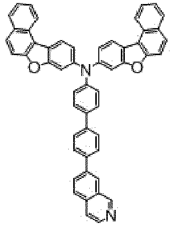 | 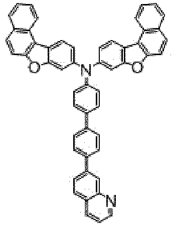 | 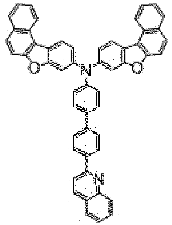 |
| **115** | **116** | **117** |
| 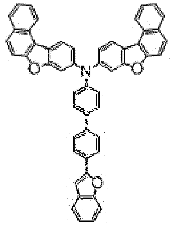 | 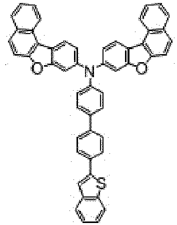 | 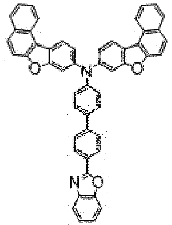 |
| **118** | **119** | **120** |
| 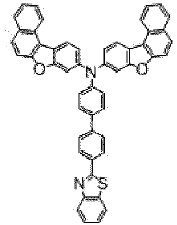 | 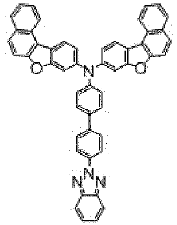 | 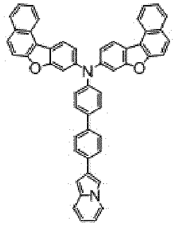 |
| **121** | **122** | **123** |
| 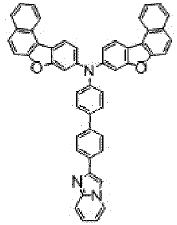 | 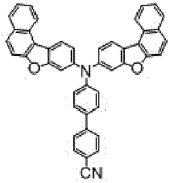 | 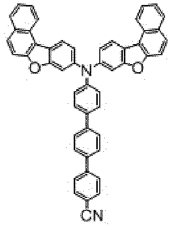 |
| **124** | **125** | **126** |
| 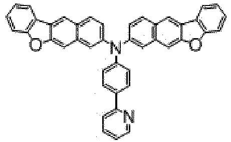 | 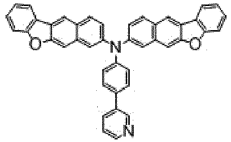 | 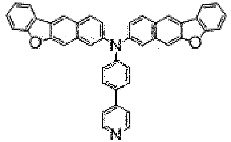 |
| **127** | **128** | **129** |
| 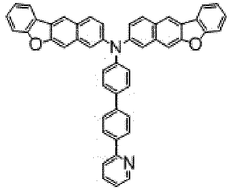 | 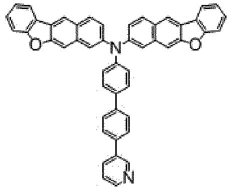 | 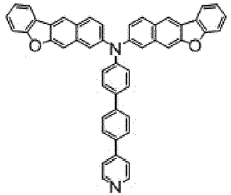 |
| **130** | **131** | **132** |
| 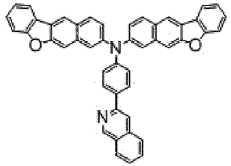 | 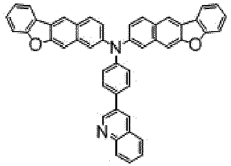 | 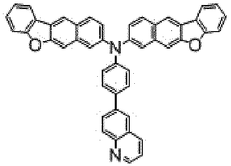 |
| **133** | **134** | **135** |
| 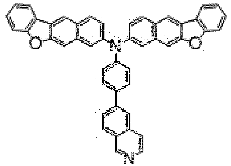 | 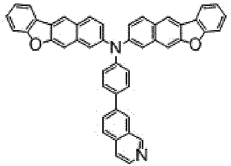 | 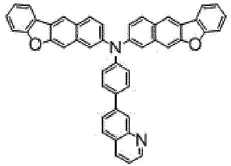 |
| **136** | **137** | **138** |
| 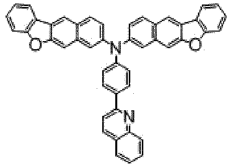 | 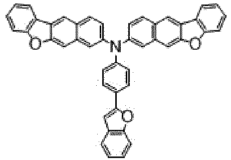 | 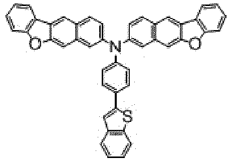 |
| **139** | **140** | **141** |
| 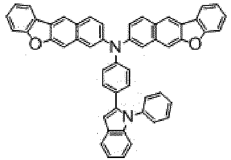 | 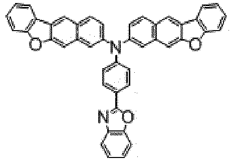 | 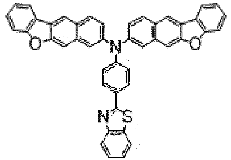 |
| **142** | **143** | **144** |
| 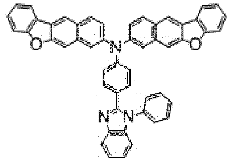 | 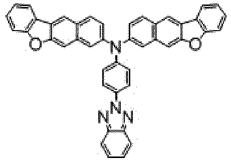 | 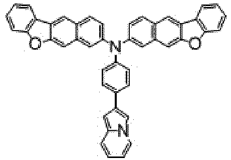 |
| **145** | **146** | **147** |
| 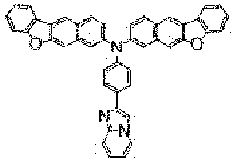 | 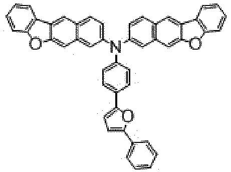 | 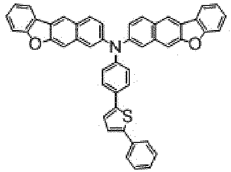 |
| **148** | **149** | **150** |
| 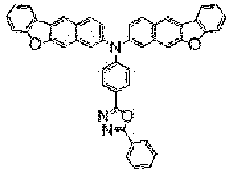 | 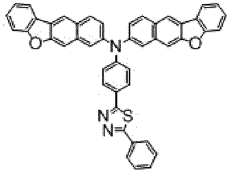 | 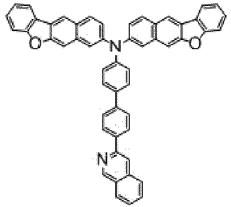 |
| **151** | **152** | **153** |
| 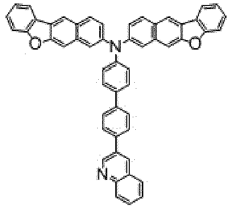 | 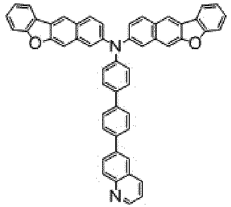 | 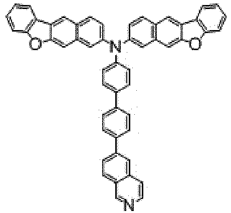 |
| **154** | **155** | **156** |
| 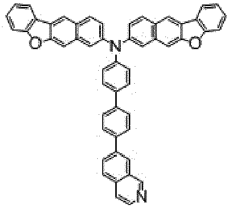 | 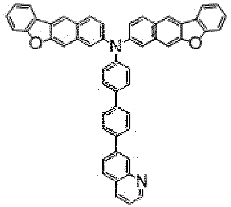 | 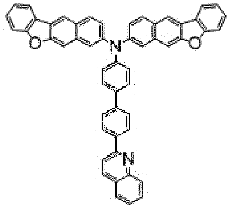 |
| **157** | **158** | **159** |
| 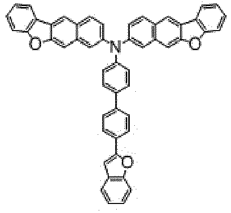 | 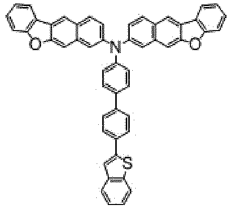 | 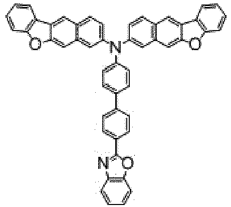 |
| **160** | **161** | **162** |
| 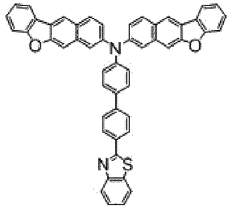 | 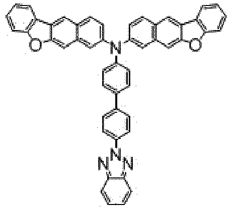 | 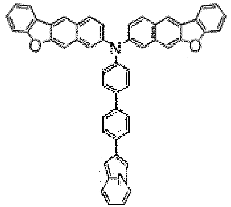 |
| **163** | **164** | **165** |
| 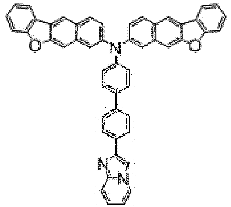 | 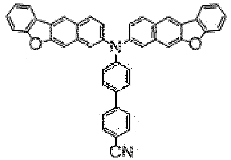 | 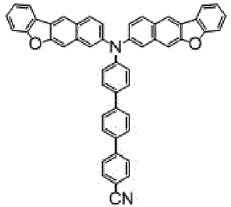 |
| **166** | **167** | **168** |
| 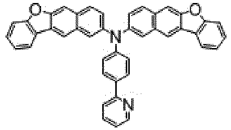 | 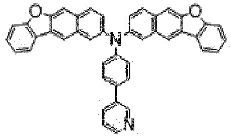 | 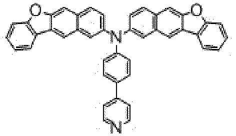 |
| **169** | **170** | **171** |
| 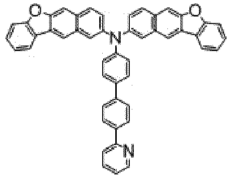 | 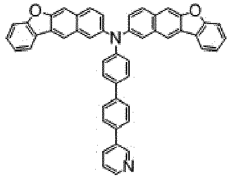 | 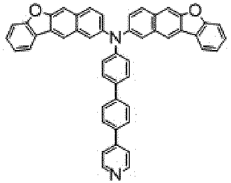 |
| **172** | **173** | **174** |
| 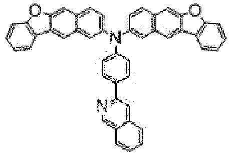 | 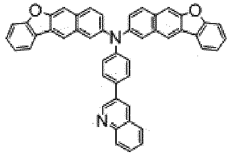 | 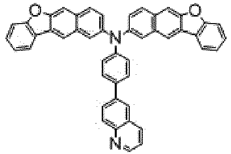 |
| **175** | **176** | **177** |
| 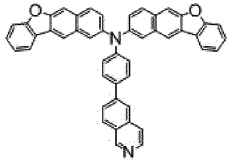 | 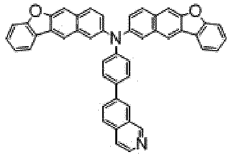 | 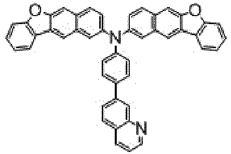 |
| **178** | **179** | **180** |
| 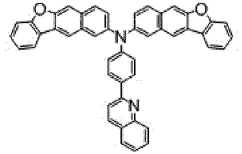 | 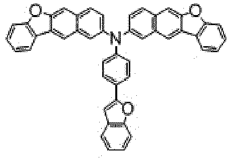 | 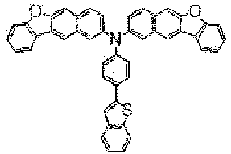 |
| **181** | **182** | **183** |
| 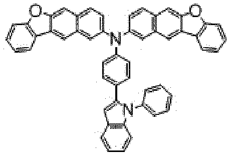 | 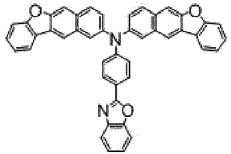 | 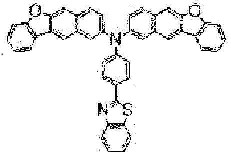 |
| **184** | **185** | **186** |
| 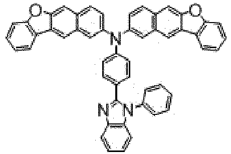 | 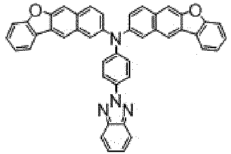 | 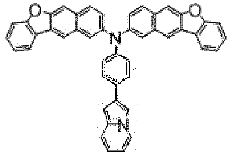 |
| **187** | **188** | **189** |
| 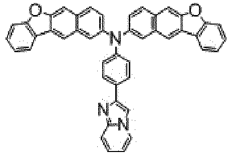 | 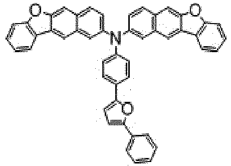 | 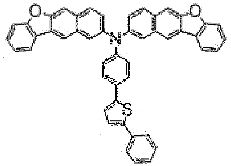 |
| **190** | **191** | **192** |
| 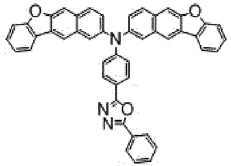 | 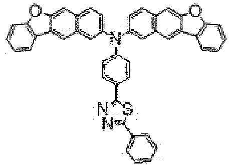 | 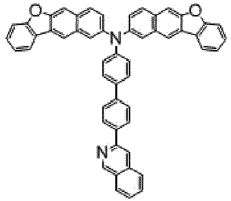 |
| **193** | **194** | **195** |
| 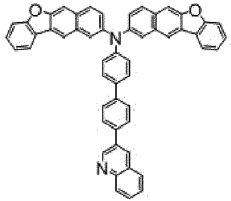 | 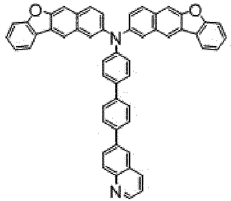 | 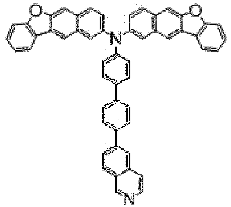 |
| **196** | **197** | **198** |
| 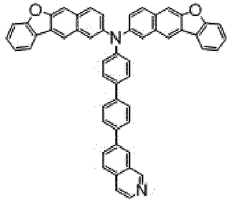 | 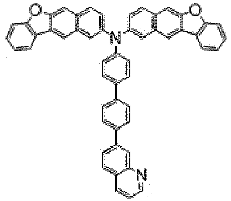 | 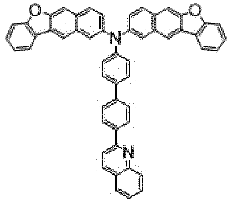 |
| **199** | **200** | **201** |
| 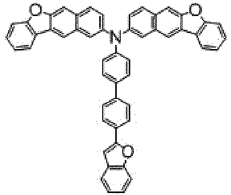 | 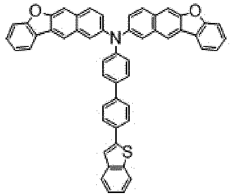 | 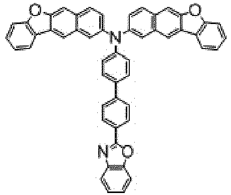 |
| **202** | **203** | **204** |
| 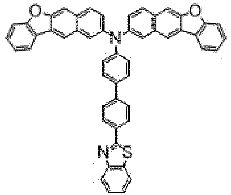 | 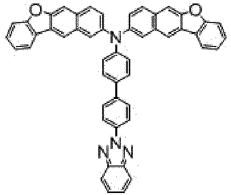 | 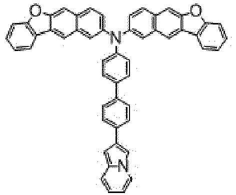 |
| **205** | **206** | **` 207** |
| 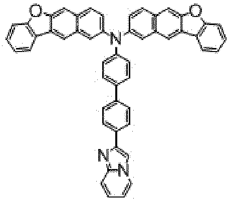 | 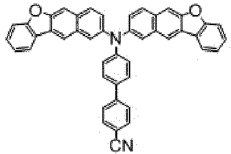 | 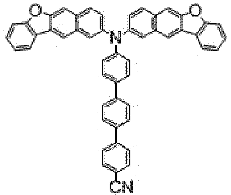 |
| **208** | **209** | **210** |
| 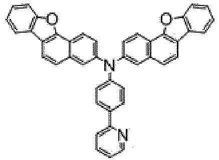 | 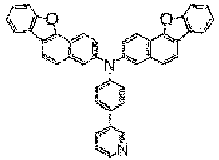 | 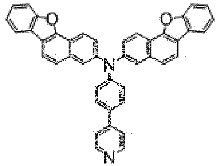 |
| **211** | **212** | **213** |
| 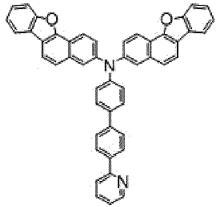 | 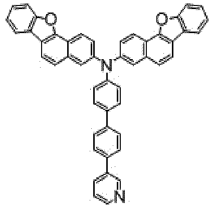 | 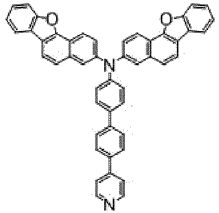 |
| **214** | **215** | **216** |
| 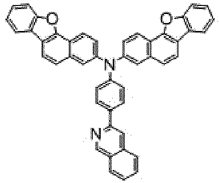 | 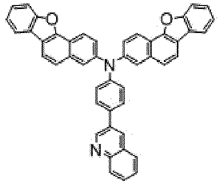 | 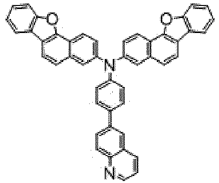 |
| **217** | **218** | **219** |
| 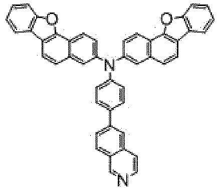 | 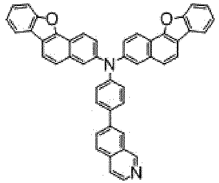 | 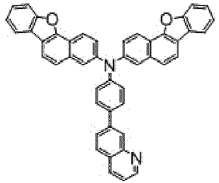 |
| **220** | **221** | **222** |
| 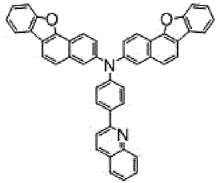 | 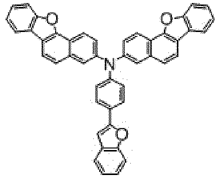 | 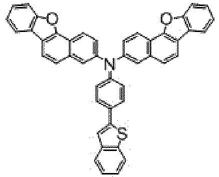 |
| **223** | **224** | **225** |
| 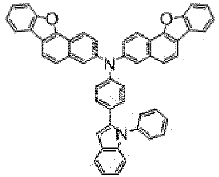 | 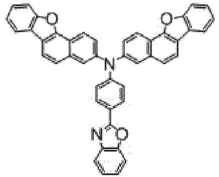 | 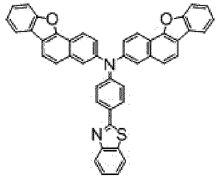 |
| **226** | **227** | **228** |
| 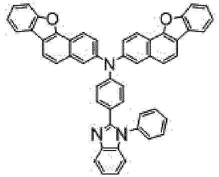 | 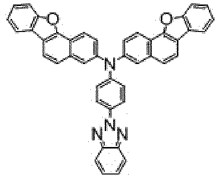 | 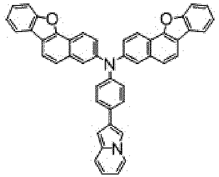 |
| **229** | **230** | **231** |
| 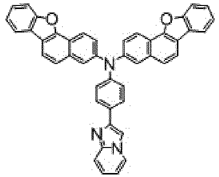 | 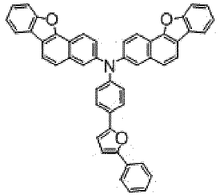 | 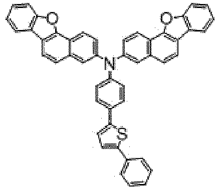 |
| **232** | **233** | **234** |
| 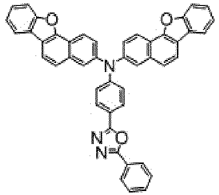 | 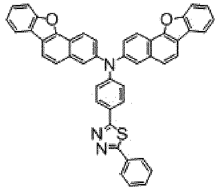 | 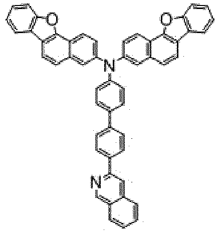 |
| **235** | **236** | **237** |
| 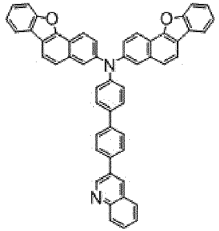 | 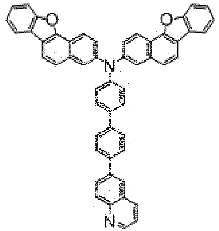 | 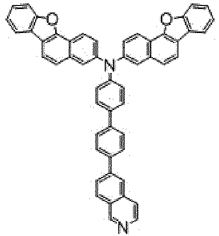 |
| **238** | **239** | **240** |
| 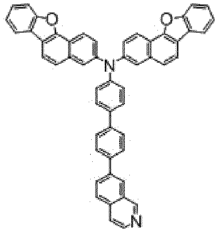 | 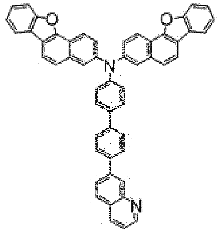 | 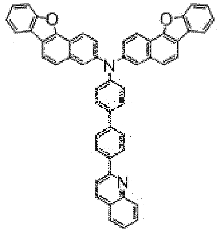 |
| **241** | **242** | **243** |
| 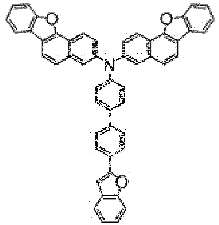 | 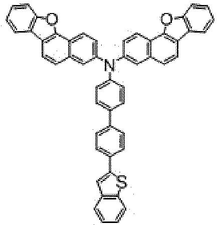 | 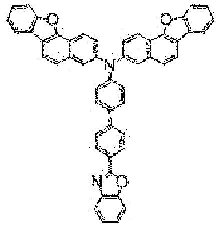 |
| **244** | **245** | **246** |
| 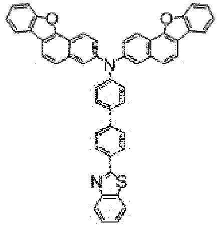 | 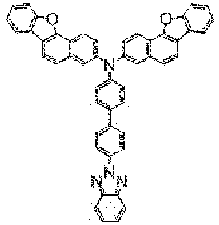 | 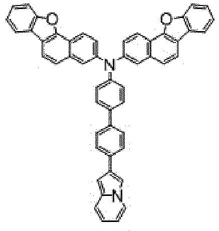 |
| **247** | **248** | **249** |
| 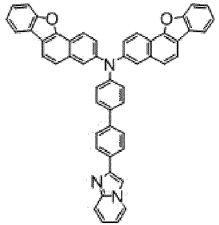 | 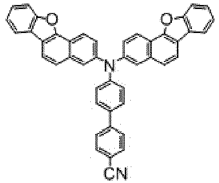 | 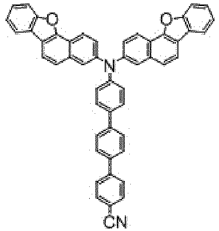 |
| **250** | **251** | **252** |
| 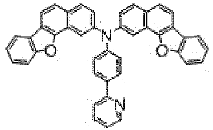 | 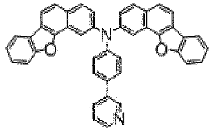 | 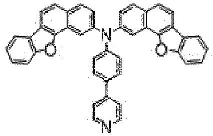 |
| **253** | **254** | **255** |
| 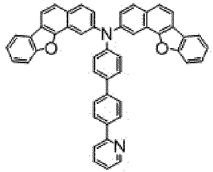 | 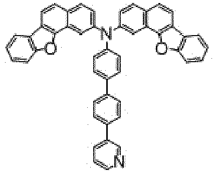 | 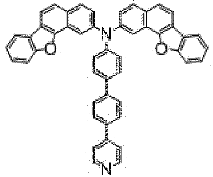 |
| **256** | **257** | **258** |
| 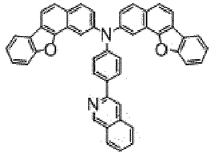 | 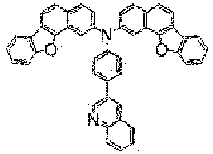 | 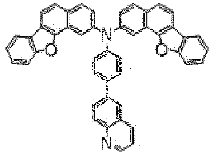 |
| **259** | **260** | **261** |
| 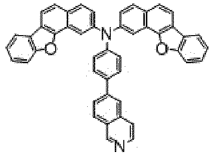 | 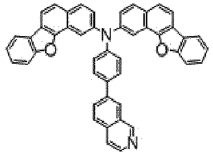 | 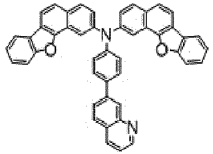 |
| **262** | **263** | **264** |
| 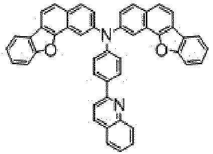 | 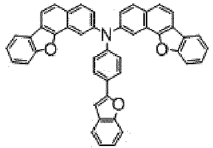 | 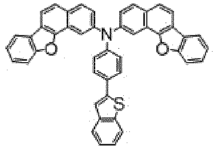 |
| **265** | **266** | **267** |
| 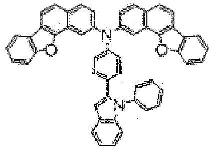 | 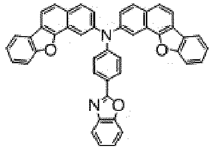 | 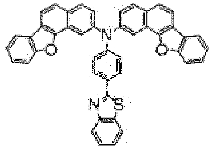 |
| **268** | **269** | **270** |
| 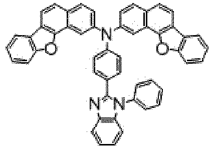 | 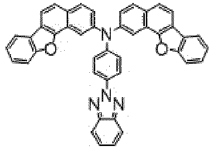 | 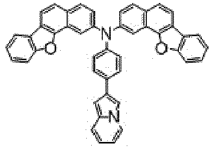 |
| **271** | **272** | **273** |
| 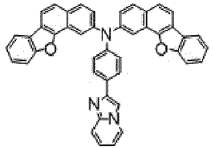 | 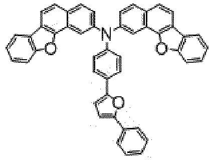 | 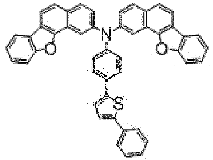 |
| **274** | **275** | **276** |
| 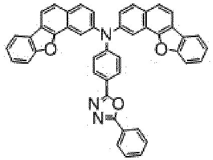 | 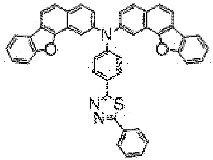 | 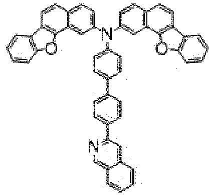 |
| **277** | **278** | **279** |
| 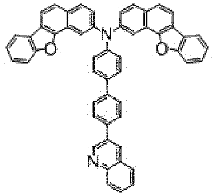 | 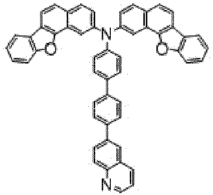 | 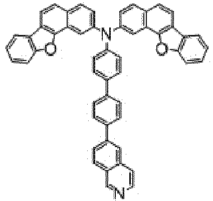 |
| **280** | **281** | **282** |
| 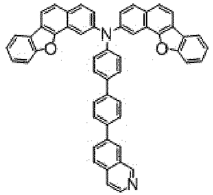 | 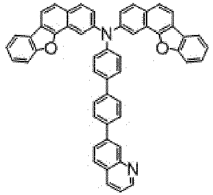 | 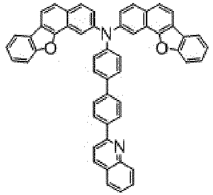 |
| **283** | **284** | **285** |
| 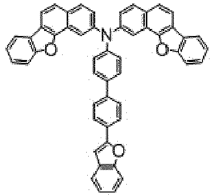 | 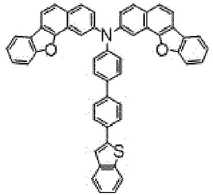 | 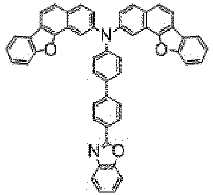 |
| **286** | **287** | **288** |
| 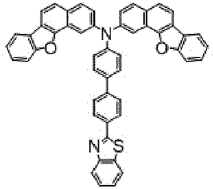 | 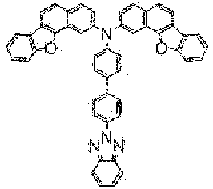 | 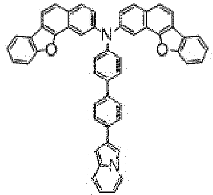 |
| **289** | **290** | 291 |
| 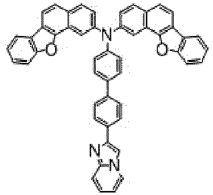 | 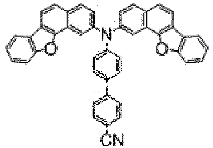 | 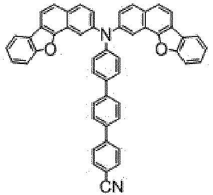 |
| **292** | **293** | **294** |
| 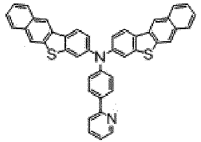 | 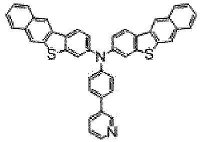 | 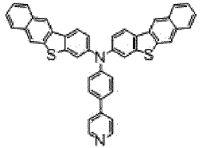 |
| **295** | **296** | **297** |
| 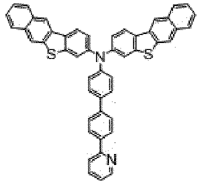 | 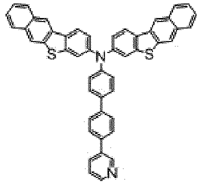 | 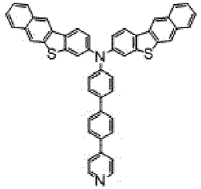 |
| **298** | **299** | **300** |
| 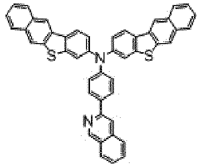 | 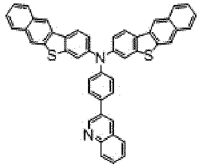 | 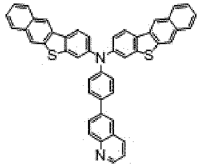 |
| **301** | **302** | **303** |
| 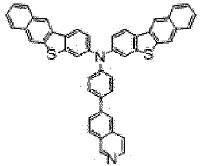 | 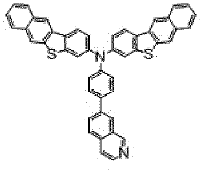 | 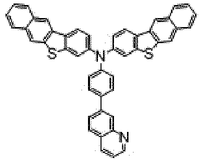 |
| **304** | **305** | **306** |
| 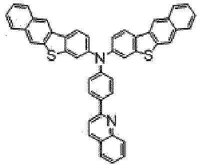 | 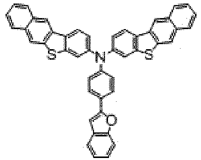 | 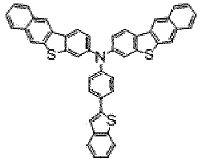 |
| **307** | **308** | **309** |
| 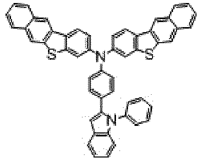 | 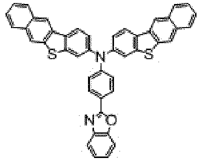 | 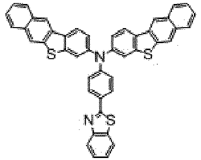 |
| **310** | **311** | **312** |
| 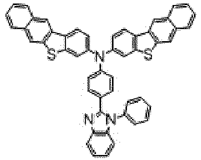 | 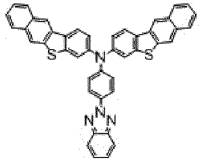 | 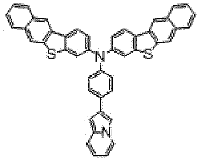 |
| **313** | **314** | **315** |
| 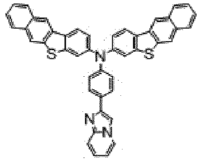 | 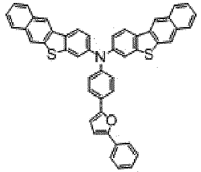 | 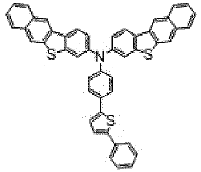 |
| **316** | **317** | **318** |
| 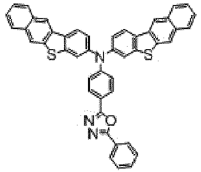 | 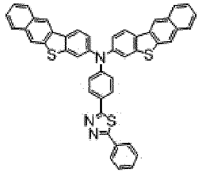 | 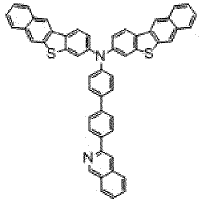 |
| **319** | **320** | **321** |
| 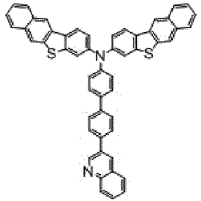 | 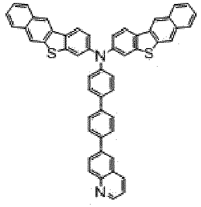 | 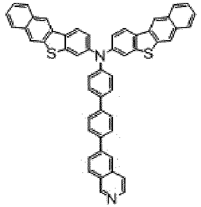 |
| **322** | **323** | **324** |
| 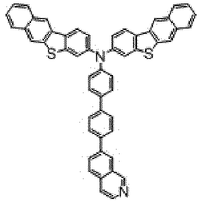 | 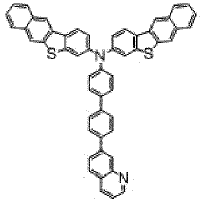 | 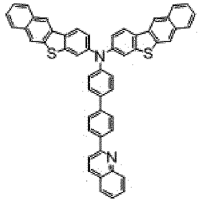 |
| **325** | **326** | **327** |
| 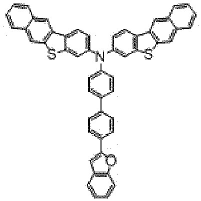 | 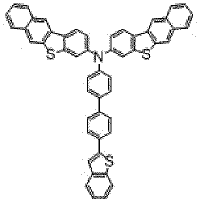 | 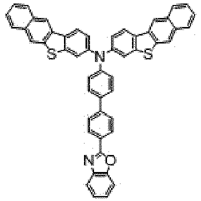 |
| **328** | **329** | **330** |
| 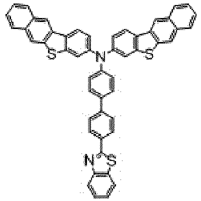 | 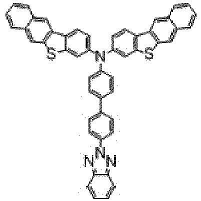 | 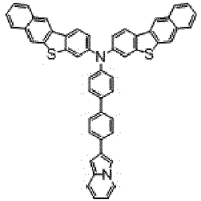 |
| **331** | **332** | **333** |
| 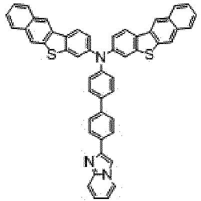 | 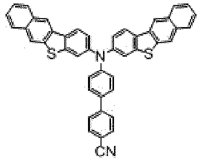 | 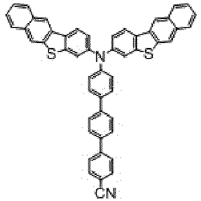 |
| **334** | **335** | **336** |
| 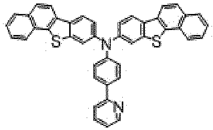 | 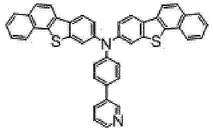 | 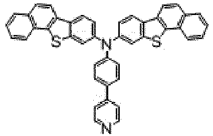 |
| **337** | **338** | **339** |
| 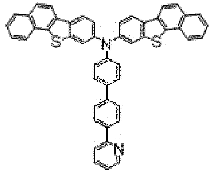 | 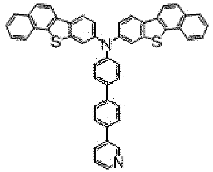 | 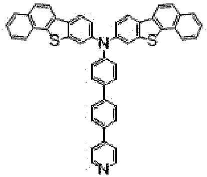 |
| **340** | **341** | **342** |
| 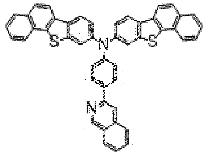 | 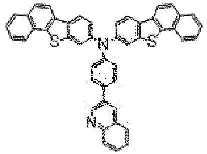 | 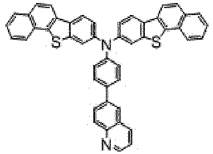 |
| **343** | **344** | **345** |
| 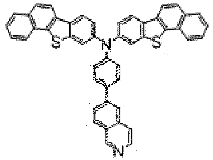 | 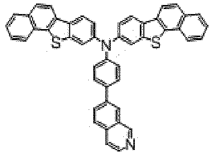 | 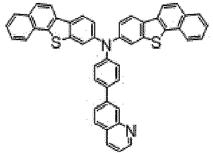 |
| **346** | **347** | **348** |
| 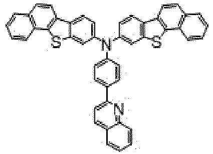 | 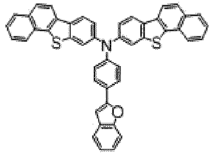 | 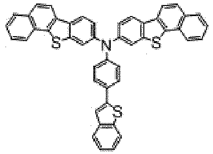 |
| **349** | **350** | **351** |
| 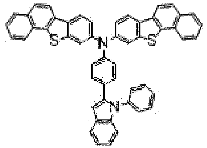 | 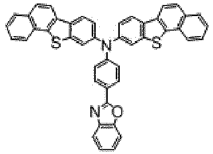 | 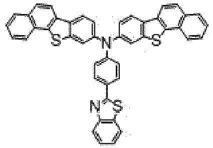 |
| **352** | **353** | **354** |
| 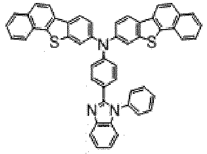 | 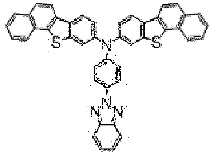 | 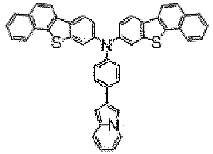 |
| **355** | **356** | **357** |
| 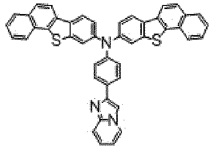 | 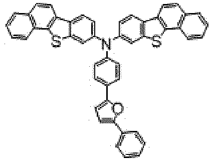 | 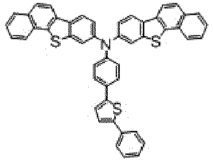 |
| **358** | **359** | **360** |
| 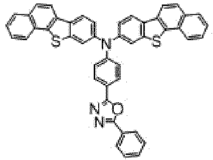 | 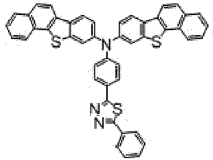 | 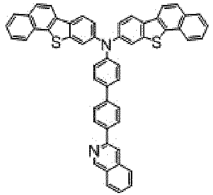 |
| **361** | **362** | **363** |
| 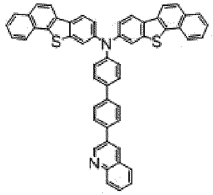 | 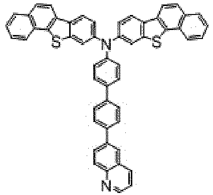 | 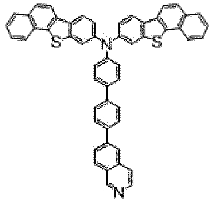 |
| **364** | **365** | **366** |
| 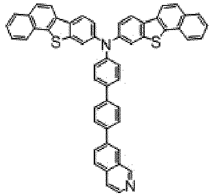 | 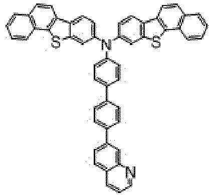 | 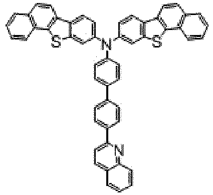 |
| **367** | **368** | **369** |
| 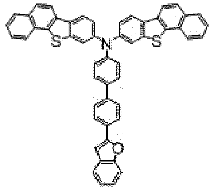 | 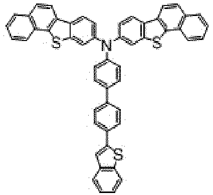 | 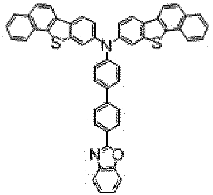 |
| **370** | **371** | **372** |
| 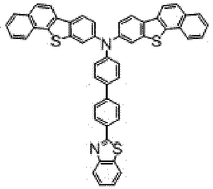 | 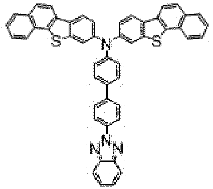 | 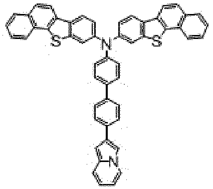 |
| **373** | **374** | **375** |
| 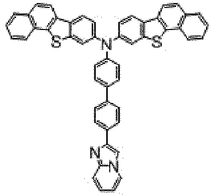 | 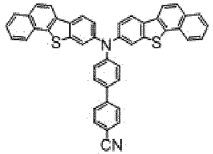 | 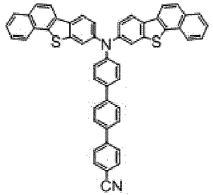 |
| **376** | **377** | **378** |
| 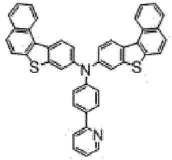 | 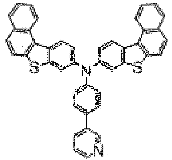 | 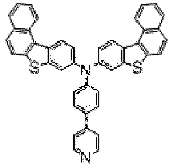 |
| **379** | **380** | **381** |
| 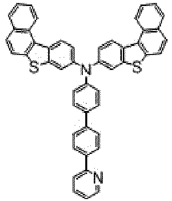 | 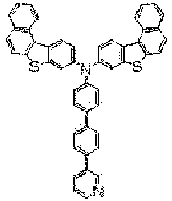 | 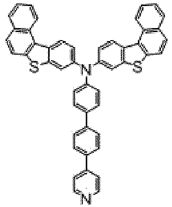 |
| **382** | **383** | **384** |
| 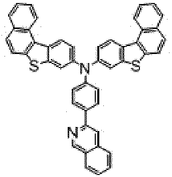 | 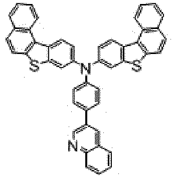 | 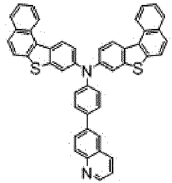 |
| **385** | **386** | **387** |
| 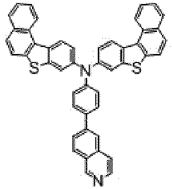 | 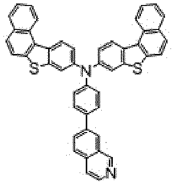 | 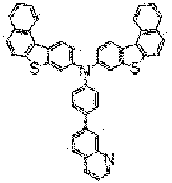 |
| **388** | **389** | **390** |
| 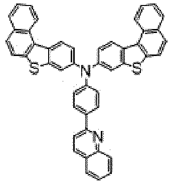 | 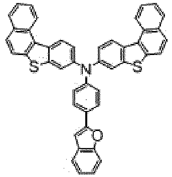 | 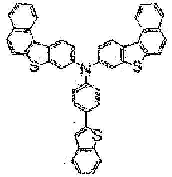 |
| **391** | **392** | **393** |
| 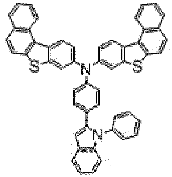 | 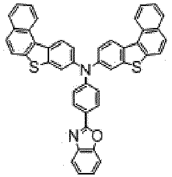 | 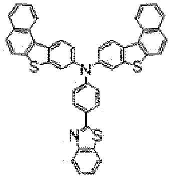 |
| **394** | **395** | **396** |
| 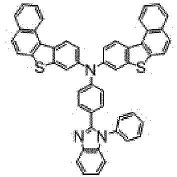 | 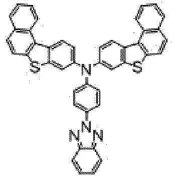 | 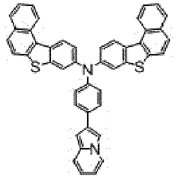 |
| **397** | **398** | **399** |
| 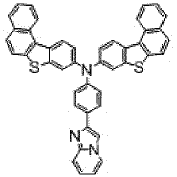 | 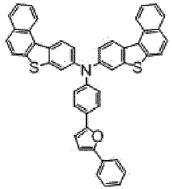 | 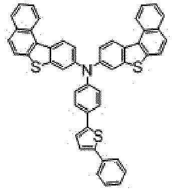 |
| **400** | **401** | **402** |
| 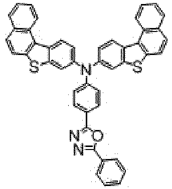 | 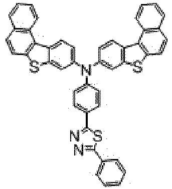 | 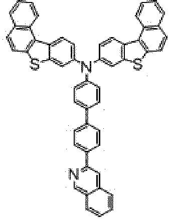 |
| **403** | **404** | **405** |
| 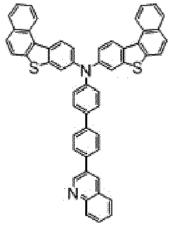 | 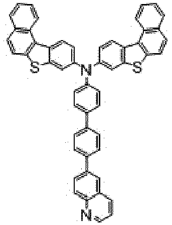 | 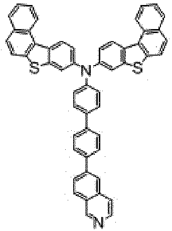 |
| **406** | **407** | **408** |
| 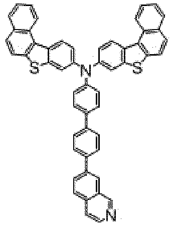 | 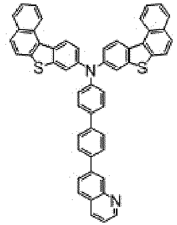 | 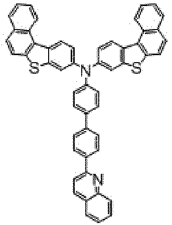 |
| **409** | **410** | **411** |
| 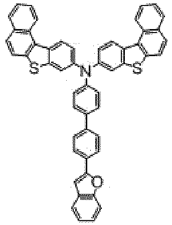 | 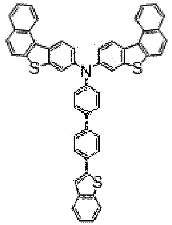 | 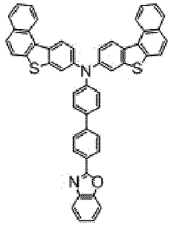 |
| **412** | **413** | **414** |
| 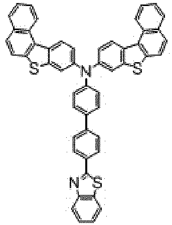 | 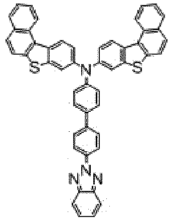 | 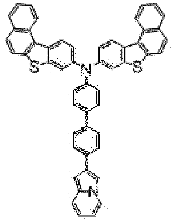 |
| **415** | **416** | **417** |
| 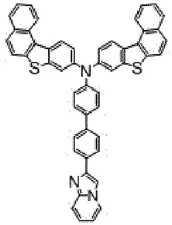 | 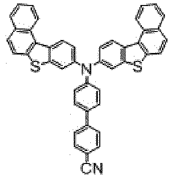 | 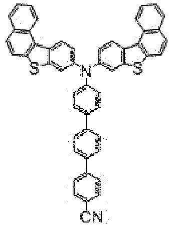 |
| **418** | **419** | **420** |
| 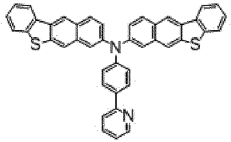 | 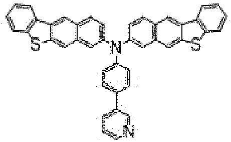 | 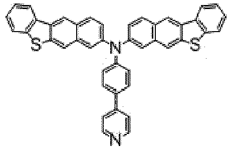 |
| **421** | **422** | **423** |
| 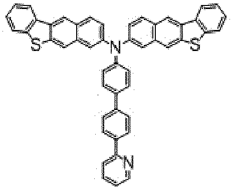 | 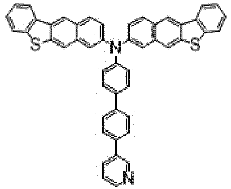 | 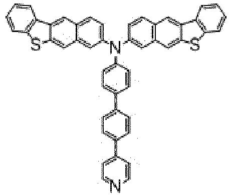 |
| **424** | **425** | **426** |
| 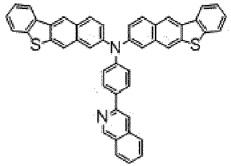 | 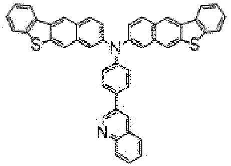 | 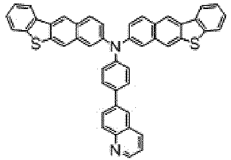 |
| **427** | **428** | **429** |
| 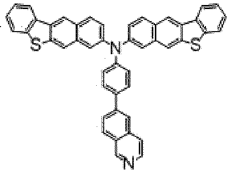 | 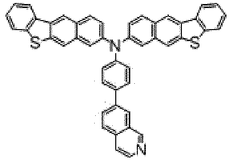 | 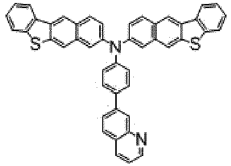 |
| **430** | **431** | **432** |
| 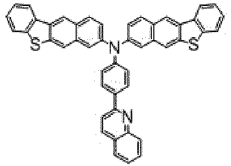 | 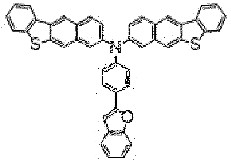 | 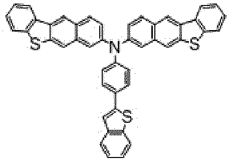 |
| **433** | **434** | **435** |
| 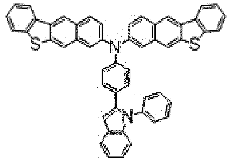 | 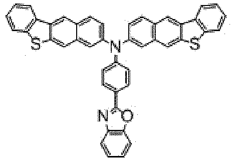 | 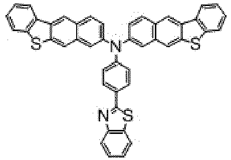 |
| **436** | **437** | **438** |
| 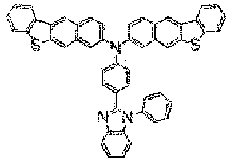 | 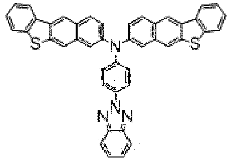 | 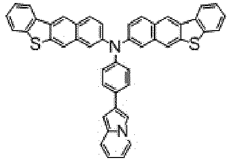 |
| **439** | **440** | **441** |
| 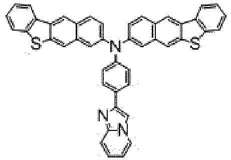 | 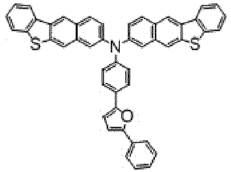 | 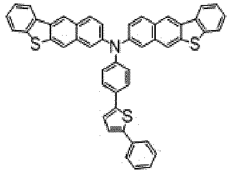 |
| **442** | **443** | **444** |
| 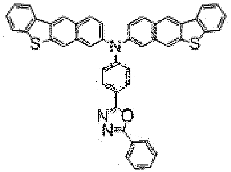 | 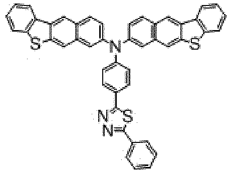 | 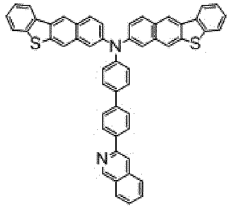 |
| **445** | **446** | **447** |
| 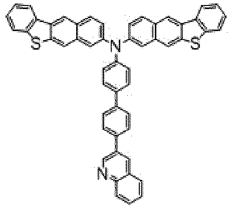 | 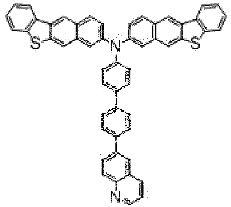 | 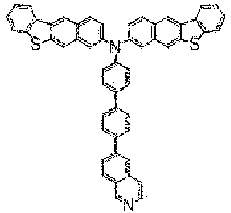 |
| **448** | **449** | **450** |
| 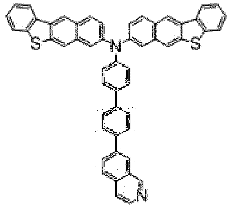 | 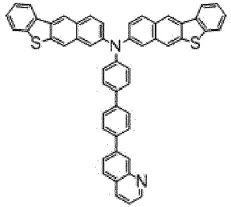 | 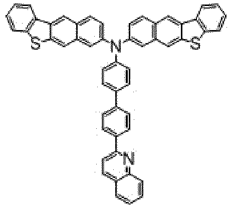 |
| **451** | **452** | **453** |
| 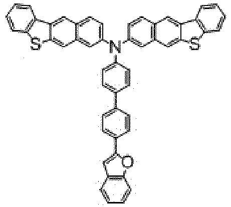 | 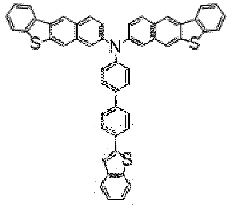 | 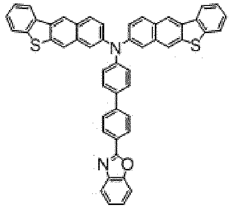 |
| **454** | **455** | **456** |
| 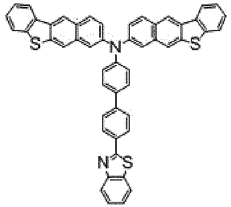 | 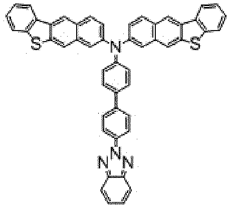 | 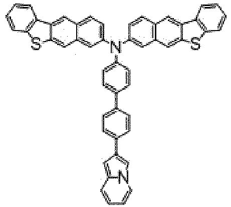 |
| **457** | **458** | **459** |
| 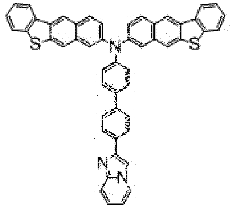 | 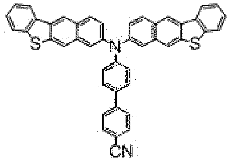 | 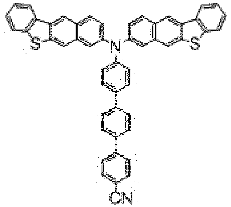 |
| **460** | **461** | **462** |
| 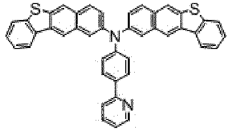 | 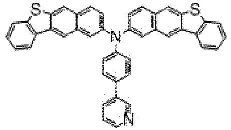 | 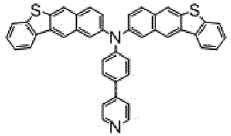 |
| **463** | **464** | **465** |
| 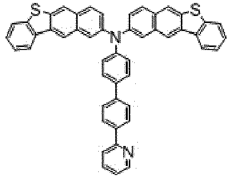 | 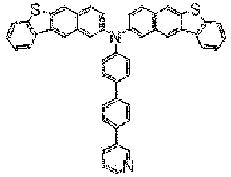 | 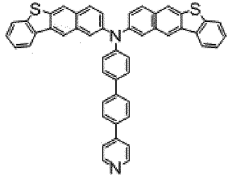 |
| **466** | **467** | **468** |
| 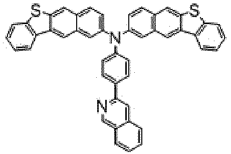 | 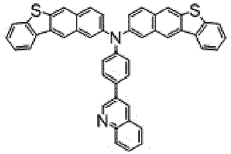 | 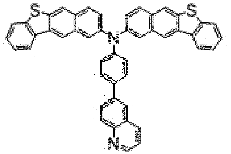 |
| **469** | **470** | **471** |
| 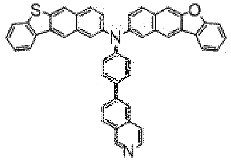 | 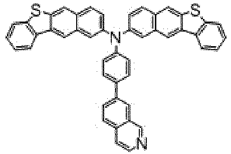 | 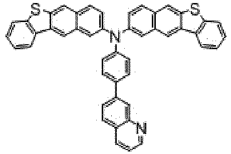 |
| **472** | **473** | **474** |
| 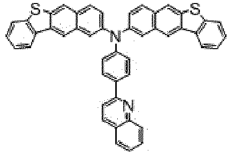 | 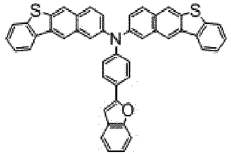 | 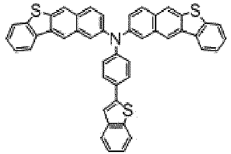 |
| **475** | **476** | **477** |
| 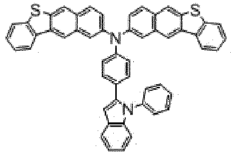 | 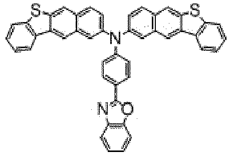 | 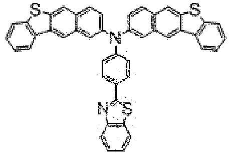 |
| **478** | **479** | **480** |
| 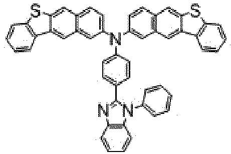 | 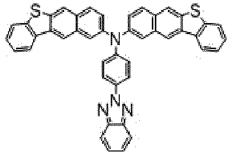 | 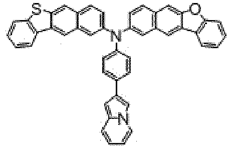 |
| **481** | **482** | **483** |
| 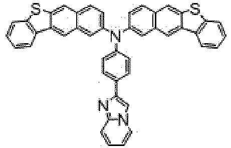 | 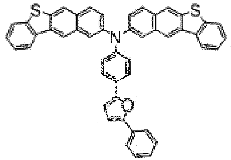 | 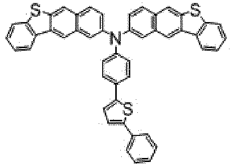 |
| **484** | **485** | **486** |
| 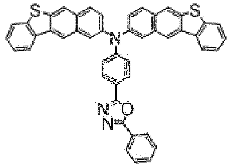 | 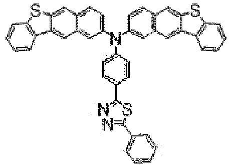 | 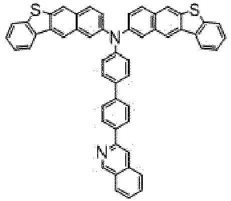 |
| **487** | **488** | **489** |
| 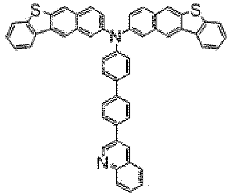 | 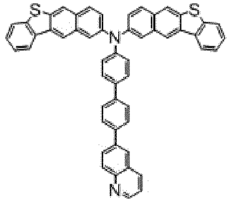 | 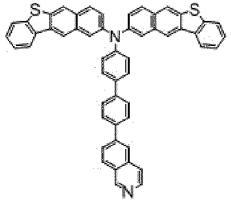 |
| **490** | **491** | **492** |
| 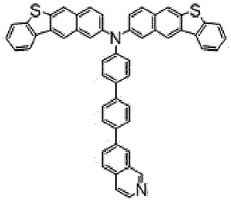 | 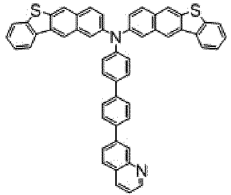 | 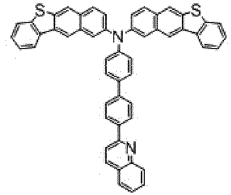 |
| **493** | **494** | **495** |
| 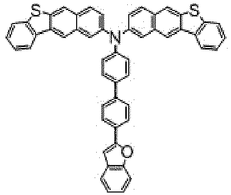 | 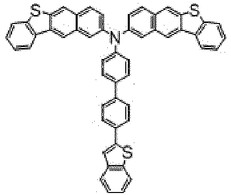 | 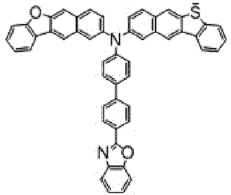 |
| **496** | **497** | **498** |
| 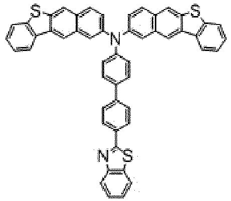 | 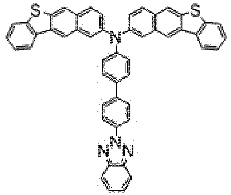 | 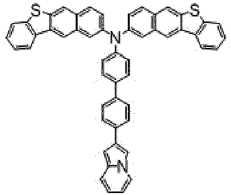 |
| **499** | **500** | **501** |
| 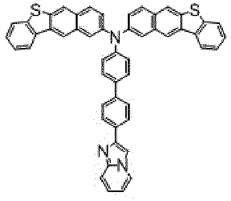 | 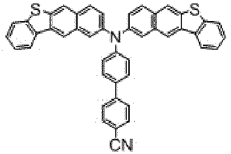 | 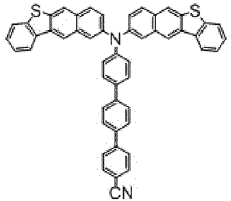 |
| **502** | **503** | **504** |
| 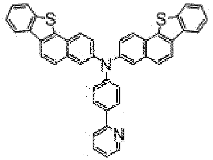 | 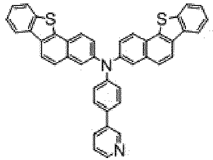 | 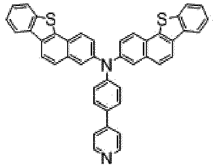 |
| **505** | **506** | **507** |
| 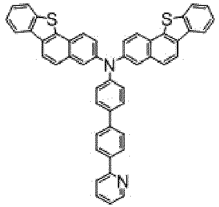 | 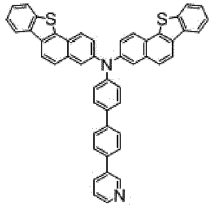 | 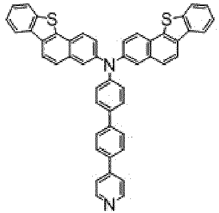 |
| **508** | **509** | **510** |
| 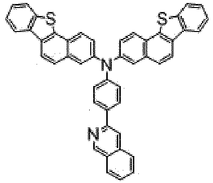 | 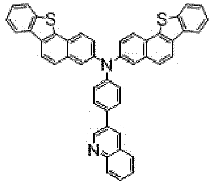 | 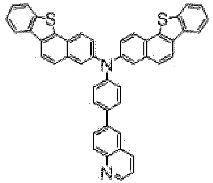 |
| **511** | **512** | **513** |
| 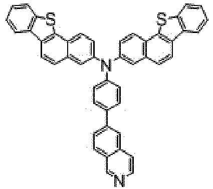 | 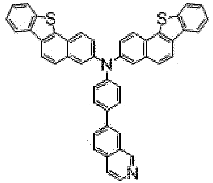 | 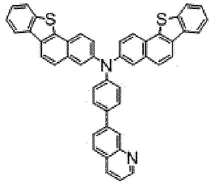 |
| 514 | **515** | **516** |
| 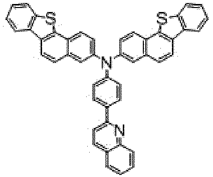 | 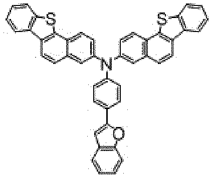 | 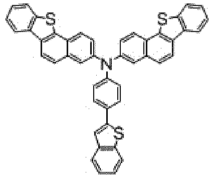 |
| **517** | **518** | **519** |
| 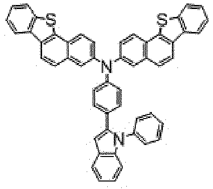 | 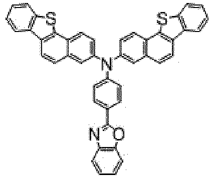 | 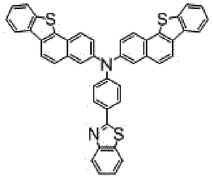 |
| **520** | **521** | **522** |
| 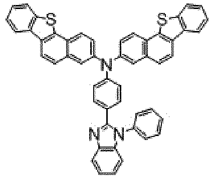 | 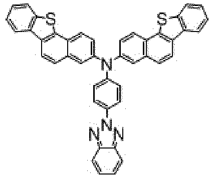 | 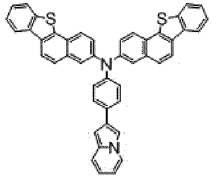 |
| **523** | **524** | **525** |
| 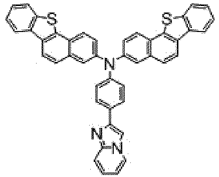 | 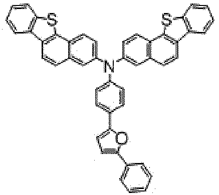 | 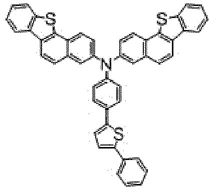 |
| **526** | **527** | **528** |
| 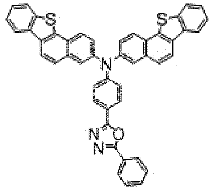 | 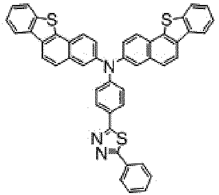 | 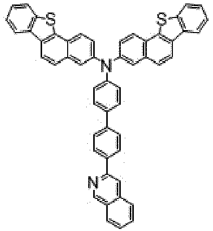 |
| **529** | **530** | **531** |
| 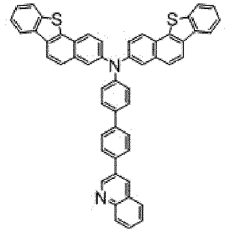 | 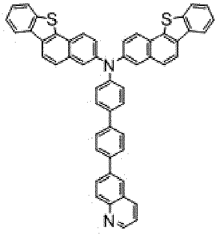 | 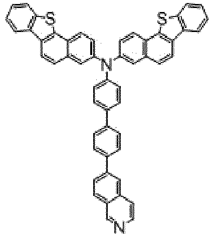 |
| **532** | **533** | **534** |
| 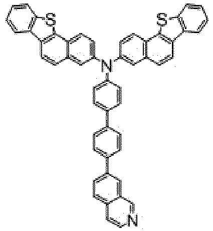 | 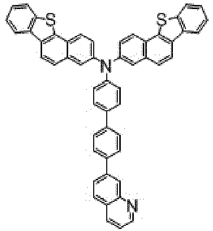 | 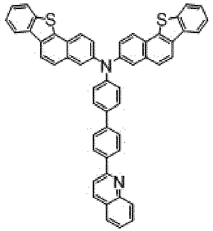 |
| **535** | **536** | **537** |
| 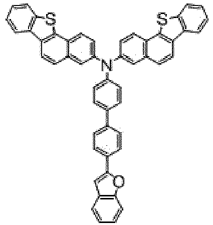 | 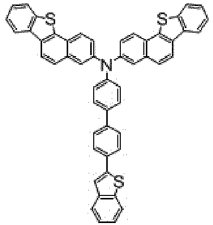 | 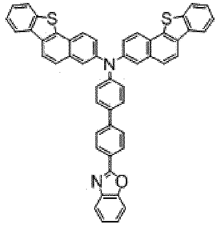 |
| **538** | **539** | **540** |
| 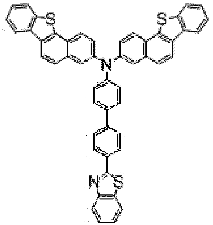 | 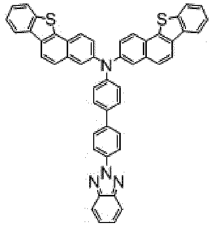 | 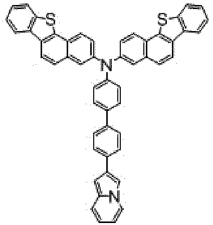 |
| **541** | **542** | **543** |
| 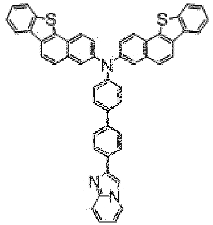 | 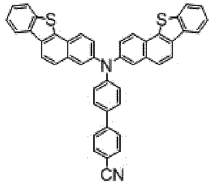 | 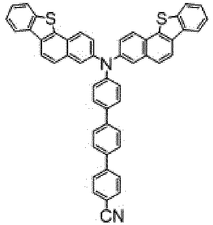 |
| **544** | **545** | **546** |
| 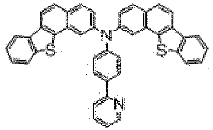 | 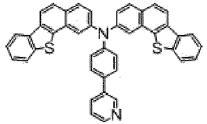 | 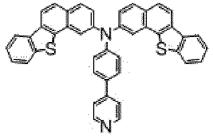 |
| **547** | **548** | **549** |
| 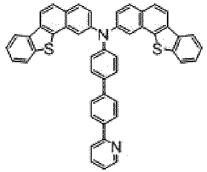 | 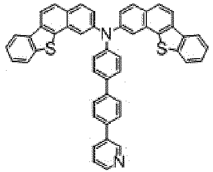 | 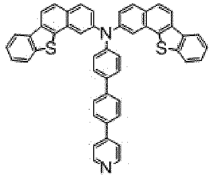 |
| **550** | **551** | **552** |
| 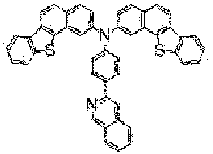 | 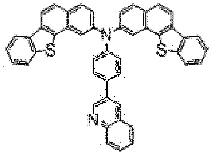 | 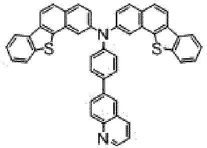 |
| **553** | **554** | **555** |
| 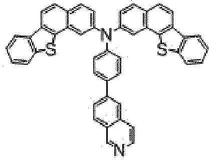 | 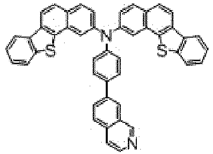 | 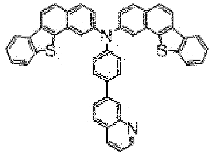 |
| **556** | **557** | **558** |
| 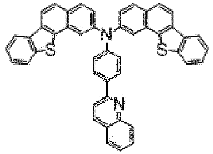 | 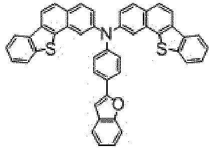 | 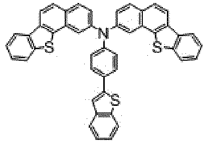 |
| **559** | **560** | **561** |
| 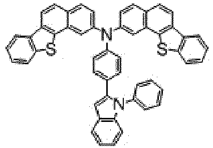 | 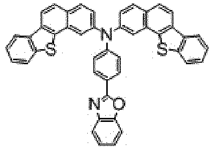 | 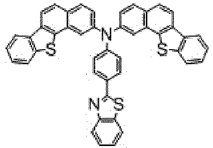 |
| **562** | **563** | **564** |
| 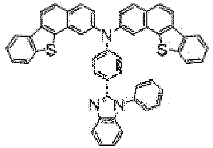 | 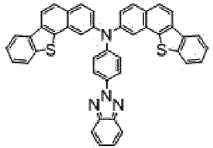 | 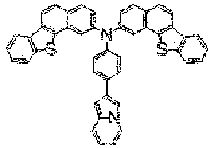 |
| **565** | **566** | 567 |
| 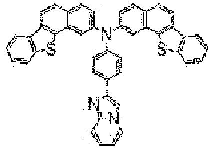 | 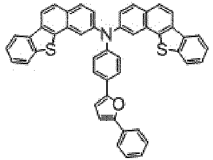 | 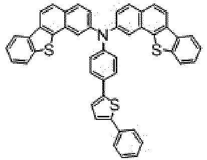 |
| **568** | **569** | **570** |
| 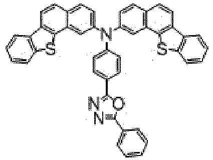 | 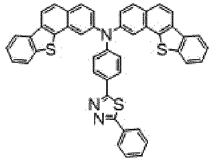 | 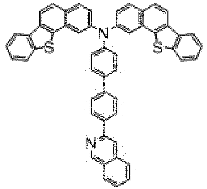 |
| **571** | **572** | **573** |
| 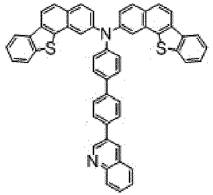 | 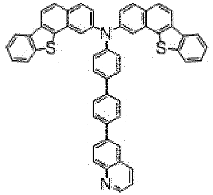 | 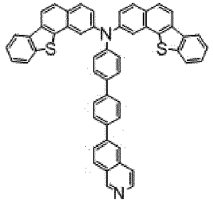 |
| **574** | **575** | **576** |
| 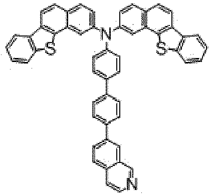 | 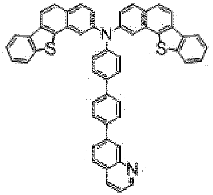 | 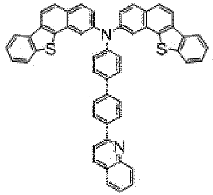 |
| **577** | **578** | **579** |
| 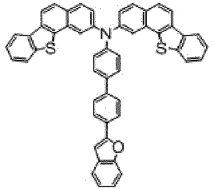 | 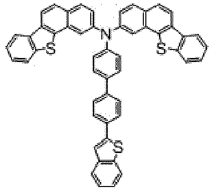 | 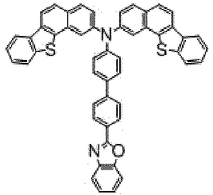 |
| **580** | **581** | **582** |
| 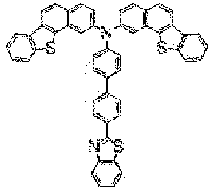 | 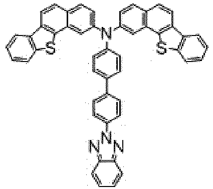 | 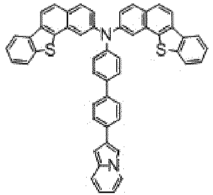 |
| **583** | **584** | **585** |
| 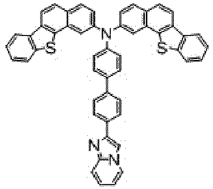 | 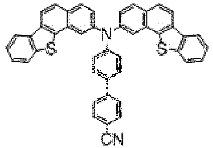 | 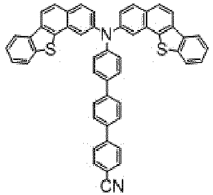 |
| **586** | **587** | **588** |

12. The compound for a capping layer of claim 1, wherein the compound has a refractive index of 2.45 or more at a wavelength of 450 nm.

13. An organic light emitting device comprising a capping layer comprising the compound for a capping layer of claim 1.

14. The organic light emitting device of claim 13, wherein the organic light emitting device comprises:
a first electrode;
a second electrode; and
one or more organic layers interposed between the first electrode and the second electrode,
wherein the capping layer is disposed outside one or more of the first electrode or the second electrode.

15. The organic light emitting device of claim 13, wherein the capping layer has a thickness in a range from 100 Å to 2000 Å.

16. The organic light emitting device of claim 13, wherein the capping layer has a refractive index of 2.45 or more at a wavelength of 450 nm.
